# EUROPEAN PATENT APPLICATION

(11) **EP 4 851 292 A1**
(43) Date of publication of application: **09.12.2026**
(21) Application number: 25747859.4
(22) Date of filing: 24.01.2025
(51) Int. Cl.: C12N 15/74, A61K 35/74

(54) **EUKARYOTIC EXPRESSION VECTOR, BACTERIUM CONTAINING SAME, AND USE**

(30) Priority: 31.01.2024 CN 202410136658
(71) Applicant: Shaoxing Salvectors Biotechnology Ltd., Shaoxing, Zhejiang 312080 (CN)
(72) Inventor: YU, Bin, Hong Kong (HK)
(74) Representative: Gille Hrabal Partnerschaftsgesellschaft mbB Patentanwälte
(86) International application number: PCT/CN2025/074820
(87) International publication number: WO 2025/162291

(57) **Abstract**

The present application relates to the field of genetic engineering and biological therapy, and provides a eukaryotic expression vector, a gram-negative bacterium containing the eukaryotic expression vector, and a use of the eukaryotic expression vector in the preparation of antitumor drugs and the treatment of tumors. By selecting and combining a phage-derived promoter, a specific phage RNA polymerase gene, a membrane-disrupting protein gene and a eukaryotic expression drug protein gene, the eukaryotic expression vector is divided into three modules : (1) a phage-derived RNA polymerase gene transcription module; (2) a membrane-disrupting protein gene expression module; and (3) a eukaryotic drug protein gene expression module. By means of organic combination of the three modules, a eukaryotic drug delivery system is constructed, and a system and method for stable and efficient eukaryotic drug protein expression, drug delivery and drug killing are implemented.

## Description

This application claims the benefit of priority to Chinese Patent Application No. 202410136658.9, filed with the China National Intellectual Property Administration (CNIPA) on January 31, 2024, entitled "Eukaryotic expression vector, bacterium containing same, and use", the entire contents of which are incorporated herein by reference.

### Technical Field

The present invention relates to a eukaryotic expression vector, a bacterium comprising the same, and applications thereof, and in particular, to a constitutive eukaryotic therapeutic mRNA/DNA mixed dual-delivery cytoplasmic expression system containing dual bacteriophage-derived promoters, a bacterium comprising the same, and applications thereof. The present invention belongs to the field of genetic engineering and biological therapy.

### Background Art

Cancer is one of the leading causes of death worldwide, and its incidence is continuously increasing year by year. Among all malignant tumors, solid tumors account for approximately 90%, such as sarcoma, melanoma, breast cancer, lung cancer, colon cancer, prostate cancer, etc.

The tumor microenvironment of solid tumors has common characteristics, including abnormal tumor vasculature, excessive connective tissue, immunosuppression, acidic environment and hypoxic regions. Moreover, the aberrant microenvironment of solid tumors serves as a natural barrier that hinders the penetration of conventional therapeutic agents, making it difficult for chemotherapeutic drugs and antibody-based therapeutics to diffuse within the tumor microenvironment. Additionally, the lack of oxygen free radicals makes tumors resistant to chemotherapy and radiotherapy.

Due to the hypoxic environment within solid tumors, facultative anaerobic bacteria and obligate anaerobic bacteria are able to invade tumors and inhibit their growth, and therefore can be used as therapeutic agents or carriers with great potential.

As early as 1868, the German phy*sician* W. Busch first reported that, in some tumor patients who were simultaneously infected with bacteria (*Streptococcus pneumoniae*), tumor growth in the body was inhibited and even eliminated. In the subsequent thirty years, the American physician Coley and another German physician, Fehleisen, respectively reported the phenomenon that bacterial infection could suppress tumors. These early studies were controversial because their results were difficult to reproduce and the virulence of the bacteria was difficult to control. However, subsequent rigorous animal experiments demonstrated that bacterial infection can indeed reduce tumor size, and that the host immune system is also activated during treatment. In 1975, Carswell first reported that endotoxin (lipopolysaccharide) of Gram-negative bacteria could stimulate the immune system to release tumor necrosis factor TNF-α and could lead to tumor cell death. In addition, some bacterial vaccines have also been shown to stimulate the immune system to thereby treat tumors. Among them, Bacillus Calmette-Guérin (BCG) is one of the earliest biological preparations used in clinical tumor therapy. BCG is a live attenuated bacterial suspension prepared by serially passaging highly virulent *Mycobacterium bovis* for 230 generations, and is originally intended for the prevention of tuberculosis. A large number of experiments and clinical practices have confirmed that BCG is one of the most effective means for treating bladder cancer.

In recent years, with the rapid development of molecular biology and genetic engineering technologies, it has been found that some facultative or obligate anaerobic bacteria can target, colonize and proliferate within solid tumors and induce tumor regression, such as obligate anaerobic *Clostridium* and probiotic *Bifidobacterium*. Among them, the Gram-negative facultative anaerobe *Salmonella enterica* has the broadest application prospects.

Studies have shown that, after attenuation of *Salmonella typhimurium* using different methods, its colonization capacity in tumor tissues can reach 1,000-10,000 times than that in normal tissues, making it a potential targeted drug for tumor treatment.

Regarding improvements in recombinant bacteria targeting tumor regions, current literature reports that these recombinant bacteria can also carry different types of drugs to achieve targeted drug delivery to tumor regions. However, their effects are not ideal. The reason may be that the efficiency of drug synthesis by bacteria within tumors and delivery has not been comprehensively considered.

Moreover, because bacteria are used as carriers for drug delivery, the carried drugs themselves are limited by the bacteria's inherent conditions. First, bacteria are prokaryotes, and proteins directly produced by their expression systems lack glycosylation modifications and chaperone systems necessary for protein folding. Therefore, if the carried drug is a eukaryotic cell protein, it is difficult to express a correctly folded or glycosylated protein, thereby affecting its efficacy. Second, if the expressed therapeutic protein is inherently toxic to the bacterium, the bacterium will be unable to express it efficiently.

Therefore, when using bacteria as a carrier to deliver a eukaryoticly expressed therapeutic gene to eukaryotic cells for expression, since the DNA transcription program of the eukaryotic system occurs in the nucleus, directly delivering DNA via bacteria requires the DNA to shuttle into the nucleus. However, the probability of exogenous DNA entering the nucleus is extremely low. Only during cell mitosis, when the nuclear envelope depolymerizes, it is possible for exogenous DNA to enter the nuclear region. Thus, adopting a cytoplasmic expression mode is the key to increasing delivery efficiency.

The 2023 Nobel Prize in Physiology or Medicine was awarded to scientists Katalin Karikó and Drew Weissman in recognition of their discoveries concerning nucleoside base modifications. Their contributions enabled the development of effective messenger RNA (mRNA) vaccines against COVID-19 infection. The success of mRNA vaccines relative to DNA vaccines suggests that the direct cytoplasmic expression mode of mRNA has significant advantages compared to the DNA expression mode, which requires nuclear entry.

US Patent US10987432B2 reports a method for cytoplasmic expression based on a bacterial vector. The method involves delivering an mRNA/DNA mixed vector via *Salmonella*, where the vector carries a therapeutic gene controlled by a CMV promoter and a T7 promoter, and downstream carries an autogene composed of a T7 promoter and a T7 RNA polymerase gene to automatically amplify gene expression. The purpose is to continuously synthesize T7 RNA polymerase in the cytoplasm through the carried T7 RNA polymerase gene, forming a positive feedback gene circuit, while simultaneously synthesizing the target therapeutic gene.

However, this design is not essentially a true cytoplasmic expression vector. In this design, it carries a CMV promoter (transcription initiation in the nucleus) and simultaneously carries a polyA signal regulatory element (this element needs to participate in the mRNA maturation process in the nucleus) rather than a polyadenine tract. A polyA mRNA tail cannot be effectively formed in the cytoplasm; therefore, it is not a specifically effective cytoplasmic expression method, but rather a nucleus-entry-dependent cytoplasmic expression mode.

In addition, on the therapeutic gene vector, two T7 promoters separately regulate the transcription of the therapeutic gene and the T7 RNA polymerase. This design may cause post-transcriptional mRNA diversity, ultimately leading to a decrease in the number of effective mRNAs delivered into the cytoplasm. At the same time, the expression mode of its membrane-disrupting protein genes has not been optimized, which will affect its ultimate delivery efficiency and thereby impact the overall therapeutic efficacy.

In summary, there is an urgent need in the art for a eukaryotic therapeutic protein system and method that allows bacteria to achieve stable, highly efficient expression, and highly efficient release within in vivo tumors.

### Summary of the Invention

In the present invention, unless otherwise specified, the scientific and technical terms used herein shall have the meanings commonly understood by those skilled in the art to which this invention pertains. Furthermore, the relevant terminology and laboratory procedures related to protein and nucleic acid chemistry, molecular biology, cell and tissue culture, microbiology, and immunology used in the present invention are all terms and conventional procedures widely used in the respective fields.

In view of the above-mentioned defects existing in the prior art, the present invention establishes a constitutive eukaryotic therapeutic mRNA/DNA mixed dual-delivery cytoplasmic expression system containing dual bacteriophage-derived promoters.

To this end, the present invention provides a constitutive eukaryotic therapeutic mRNA/DNA mixed dual-delivery cytoplasmic expression system containing dual bacteriophage-derived promoters, comprising three modules: (1) a bacteriophage-derived promoter and RNA polymerase transcription control module; (2) a delivery module; and (3) a eukaryotic therapeutic mRNA transcription module and a bacteriophage-derived RNA polymerase self-amplifying module. Through the organic combination of these three modules in conjunction with the characteristics of bacteria, the problem of the difficulty for bacterial vectors to carry eukaryotic therapeutics is solved.

### 1. Bacteriophage-derived promoter and RNA polymerase transcription control module

In order to achieve stable and highly efficient gene expression within the tumor microenvironment, it is necessary to use a high-strength promoter expression system that is unaffected by environmental factors. The present invention employs a constitutive artificial promoter lacUV5 to control the expression of the bacteriophage-derived T7 RNA polymerase gene, and integrates this expression cassette into the bacterial chromosome to achieve stable inheritance. Because T7 RNA polymerase specifically recognizes the T7 promoter, stable and high-strength expression of genes controlled by the T7 promoter can be achieved. Therefore, the delivery module, the eukaryotic therapeutic mRNA transcription module, and the bacteriophage-derived RNA polymerase self-amplifying module are respectively placed downstream of the T7 promoter and localized on a plasmid for expression regulation.

### 2. Delivery module

When bacteria invade tumor cells, phagosomes are formed. The presence of phagosomes can restrict the intracellular release of bacteria and the drugs synthesized by them. To address this problem, the present invention preferably uses listeriolysin-O (LLO) as a membrane-disrupting protein to achieve drug delivery. In order to increase its efficiency as a membrane-disrupting protein, the present invention places the membrane-disrupting protein genes (LLO gene) on the expression vector plasmid and uses the T7 promoter to control expression. This not only increases the overall expression level of LLO but also, by expressing it simultaneously with the eukaryotic therapeutic mRNA in advance, allows for highly efficient membrane disruption synchronous with the formation of phagocytic vesicles. Compared with patent US10987432B2, the overall delivery efficiency is improved.

### 3. Eukaryotic therapeutic mRNA transcription module and bacteriophage-derived RNA polymerase self-amplifying module

### (1) Selection of eukaryotic therapeutics

In order to realize a precision drug that exerts a highly efficient killing effect exclusively within tumor cells, the present invention utilizes the N-terminal fragment of a eukaryotic pyroptotic protein. Pyroptosis is a form of cell death crucial for immunity. It is typically induced by canonical caspase-1 inflammasomes or activated caspase-4, -5, and -11. Activated caspases cleave the pyroptotic protein gasdermin (GSDM) (including GSDMA, GSDMB, GSDMC, GSDMD, and GSDME) within its intermediate linker to release its gasdermin-N-terminal fragment domain, which is in an activated state; this fragment can penetrate the cell membrane, thereby inducing pyroptosis.

Studies have found that upon caspase activation, GSDMD undergoes proteolytic cleavage at the Asp 275 site, while GSDME is specifically cleaved by caspase-3 at the Asp 270 site to break into two parts and gain activity. The N-terminal protein of the cleaved GSDMD or GSDME possesses pore-forming activity and can insert into the cell membrane to form pores, thereby triggering pyroptosis. GSDME is silenced in most cancer cells but expressed in many normal tissues. Upon activation of caspase-3 by chemotherapeutic drugs, human primary cells exhibit GSDME-dependent pyroptosis. Conversely, the C-terminus of GSDMD or GSDME acts as an inhibitor, suppressing the activity of the N-terminus in the uncleaved state. The N-terminal cleavage product of GSDM localizes to the plasma membrane by anchoring to membrane lipids and specifically interacts with phosphatidylinositol 4-phosphate [PI(4)P] and phosphatidylinositol 4,5-bisphosphate [PI(4,5)P] on the inner leaflet of the mammalian cell membrane, mediated through the negatively charged head groups of [PI(4)P] and [PI(4,5)P] and the positively charged surface of GSDM-N exposed after cleavage. Lipid binding allows GSDM-N to insert into the lipid bilayer and induce membrane oligomerization, forming extensive pores with an inner diameter of 10-14 nm. Osmotic pressure is disrupted by pore formation, resulting in cell swelling and lysis, which are the morphological hallmarks of pyroptosis. These extensive pores also serve as protein secretion channels, promoting the secretion of inflammatory cytokines to achieve a rapid innate immune response. Due to the extracellular lack of [PI(4)P] and [PI(4,5)P], GSDM-N leaking outside the cell is non-toxic. Because the action of GSDM-N on the inner cell membrane is indiscriminate, GSDM-N is also toxic to bacteria.

The two characteristics of GSDM-N-its eukaryotic origin requiring glycosylation modification, and its indiscriminate attack on the bacterial inner membrane-render the direct expression of the GSDM-N gene by bacteria unfeasible. Therefore, opting for a strategy where bacteria do not express it directly but instead deliver a eukaryotic system is an exceptionally advantageous approach.

### (2) Selection of the eukaryotic mRNA cytoplasmic expression system

Studies have shown that *Salmonella* can carry eukaryotic plasmids and deliver them into cells. However, because plasmid DNA must enter the nucleus to undergo transcription into mRNA before translation can commence, this efficiency is extremely low. To overcome this problem, the present invention employs a bacterial system to directly synthesize eukaryotic mRNA within the bacteria, and through a delivery system, the mRNA/DNA vector can be directly delivered into the cytoplasm of cancer cells to synthesize eukaryotic therapeutic proteins utilizing the cytoplasmic translation system. The structure of the mature mRNA includes a viral internal ribosome entry site (IRES) sequence, a Kozak sequence, the open reading frame (ORF) region of the eukaryotic therapeutic, a 3'-UTR region, and a polyA tail. In the present invention, because bacteria themselves lack the relevant enzymes for mRNA capping, the viral internal entry sequence IRES is used as a substitute, successfully enabling the bacteria to directly synthesize and carry the IRES-containing mRNA for delivery to the cytoplasm of cancer cells.

### (3) Bacteriophage-derived RNA polymerase self-amplifying system based on mRNA/DNA mixed expression

Although bacteria can directly deliver the synthesized mRNA into the cytoplasm to achieve expression, the inherently low efficiency of bacteria in delivering nucleic acids necessitates further improvement. In addition to adjusting the spatiotemporal expression pattern of LLO as described above, the approach of the present invention provides further enhancement through bacteriophage-derived RNA polymerase self-amplification. Compared with the method in patent US10987432B2, the present patent completely abandons the paradigms of nuclear expression and dual monocistrons (dual T7 promoters controlling self-amplifying transcription), adopting a purely cytoplasmic expression method. The eukaryotic promoter CMV is removed, and the redundant design of a polyA signal (BGH polyA site "AATAAA") regulatory element (an element that needs to participate in the mRNA maturation process in the nucleus) is discarded. Instead, it is directly designed as a single mRNA bicistronic transcription mode: a single T7 promoter is directly followed by an IRES to control the stable expression of the therapeutic gene and a 3'-UTR, supplemented by a second IRES to control the stable expression of the T7 RNA polymerase and a 3'-UTR, with a polyA sequence directly adopted downstream as the 3' end of the mRNA. This design can realize a highly efficient mRNA/DNA mixed delivery mode.

In the tumor treatment process of the present invention, the bacteria enter the tumor tissue through the circulatory system and colonize therein. Inside the bacteria, the stable expression of T7 RNA polymerase promotes the massive synthesis of the bicistronic mRNA for the GSDM-N fragment and the T7 RNA polymerase located on the plasmid, as well as the stable and high expression of the membrane-disrupting protein LLO of the drug delivery module. When the bacteria approach cancer cells within the tumor tissue, they utilize their innate invasion system to induce endocytosis by the cancer cell, forming bacteria-containing phagocytic vesicles. Subsequently, these phagocytic vesicles fuse with intracellular vacuoles such as lysosomes, thereby lowering the pH inside the phagocytic vesicles. At this point, the membrane-disrupting protein LLO expressed by the bacteria is activated, causing the bicistronic mRNA of the GSDM-N fragment and T7 RNA polymerase, along with the vector DNA, to be released into the cytoplasm. Translation is then initiated in the cytoplasm, and the newly generated T7 RNA polymerase acts on the vector DNA again to launch the next round of transcription. The accumulated GSDM-N fragment exerts its function, ultimately leading to the pyroptosis of the cancer cells. This process is continuously repeated as the bacteria grow and replicate within the tumor, driving the rapid resolution of tumor cells. The features of the present invention can only be realized based on the eukaryotic therapeutic mRNA/DNA mixed dual-delivery cytoplasmic expression system.

In the present invention, dual bacteriophage-derived promoters are employed to respectively control the self-amplifying transcription of the GSDM-N fragment and T7 RNA polymerase, and the synchronous expression of the membrane-disrupting protein LLO; furthermore, the corresponding bacteriophage RNA polymerase gene controlled by a constitutively expressed lacUV5 promoter is integrated into the host bacterial chromosome.

In a preferred embodiment of the present invention, the dual bacteriophage-derived promoters are selected from the T7 promoter (SEQ ID NO: 1), the T3 promoter (SEQ ID NO: 2), and the SP6 promoter (SEQ ID NO: 3).

In a more preferred embodiment of the present invention, the dual bacteriophage-derived promoter is the T7 promoter (SEQ ID NO: 1).

In a preferred embodiment of the present invention, the specific bacteriophage RNA polymerase gene is a gene corresponding to the dual bacteriophage-derived promoter.

In a more preferred embodiment of the present invention, the specific bacteriophage RNA polymerase gene is selected from the *Salmonella* codon-optimized T7 RNA polymerase gene (SEQ ID NO: 4) and T7 RNA polymerase protein sequence (SEQ ID NO: 5), the T3 RNA polymerase gene (SEQ ID NO: 6) and T3 RNA polymerase protein sequence (SEQ ID NO: 7), and the SP6 RNA polymerase gene (SEQ ID NO: 8) and SP6 RNA polymerase protein sequence (SEQ ID NO: 9).

In a further preferred embodiment of the present invention, the specific bacteriophage RNA polymerase gene is the T7 RNA polymerase gene.

In order to achieve stable and highly efficient expression of the therapeutic protein, the present invention places the therapeutic protein gene on a multi-copy expression plasmid vector and controls it via the T7 promoter to maximize the expression intensity of the therapeutic protein gene.

To achieve an even higher expression intensity, in a preferred embodiment of the present invention, the T7 bacteriophage RNA polymerase expression system is adopted, which includes a T7 RNA polymerase and a T7 promoter to control the expression of the target therapeutic protein.

The T7 RNA polymerase system is derived from the expression system of the *Escherichia coli* T7 bacteriophage, and it is widely used for gene expression in *E. coli* due to its robust protein expression capabilities. A key characteristic of T7 RNA polymerase is that it can specifically recognize the T7 promoter without being affected by environmental factors. Moreover, the RNA synthesis rate of T7 RNA polymerase is five times that of *E. coli* RNA polymerase, enabling high-intensity expression of the target protein. In addition, transcription by T7 RNA polymerase does not rely on the assistance of other transcription factors and can be executed independently; thus, it can independently initiate transcription even within the cytoplasm of eukaryotic cells. This provides a tremendous advantage for cytoplasmic expression systems.

To ensure stable expression that is unaffected by the internal metabolism of the bacteria or the microenvironment within the tumor, in a more preferred embodiment of the present invention, a constitutively expressed lacUV5 promoter (SEQ ID NO: 10) is further adopted to control the expression of the specific bacteriophage RNA polymerase gene.

The lacUV5 promoter is highly similar to the classical lac promoter. Compared to the lac promoter, it contains only a two-base pair mutation in the -10 region. The lacUV5 promoter does not require additional activators and can drive high levels of gene expression. Although an activator is not needed, the expression from the lacUV5 promoter can be regulated by the LacI repressor in *E. coli* and can be induced by IPTG. IPTG is an effective inducer when used at a concentration range of 100 µM to 1.5 mM.

Because *Salmonella* has lost the *lacI* gene and the entire lac operon during its evolution, the use of the lacUV5 promoter in *Salmonella* constitutes an excellent constitutive expression system.

Therefore, in a more preferred embodiment of the present invention, the T7 RNA p*o*lymerase expression cassette controlled by the *lacUV5* promoter is integrated into the chromosome of *Salmonella*, thereby realizing stable constitutive expression.

In a preferred embodiment of the present invention, the T7 polymerase and T7 promoter can alternatively be replaced by a T3 polymerase and T3 promoter. Similar to the T7 RNA polymerase system, the T3 RNA polymerase system is an RNA polymerase system derived from T3 bacteriophage that highly specifically recognizes the T3 promoter sequence. Alternatively, the T7 polymerase and T7 promoter can be replaced by an SP6 polymerase and SP6 promoter. Similar to the T7 RNA polymerase system, SP6 polymerase is also an RNA polymerase system derived from SP6 bacteriophage that highly specifically recognizes the SP6 promoter sequence. T3 RNA polymerase or SP6 RNA polymerase, together with their respective promoters, can functionally replace T7 RNA polymerase to specifically control the expression of downstream target genes.

In a preferred embodiment of the present invention, T7 promoter is employed to control a bicistronic tandem array for the eukaryotic expression of the therapeutic protein and the T7 RNA polymerase. This provides two advantages: First, when inside the bacteria, the transcription of the therapeutic protein and the T7 RNA polymerase by the T7 polymerase produces a single bicistronic mRNA product. A single product is highly advantageous for controlling the ratio between the therapeutic protein and the T7 RNA polymerase to achieve optimal delivery efficiency. Second, using a T7 promoter to directly control the T7 RNA polymerase can easily trigger a continuous positive-feedback self-amplifying loop, which could deplete internal bacterial resources and lead to bacterial cell death. Although the T7 RNA polymerase used on the vector follows a eukaryotic expression mode and lacks a necessary ribosome binding site (RBS)-which reduces the efficiency of prokaryotic expression within the bacteria-leaky expression cannot be completely prevented. Therefore, the bicistronic self-amplifying design enhances overall bacterial viability and efficiency.

Moreover, to prevent the loss of the plasmid expression vector, an essential *Salmonella* gene needs to be incorporated during vector construction, while simultaneously deleting the corresponding gene from the host bacterial chromosome. This creates a balanced lethal control mechanism, meaning that if *Salmonella* loses the carried plasmid vector, the bacteria rapidly die due to the lack of the essential gene. All surviving bacteria will carry the plasmid expression vector, thereby ensuring stable and efficient expression of the protein drug by the entire system. The *asd* gene (SEQ ID No. 11) and the asd protein sequence (SEQ ID No. 12) from *Salmonella* encode aspartate β-semialdehyde dehydrogenase, an enzyme required for the synthe*s*i*s* of diaminopimelic acid (DAP), an important component of Gram-negative bacterial cell walls. Deleting the *asd* gene from *Salmonella* leads to bacterial lysis and death; howev*e*r, normal *Salmonella* growth can be maintained by supplementing DAP in the culture medium or by having *Salmonella* carry a vector containing the *asd* gene. Therefore, in the present invention, *asd* is utilized as the essential gene for the plasmid vector. In a preferred embodiment of the present invention, the expression vector comprises the essential *Salmonella* gene *asd*.

To achieve stable and efficient drug delivery, the present invention designs a precise drug delivery system. Its objective is to precisely release the therapeutic protein synthesized by bacteria into tumor cells, which is further divided into two steps: bacterial invasion of host cancer cells and release of the synthesized therapeutic protein.

In a preferred embodiment of the present invention, the natural ability of *Salmonella* to invade host animal cells is utilized to achieve bacterial invasion of host cancer cells.

*Salmonella enterica* can invade host cells and replicate within them, including host epithelial cells and macrophages. This ability resides in *Salmonella* pathogenicity island 1 (SPI-1). Through the type III secretion system 1 (T3SS1), *Salmonella* secretes T3SS effector proteins, inducing extensive actin rearrangement in host cells, leading to membrane ruffling and the formation of phagosomes containing *Salmonella*. Subsequently, the type III secretion system 2 (T3*SS*2), encoded by *Salmonella* pathogenicity island 2 (SPI-2) genes, promotes the survival and proliferation of *Salmonella* within the phagosomes.

In a preferred embodiment of the present invention*,* the purpose of releasing the synthesized therapeutic protein is achieved using *Salmonella*-synthesized *Salmonella* codon-optimized listeriolysin-O (LLO) gene (SEQ ID No. 13) and its protein sequence (SEQ ID No. 14).

LLO is encoded by the hlyA gene of Listeria monocytogenes (LM) and can bind to cholesterol in host cell membranes, forming pore structures with a diameter of 35 nm.

LLO has a pH-sensitive acidic domain. The optimal pH for the maturation of LLO precursor protein and the initiation of its membrane-perforating activity is approximately 5.0-5.5, while it is inactivated at neutral pH.

When *Salmonella* invades and forms phagosomes, the phagosomes fuse with intracellular vacuoles such as lysosomes, thereby significantly lowering the pH. Under these conditions, LLO's activity is activated, exerting its perforating effect and enabling *Salmonella* to release the synthesized therapeutic protein.

Therefore, in a preferred embodiment of the present invention, the membrane-disrupting protein gene is the listeriolysin *hlyA* gene.

In the present invention, the expression regulation of LLO is also optimized. In US10987432B2, LLO is controlled by the promoter of the *Salmonella* pathogenicity island II *sseA* gene, and its expression is induced after the bacteria invade cells and form phagocytic vesicles. However, its expression level and efficiency exhibit a severe lagging effect relative to the vector that needs to be delivered. Therefore, in the present invention, the same T7 promoter is employed to control the expression of LLO. This ensures that the expression of LLO is synchronized with the transcription and release of the eukaryotic mRNA, guaranteeing the maximization of the overall release efficiency.

In order to achieve stable and highly efficient therapeutic killing, the present invention selects a therapeutic protein with highly efficient and specific killing capabilities, which, while killing tumor cells, can also prevent accidental injury to other organs upon leakage of the therapeutic protein.

In a preferred embodiment of the present invention, the therapeutic protein is the pyroptotic protein GSDMD-N (the N-terminal fragment of GSDMD), comprising the gene of GSDMD-N (SEQ ID NO: 23) and the protein sequence of GSDMD-N (SEQ ID NO: 24), and GSDME-N (the N-terminal fragment of GSDME), comprising the gene of GSDME-N (SEQ ID NO: 25) and the protein sequence of GSDME-N (SEQ ID NO: 26).

In a preferred embodiment of the present invention, the therapeutic protein can also be the pyroptotic protein GSDMA-N (the N-terminal fragment of GSDMA), comprising the gene of GSDMA-N (SEQ ID NO: 44) and the protein sequence of GSDMA-N (SEQ ID NO: 45); GSDMB-N (the N-terminal fragment of GSDMB), comprising the gene of GSDMB-N (SEQ ID NO: 46) and the protein sequence of GSDMB-N (SEQ ID NO: 47); and GSDMC-N (the N-terminal fragment of GSDMC), comprising the gene of GSDMC-N (SEQ ID NO: 48) and the protein sequence of GSDMC-N (SEQ ID NO: 49).

In a preferred embodiment of the present invention, the expression vector further comprises a hypoxia-specific gene expression cassette, which comprises:
a) a forward hypoxia promoter, which is a promoter comprising FNR binding site, wherein the forward hypoxia promoter is capable of being induced for expression under hypoxia;
b) a survival-essential gene; and
c) a reverse hyperoxia promoter, which is a promoter comprising FNR and ArcA binding site, wherein
the reverse hyperoxia promoter is capable of functioning under oxygen concentrations of normal organs; wherein the survival-essential gene is a gene encoding alanine racemase.

The terms "polypeptide", "peptide", and "protein" are used interchangeably herein and refer to a polymer of amino acid residues. The term applies to amino acid polymers in which one or more amino acid residues are artificial chemical analogs of a corresponding natural amino acid, as well as to polymers of natural amino acids. The terms "polypeptide", "peptide", "amino acid sequence", and "protein" may also include modified forms, including but not limited to glycosylation, lipidation, sulfation, γ-carboxylation of glutamic acid residues, hydroxylation, and ADP-ribosylation.

In the present invention, "polynucleotide" refers to a macromolecule formed by multiple nucleotides linked via phosphodiester bonds, wherein the nucleotides include ribonucleotides and deoxyribonucleotides. The sequence of the polynucleotide of the present invention can be codon-optimized for different host cells (such as *E. coli*) to improve polypeptide expression. Methods for codon optimization are known in the art.

"Sequence identity" between two polypeptide sequences or two polynucleotide sequences refers to the percentage of identical amino acids or nucleotides between said sequences. Methods for assessing the level of sequence identity between polypeptide or polynucleotide sequences are known in the art. Sequence identity can be assessed using various known sequence analysis software. For example, sequence identity can be evaluated using the online alignment tools provided by EMBL-EBI (https://www.ebi.ac.uk/Tools/psa/). Sequence identity between two sequences can be assessed using the Needleman-Wunsch algorithm with default parameters.

To enable bacteria to survive only under low oxygen conditions, three requirements need to be met:
1)Control over the strength of the upstream promoter and basal leaky expression.

If the oxygen sensor exhibits partial leaky expression even without binding to the upstream promoter, it would disrupt oxygen-dependent regulation, resulting in bacterial survival under all oxygen concentrations; or if the strength of the upstream promoter is too weak to initiate the expression of the downstream gene, the bacteria will fail to survive under any oxygen concentration;

2) Selection of a suitable survival-essential gene.

Through the selection of the survival-essential gene, it can be ensured that the bacteria die when the survival-essential gene is not expressed, while bacterial survival can be rapidly ensured when expression is induced under hypoxia;

3) Upon transition between hypoxia and hyperoxia, the upstream promoter can rapidly initiate transcription to rapidly achieve the synthesis and expression of the survival-essential gene, ensuring that the modified bacteria can survive, whereas under high oxygen conditions, the expression of the survival-essential gene will not be initiated, thereby resulting in bacterial death.

Therefore, in the present invention, a "hypoxia-specific gene expression cassette" refers to a segment of DNA capable of initiating the expression of an essential gene under hypoxic conditions, comprising an essential gene under the control of a hypoxia-inducible promoter, and, if necessary, further comprising other regulatory elements required for the expression of the essential gene.

In the present invention, "essential gene" refers to a gene that plays a decisive role in the growth and/or survival of bacteria; once a bacterium lacks this gene or its functional expression product, it cannot survive, divide, and/or grow normally. A typical example of lacking an essential gene or its functional expression product is an auxotrophic strain, which cannot survive, divide, and/or grow normally under *in vitro* culture conditions or in an *in vivo* environment in the absence of a specific exogenous supplement. Essential genes usually exist as a single copy on the bacterial chromosome.

Accordingly, the requirements for the survival-essential gene are as follows:
1. it is an essential gene for bacterial replication, and its absence results in rapid death of the bacteria;
2. the product of this gene does not exist in the ordinary environment or the human body, which ensures that the bacteria will not escape control in the human body environment, and facilitates the cultivation and preparation of the bacteria in an ordinary culture environment by adding the corresponding expression product of the gene;
3. this gene needs to be rapidly initiated under the control of a hypoxia promoter, and can rapidly synthesize the product to achieve the regulatory function of the host bacteria.

In the present invention, the survival-essential gene is a gene encoding alanine racemase.

For Gram-negative bacteria such as *Escherichia coli* and *Salmonella*, the cell wall is an indispensable component. The core component of the cell wall is peptidoglycan. During the synthesis of peptidoglycan, bacteria require D-alanine as an important constituent part. If D-alanine is absent, the bacteria will be unable to synthesize the cell wall, thereby resulting in lysis.

Amino acids existing in nature are typically L-form amino acids; therefore, two genes exist in Gram-negative bacteria: the *alr* gene and *dadX* gene, which synthesize alanine racemase and are responsible for converting L-alanine into D-alanine to meet the requirements of cell wall synthesis.

Studies have found that if both the *alr* gene and the *dadX* gene are mutated simultaneously, a lethal mutation of *Salmonella* can be achieved, and this mutation can be compensated for by additionally supplementing D-alanine in the culture medium.

In our previous studi*e*s, the *asd* gene was utilized as an essential gene for regulation in YB1 *Salmonella*. Compared to YB1 *Salmonella*, the present invention selects the *alr* gene and the *dadX* gene as survival-essential genes. The *alr* gene and the *dadX* gene *are* functionally homologous genes. Therefore, in the present invention, the alanine racemase gene can be the *Salmonella alr* gene (SEQ ID No. 27) and the *Salmonella alr* protein sequence (SEQ ID No. 28) or the *Salmonella dadX* gene (SEQ ID No. 29) and the *Salmonella dadX* protein sequence (SEQ ID No. 30); or the *alr* or *dadX* gene from other Gram-negative bacteria or genes with equivalent function.

In the present invention, the alanine racemase *alr* gene and *dadX* gene comprise nucleotide sequences having greater than or equal to 81%, preferably greater than or equal to 82%, more preferably greater than or equal to 83%, greater than or equal to 84%, greater than or equal to 85%, greater than or equal to 86%, greater than or equal to 87%, greater than or equal to 88%, greater than or equal to 89%, greater than or equal to 90%, greater than or equal to 91%, greater than or equal to 92%, greater than or equal to 93%, greater than or equal to 94%, greater than or equal to 95%, greater than or equal to 96%, greater than or equal to 97%, greater than or equal to 98%, or most preferably greater than or equal to 99% sequence identity to the specific sequences described above.

In the present invention, first, bacteria with an *alr* gene knockout are constructed, and then the *dadX* gene is modified so that the bacteria become *alr* and *dadX* deficient strains after editing. Simultaneously, a forward hypoxia promoter and a reverse hyperoxia promoter are used to regulate an additional *alr* or *dadX* gene.

The forward hypoxia promoter described in the present invention is a hypoxia-condition promoter regulated by FNR; the reverse hyperoxia promoter described in the present invention is an antisense promoter negatively regulated by FNR and/or ArcA. The fumarate and nitrate reduction gene *fnr* is an important gene regulating the aerobi*c* and anaerobic growth of *Salmonella*. This complex regulatory system has been extensively studied in *Escherichia coli* and *Salmonella*. The DNA-binding protein FNR encoded by the *fnr* gene senses changes in oxygen and controls the expression of different genes, achieving a switch at the overall metabolic level. Therefore, DNA binding sequences such as FNR and ArcA become important modes for controlling downstream gene expression.

FNR possesses an oxygen-sensitive [4Fe-4S]²⁺ domain, which can directly sense oxygen and regulate site-specific DNA binding. In contrast, ArcA senses signals from the aerobic respiratory chain. Thus, two distinct mechanisms exist in Gram-negative facultative anaerobic bacteria such as *Salmonella* and *Escherichia coli* for sensing changes in oxygen concentration. When regulating gene expression under hypoxia, FNR can act through two modes: activation and repression.

To this end, the present invention employs the forward hypoxia promoters *yhbU* and *ynfK*, which activate downstream gene expression via FNR under hypoxic conditions, and the reverse hyperoxia promoters *ydcI* and *cyoA*, which inhibit downstream gene expression via FNR and ArcA under hypoxic conditions.

Wherein, the forward hypoxia promoters applied in the present invention are as follows:
1. *Salmonella yhbU* promoter (yhbU-S) (SEQ ID NO: 31), wherein the FNR binding site is "CTGCCTTAAATCAA";
2. *Escherichia coli ynfK* promoter (ynfK-E) (SEQ ID NO: 32), wherein the FNR binding site is "TTGCGCTATCTCAA";

Wherein, the reverse hyperoxia promoters applied in the present invention are as follows:
1. *Salmonella cyoA* promoter (cyoA-S) (SEQ ID NO: 33), wherein the FNR binding site is "TTTATTGATAATAA", and the ArcA binding site is "GTTAAGTA";
2. *Salmonella ydcI* promoter (ydcI-S) (SEQ ID NO: 34), wherein the FNR binding site is "GTTATCAAAAACAA", and the ArcA binding site is "GTTAATAA";

Through analysis of the aforementioned forward hypoxia promoters and reverse hyperoxia promoters, we found that the FNR binding sites conform to the pattern of "TTGATNNNNATCAA", and wherein any base in the TTGAT and ATCAA sequences of the conserved binding site can be substituted, provided that the total number of substitutions does not exceed 3, and 3 consecutive adjacent bases cannot be substituted; and the ArcA conforms to the pattern of its core region "GTTAATTA", and wherein any base in the GTTAATTA sequence of the conserved binding site can be substituted, provided that the total number of substitutions does not exceed 2.

Thus, in a preferred embodiment of the present invention, the FNR binding site of the forward hypoxia promoter or reverse hyperoxia promoter conforms to the pattern of TTGATNNNNATCAA, wherein N is any base selected from A, T, C, and G, and wherein any base in the TTGAT and ATCAA sequences of the conserved binding site can be substituted, provided that the total number of substitutions does not exceed 3, and 3 consecutive adjacent bases cannot be substituted; the ArcA binding site of the reverse hyperoxia promoter conforms to the pattern of GTTAATTA, wherein any base can be substituted, provided that the total number of substitutions does not exceed 2.

In a more preferred embodiment of the present invention, the forward hypoxia promoter is selected from *yhbU* or *ynfK*; the survival-essential gene is selected from *alr* or *dadX*; the reverse hyperoxia promoter is selected from *cyoA* or *ydcI*.

In a preferred embodiment of the present invention, the expression cassette consists of the forward hypoxia promoter *yhbU*, the survival-essential gene *alr*, and the reverse hyperoxia promoter *cyoA*.

In a preferred embodiment of the present invention, the expression cassette consists of the forward hypoxia promoter *yhbU*, the survival-essential gene *alr*, and the reverse hyperoxia promoter *ydcI*.

In a preferred embodiment of the present invention, the expression cassette consists of the forward hypoxia promoter *ynfK*, the survival-essential gene *dadX*, and the reverse hyperoxia promoter *cyoA*.

In a preferred embodiment of the present invention, the expression cassette consists of the forward hypoxia promoter *ynfK*, the survival-essential gene *dadX*, and the reverse hyperoxia promoter *ydcI*.

The forward hypoxia promoter disclosed in patent CN104471057B is *pepT*. The *pepT* promoter is not completely regulated by FNR, but is a promoter dually regulated by CRP-cAMP and FNR. Therefore, the FNR binding site in the *pepT* promoter is half a CRP binding site and half an FNR binding site. The CRP-FNR binding region sequence of the *pepT* promoter is GTGACCTGACGCAA, wherein the first half, GTGA, conforms to the first half of the CRP conserved binding site GTGANNNNNNTCAC, and the latter half, CGCAA, conforms to a part of the FNR conserved region ATCAA, wherein the tenth position A is substituted by C, and the eleventh position T is substituted by G, and thus it does not conform to the rules for the forward hypoxia promoter of the present invention.

The FNR binding region of the reverse hyperoxia promoter *sodA* disclosed in patent CN104471057B, TTGATAATCATTTT, only contains the first half-region of FNR, TTGAT, and the latter half-region contains three consecutive substitutions, with ATCAA being substituted by ATTTT; therefore, it does not comprise a complete FNR binding site. The ArcA binding site comprised therein is TTTAATTA, which has the 'G' at the first position substituted by 'T' when compared to the conserved core ArcA binding site 5'-GTTAATTA-3'. Therefore, the *sodA* promoter only comprises a single ArcA binding site, and its FNR binding site is incomplete, and thus it does not conform to the rules for the reverse hyperoxia promoter in the present invention.

In summary, the present invention obtains a more efficient and safer hypoxia-specific gene expression cassette by screening appropriate forward hypoxia promoters and reverse hyperoxia promoters, in combination with suitable survival-essential genes.

In a preferred embodiment of the present invention, the hypoxia-specific gene expression cassette is regulated by oxygen concentration.

To enable Gram-negative bacteria such as *Salmonella* to survive in hypoxic regions, a 1% oxygen concentration is a very important key point, as it represents pathological hypoxia. This is because an oxygen concentration below 1% is a clear hallmark of a tumor hypoxic region, and regions with an oxygen concentration below 1% do not exist in normal organ tissues. For example, the oxygen concentration in the hypoxic regions of various tumors, such as pancreatic cancer, cervical cancer, and prostate cancer, is lower than 0.7%.

The hypoxia-sp*e*cific gene expression cassette designed by the present invention enables Gram-negative bacteria such as *Salmonella* to recognize tumor hypoxic regions and proliferate therein through precise oxygen regulation. The objective achieved by the present invention is that when the oxygen concentration is lower than 0.8%, the modified Gram-negative bacteria such as *Salmonella* can survive and proliferate, and achieve suicide lysis in a normal oxygen concentration environment.

Thus, in a more preferred embodiment of the present invention, the forward hypoxia promoter functions when the oxygen content is lower than 1%, and does not function when the oxygen content is higher than 1%; and/or

the reverse hyperoxia promoter functions when the oxygen content is higher than 1%, and does not function when the oxygen content is lower than 1%.

In a further preferred embodiment of the present invention, the forward hypoxia promoter functions when the oxygen content is lower than 0.8%, and does not function when the oxygen content is higher than 0.8%; and/or

the reverse hyperoxia promoter functions when the oxygen content is higher than 0.8%, and does not function when the oxygen content is lower than 0.8%.

In a preferred embodiment of the present invention, among the dual bacteriophage-derived promoters, one is sequentially linked to a ribosomal binding site (RBS) (SEQ ID NO: 35) and the membrane-disrupting protein genes, and the other is sequentially linked to an IRES sequence, a Kozak sequence, the open reading frame (ORF) region of a eukaryotic therapeutic, a 3'-UTR region, and a sequentially expressed IRES sequence, Kozak sequence, ORF region of a specific bacteriophage RNA polymerase gene, a polyA tail, and a T7 terminator sequence.

In a preferred embodiment of the present invention, the origin of replication of the expression vector of the present invention is a high-copy replicon pUC (SEQ ID NO: 36), or a low-copy replicon p15A, ColE1, or R6K.

Meanwhile, the expression vector of the present invention does not comprise a resistance gene.

In a preferred embodiment of the present invention, the host bacteria of the expression vector are Gram-negative bacteria.

In the present invention, the term "Gram-negative bacteria" refers to bacteria that do not retain the initial basic dye stain (e.g., crystal violet) after the known partial procedure of Gram staining. In an exemplary Gram staining, cells are first fixed on a slide by heating and stained with a basic dye (e.g., crystal violet), the basic dye being absorbed by both Gram-negative and Gram-positive bacteria. Then, the slide is treated with a mordant (e.g., Gram's iodine), which binds to the basic dye (e.g., crystal violet) and traps it within the cells. The cells are then washed with acetone or alcohol and counterstained with a second dye of different color (e.g., safranin). Gram-positive organisms retain the initial purple stain, whereas Gram-negative organisms are decolorized by the organic solvent wash and thus show the counterstain. Exemplary Gram-negative bacteria include, but are not limited to, *Escherichia* spp., *Shigella* spp., *Salmonella* spp., *Campylobacter* spp., *Neisseria* spp., *Haemophilus* spp., *Aeromonas* spp., *Francisella* spp., *Yersinia* spp., *Klebsiella* spp., *Bordetella* spp., *Legionella* spp., *Corynebacterium* spp., *Citrobacter* spp., *Chlamydia* spp., *Brucella* spp., *Pseudomonas* spp., *Helicobacter* spp., and *Vibrio* spp.

Thus, in a more preferred embodiment of the present invention*,* the Gram-negative bacterium is selected from the group consisting of *Salmonella*, *Escherichia coli*, *Shigella*, *Yersinia*, *Enterobacter cloacae*, *Cronobacter*, *Klebsiella*, *Pantoea*, *Serratia*, *Shimwellia*, *Enterobacter ludwigii*, *Haemophilus*, *Vibrio*, *Pseudomonas*, *Pasteurella*, *Bordetella*, *Bordetella pertussis*, *Acinetobacter baumannii*, *Burkholderia*, *Vibrio vulnificus*, *Bacteroides fragilis*, *Pseudomonas syringae*, *Pseudomonas putida*, *Legionella*, *Klebsiella pneumoniae*, *Vibrio parahaemolyticus*, *Vibrio cholerae*, *Yersinia pestis*, *Catarrhalis coccus*, *Moraxella catarrhalis*, *Campylobacter jejuni*, *Shigella dysenteriae*, *Neisseria gonorrhoeae*, *Haemophilus influenzae*, *Moraxella*, *Neisseria meningitidis*, *Proteus vulgaris*, *Proteus mirabilis*, *Pasteurella haemolytica*, *Salmonella enterica*, *Salmonella bongori*, *Salmonella paratyphi*, *Salmonella typhi*, *Legionella pneumophila*, *Yersinia pestis*, *Shigella sonnei*, *Pseudomonas aeruginosa*, *Yersinia enterocolitica*, *Cryptococcus neoformans*, *Burkholderia cenocepacia*, and *Helicobacter pylori*.

In the present invention, the term "live bacteria" refers to a bacterial strain that is viable, possesses active nutritional metabolic characteristics, and is capable of exercising its own biological functions. Live bacteria may include bacterial biomass generated during the metabolic processes of the strain.

In a more preferred embodiment of the present invention, the Gram-negative bacterium is *Salmonella*.

In another aspect, the present invention provides a method for a constitutive eukaryotic therapeutic mRNA/DNA mixed dual-delivery cytoplasmic expression system comprising dual bacteriophage-derived promoters, comprising the following steps:
1. Sequentially linking the bacteriophage-derived promoter of the present invention, a ribosomal binding site, and the membrane-disrupting protein genes, to construct a membrane-disrupting protein gene expression unit;
2. Sequentially linking the bacteriophage-derived promoter of the present invention, an IRES sequence, a Kozak sequence, the ORF region of a eukaryotic therapeutic, a 3'-UTR region, and a sequentially expressed IRES sequence, Kozak sequence, ORF region of a specific bacteriophage RNA polymerase gene, a polyA tail, and a T7 terminator sequence, to construct a therapeutic eukaryotic protein gene transcription unit and a self-amplifying unit within the eukaryotic cytoplasm.

In another aspect, the present invention provides a method for controlling the expression of therapeutic mRNA in a prokaryotic cell using the expression vector of the present invention, comprising the following steps:
1. Preparing the expression vector of the present invention;
2. Integrating a constitutive expression promoter that controls the expression of a bacteriophage RNA polymerase gene into the chromosome of a Gram-negative bacterium;
3. Transforming the expression vector of the present invention into the Gram-negative bacterium.

When the expression vector of the present invention is transformed into a Gram-negative bacterium (e.g., *Salmonella typhimurium*) to serve as a therapeutic bacterium, the high expression of the therapeutic protein gene and membrane-disrupting protein gene controlled by the specific bacteriophage RNA polymerase integrated into the bacterial chromosome and the dual bacteriophage-derived promoters can lead to increased bacterial toxicity. Therefore, to ensure safety for use as a therapeutic bacterium, the aforementioned bacteria integrated with the expression vector of the present invention need to be modified by one or more attenuation methods. Attenuation methods include, but are not limited to, hypoxia-specific gene expression cassette regulatory systems, auxotrophy, stress response defects, and virulence island regulation defects.

Therefore, in a preferred embodiment of the present invention, the Gram-negative bacterium is an attenuated Gram-negative bacterium.

In a more preferred embodiment of the present invention, the attenuated Gram-negative bacterium is *Salmonella*.

In a more preferred embodiment of the present invention, the attenuation method is selected from *Salmonella aroA* gene deficiency, and hypoxia-specific gene expression cassette regulatory system.

In another aspect, the present invention provides a modified Gram-negative bacterium, which comprises the expression vector described herein.

In a more preferred embodiment of the present invention*,* the Gram-negative bacterium is selected from the group consisting of *Salmonella, Escherichia coli, Shigella, Yersinia, Enterobacter cloacae, Cronobacter, Klebsiella*, *Pantoea*, *Serratia*, *Shimwellia*, *Enterobacter ludwigii*, *Haemophilus*, *Vibrio*, *Pseudomonas*, *Pasteurella*, *Bordetella*, *Bordetella pertussis*, *Acinetobacter baumannii*, *Burkholderia*, *Vibrio vulnificus*, *Bacteroides fragilis*, *Pseudomonas syringae*, *Pseudomonas putida*, *Legionella*, *Klebsiella pneumoniae*, *Vibrio parahaemolyticus*, *Vibrio cholerae*, *Yersinia pestis*, *Catarrhalis coccus*, *Moraxella catarrhalis*, *Campylobacter jejuni*, *Shigella dysenteriae*, *Neisseria gonorrhoeae*, *Haemophilus influenzae*, *Moraxella*, *Neisseria meningitidis*, *Proteus vulgaris*, *Proteus mirabilis*, *Pasteurella haemolytica*, *Legionella pneumophila*, *Yersinia pestis*, *Shigella sonnei*, *Pseudomonas aeruginosa*, *Yersinia enterocolitica*, *Cryptococcus neoformans*, *Burkholderia cenocepacia*, and *Helicobacter pylori*.

In a more preferred embodiment of the present invention, the Gram-negative bacterium is *Salmonella*.

Another aspect of the present invention provides the use of the expression vector of the present invention or the Gram-negative bacterium of the present invention in the preparation of tumor drugs.

In a preferred embodiment of the present invention, the tumor is a solid tumor.

The term "solid tumor" as used in the present invention refers to an abnormal mass of tissue, which usually does not contain cysts or liquid areas. Solid tumors may be benign (non-cancerous) or malignant (cancerous). Different types of malignant solid tumors are named according to the type of cells from which they form. Examples of malignant solid tumors are sarcomas, carcinomas, and lymphomas. Leukemias (blood cancers) generally do not form malignant solid tumors. Malignant solid tumors include, but are not limited to, abnormal cell masses that may originate from different tissue types, such as liver, colon, colorectal, skin, breast, pancreas, cervix, uterine body, bladder, gallbladder, kidney, larynx, lip, oral cavity, esophagus, ovary, prostate, stomach, testis, thyroid, or lung, etc.; therefore, malignant solid tumors include malignant solid liver tumors, colon tumors, colorectal tumors, skin tumors, breast tumors, pancreatic tumors, cervical tumors, uterine body tumors, bladder tumors, gallbladder tumors, kidney tumors, laryngeal tumors, lip tumors, oral cavity tumors, esophageal tumors, ovarian tumors, prostate tumors, stomach tumors, testicular tumors, thyroid tumors, or lung tumors, etc.

Thus, in a more preferred embodiment of the present invention, the solid tumor is selected from the group consisting of tumors/cancers of the breast, bone, liver, lung, skin, kidney, stomach, pancreas, prostate, lymph (non-Hodgkin's lymphoma, Hodgkin's lymphoma), intestine (colon cancer, rectal cancer), pelvis (cervical cancer, ovarian malignancy, endometrial cancer, ovarian cancer), nervous system, head and neck, and bladder.

In a further preferred embodiment of the present invention, the solid tumor is breast cancer, osteosarcoma, liver cancer, lung cancer, melanoma, kidney cancer, stomach cancer, pancreatic cancer, prostate cancer, colon cancer, ovarian cancer, neuroblastoma, squamous cell carcinoma, and bladder cancer.

In the present invention, for therapeutic purposes, the term "subject" preferably refers to a subject in need of treatment for a target pathological condition such as a tumor. For prevention purposes, the subject is preferably a subject at risk of developing a target pathological condition or predisposed to developing a target pathological condition. The term "subject" includes living organisms, such as prokaryotes and eukaryotes. Examples of subjects include mammals, such as humans, dogs, cows, horses, pigs, sheep, goats, cats, mice, rabbits, hedgehogs, rats, and transgenic non-human animals. In a specific embodiment of the present invention, the subject is a human.

As used in the present invention, "treatment" is a process used to obtain beneficial or desired clinical results. For the purposes of the present invention, beneficial or desired clinical results include, but are not limited to, one or more of the following: reducing the proliferation of neoplastic or cancerous cells (or destroying neoplastic or cancerous cells), inhibiting metastasis of neoplastic cells, shrinking or reducing tumor size, alleviating malignancy, alleviating symptoms caused by malignancy, improving the quality of life of subjects suffering from malignancy, reducing the dose of other drugs required to treat malignancy, delaying the progression of malignancy, curing malignancy, and/or prolonging the survival of patients suffering from malignancy.

As used in the present invention, an "effective amount" or "effective dose" of a bacterium, drug, or pharmaceutical composition is an amount sufficient to achieve any one or more beneficial or desired results. For prophylactic use, beneficial or desired results include eliminating or reducing the risk of disease, reducing the severity of disease, or delaying the onset of disease, including biochemical, histological, and/or behavioral symptoms of the disease, its complications, and intermediate pathological phenotypes presenting during the development of the disease. For therapeutic use, beneficial or desired results include such as alleviating one or more symptoms of a disease (such as tumor), reducing the dose of other drugs required to treat the disease, enhancing the effect of another drug, prolonging the survival of the treated subject, and/or delaying the progression of cancer in patients. For example, compared to untreated subject, an "effective amount" preferably inhibits cell growth or tumor growth by at least about 10%, preferably at least about 20%, more preferably at least about 30%, more preferably at least about 40%, more preferably at least about 50%, more preferably at least about 60%, more preferably at least about 70%, more preferably at least about 80%. The ability to inhibit tumor growth can be evaluated in animal model systems predictive of efficacy against human tumors. Alternatively, it can also be evaluated by examining the ability to inhibit cell growth, which can be determined *in vitro* by assays well known to those skilled in the art. A therapeutically effective amount of a therapeutic compound can reduce tumor size or otherwise alleviating symptoms in a subject. Those skilled in the art can determine such amounts based on factors such as the size of the subject, the severity of the subject's symptoms, and the specific composition or route of administration selected.

In some embodiments of the present invention, the effective amount of bacteria in the bacterial agent, drug, or pharmaceutical composition comprises at least about 10⁴ colony-forming units (cfu), for example, at least about 10⁴, 10⁵, 10⁶, 10⁷, 10⁸, 10⁹, 10¹⁰, 10¹¹, 10¹², or 10¹³ cfu, including any ranges between the specified values, such as 10⁴-10⁸cfu, 10⁴-10⁹cfu, 10⁴-10¹⁰cfu, 10⁴-10¹¹cfu, 10⁴-10¹²cfu, 10⁴-10¹³cfu, 10⁵-10⁸cfu, 10⁵-10⁹cfu, 10⁵-10¹⁰cfu, 10⁵-10¹¹cfu, 10⁵-10¹²cfu, 10⁵-10¹³cfu, 10⁶-10⁸cfu, 10⁶-10⁹cfu, 10⁶-10¹⁰cfu, 10⁶-10¹¹cfu, 10⁶-10¹²cfu, 10⁶-10¹³cfu, 10⁷-10⁸cfu, 10⁷-10⁹cfu, 10⁷-10¹⁰cfu, 10⁷-10¹¹cfu, 10⁷-10¹²cfu, 10⁷-10¹³cfu, 10⁸-10⁹cfu, 10⁸-10¹⁰cfu, 10⁸-10¹¹cfu, 10⁸-10¹²cfu or 10⁸-10¹³cfu.

In some embodiments of the present invention, the volume of the effective dose of the bacteria, drug, or pharmaceutical composition of the present invention is less than or equal to about 3 mL, about 2.5 mL, about 2 mL, about 1.5 mL, about 1 mL, about 0.75 mL, about 0.5 mL, about 0.25 mL, or about 0.1 mL. In some embodiments, the volume of the effective dose of the bacteria, drug, or pharmaceutical composition is about 20 mL, about 19 mL, about 18 mL, about 17 mL, about 16 mL, about 15 mL, about 14 mL, about 13 mL, about 12 mL, about 11 mL, about 10 mL, about 9 mL, about 8 mL, about 7 mL, about 6 mL, about 5 mL, about 4 mL, about 3 mL, about 2 mL, or about 1 mL. Alternatively, the volume of the effective dose of the bacteria, drug, or pharmaceutical composition is about 20.5 mL, about 19.5 mL, about 18.5 mL, about 17.5 mL, about 16.5 mL, about 15.5 mL, about 14.5 mL, about 13.5 mL, about 12.5 mL, about 11.5 mL, about 10.5 mL, about 9.5 mL, about 8.5 mL, about 7.5 mL, about 6.5 mL, about 5.5 mL, about 4.5 mL, about 3.5 mL, about 2.5 mL, about 1.5 mL, or about 0.5 mL. In some embodiments, the volume of the effective dose of the bacteria, drug, or pharmaceutical composition is about 200 mL, about 190 mL, about 180 mL, about 170 mL, about 160 mL, about 150 mL, about 140 mL, about 130 mL, about 120 mL, about 110 mL, about 100 mL, about 90 mL, about 80 mL, about 70 mL, about 60 mL, about 50 mL, about 40 mL, or about 30 mL, as well as ranges between any of the listed values, such as 100 mL-110 mL, 100 mL-120 mL, 90 mL-120 mL, 90 mL-130 mL, etc. Alternatively, the volume of the effective dose of the bacteria, drug, or pharmaceutical composition is about 205 mL, about 195 mL, about 185 mL, about 175 mL, about 165 mL, about 155 mL, about 145 mL, about 135 mL, about 125 mL, about 115 mL, about 105 mL, about 95 mL, about 85 mL, about 75 mL, about 65 mL, about 55 mL, about 45 mL, about 35 mL, or about 25 mL, as well as ranges between any of the listed values, such as 105 mL-115 mL, 105 mL-125 mL, 95 mL-125 mL, 95 mL-135 mL, etc. Alternatively, the volume of the effective dose of the bacteria, drug, or pharmaceutical composition is about 900 microliters, about 800 microliters, about 700 microliters, about 600 microliters, about 500 microliters, about 400 microliters, about 300 microliters, about 200 microliters, or about 100 microliters; alternatively about 950 microliters, about 850 microliters, about 750 microliters, about 650 microliters, about 550 microliters, about 450 microliters, about 350 microliters, about 250 microliters, about 150 microliters, or about 50 microliters.

In some embodiments, the volume of the effective dose of the bacteria, drug, or pharmaceutical composition is less than or equal to about 2.0 mL.

The dosage ranges discussed herein are merely exemplary and do not limit the dosage ranges that may be selected by a physician. The amount of the active ingredient (e.g., the bacteria of the present invention) in the pharmaceutical composition of the present invention may vary depending on factors such as the disease state, age, gender, and weight of the individual, including the presence or absence of a tumor, the type of tumor being treated, the severity of the tumor, the activity or viability of the bacteria, drug, or pharmaceutical composition, the route of administration, the duration of treatment, the drugs used in combination with the bacteria, drug, or pharmaceutical composition (if any), the diet and general health of the subject, and similar factors well known in the art. The dosage regimen may be adjusted to provide the optimum therapeutic response. For example, it may be administered as a single dose, several divided doses may be administered over time, or the dose may be proportionally reduced or increased as indicated by the exigencies of the therapeutic situation.

As used herein, "pharmaceutically acceptable carrier" includes any material which, when combined with the active ingredient, allows the ingredient to retain its biological activity and does not react with the subject's immune system, including but not limited to disintegrants, binders, fillers, buffers, tonicity agents, stabilizers, antioxidants, surfactants, and lubricants. Preferably, the carrier is suitable for intravenous, intramuscular, subcutaneous, parenteral, spinal, or epidermal administration (e.g., by injection or infusion). For example, depending on the route of administration, the bacteria of the present invention may be coated in a material to protect the bacteria from acidic and other natural conditions that could inactivate them. Pharmaceutically acceptable carriers include physiological saline, PBS buffer, sterile aqueous solutions or dispersions, and powders for the extemporaneous preparation of injectable solutions or dispersions. The use of such media and agents for pharmaceutically active substances is well known in the art. Conventional media or agents except those incompatible with the active compound may be present in the pharmaceutical compositions of the present invention.

Accordingly, another aspect of the present invention provides a pharmaceutical composition comprising an effective amount of the expression vector of the present invention or the modified bacteria of the present invention. In one embodiment, the modified bacterium is a live bacterium.

In a preferred embodiment of the present invention, the pharmaceutical composition further comprises a pharmaceutically acceptable carrier.

In a more preferred embodiment of the present invention, the pharmaceutically acceptable carrier is selected from disintegrants, binders, fillers, buffers, tonicity agents, stabilizers, antioxidants, surfactants, and lubricants.

In a preferred embodiment of the present invention, the pharmaceutical composition is used for treating solid tumors. The bacteria, drug, or pharmaceutical composition of the present invention is administered via the following routes: intravenous injection, intratumoral injection, intramuscular injection, subcutaneous injection, intraperitoneal injection, intracerebral administration, gastrointestinal administration, topical administration, buccal administration, nasal administration, rectal administration, or vaginal administration.

In a preferred embodiment of the present invention, the bacteria, drug, or pharmaceutical composition of the present invention can be formulated into a form suitable for administration via the following routes: intravenous injection, intratumoral injection, intramuscular injection, subcutaneous injection, intraperitoneal injection, intracerebral administration, gastrointestinal administration, topical administration, buccal administration, nasal administration, rectal administration, or vaginal administration.

The dosage forms of the drug or pharmaceutical composition of the present invention may be in the form of solutions, emulsions, lyophilized formulation, or suspensions; for oral administration, the dosage form may be in the form of tablets or capsules; for intranasal administration, the dosage form may be in the form of powders, nasal drops, or aerosols; for topical administration, the dosage form may be aqueous solutions, suspensions, ointments, creams, or gels; for rectal or vaginal administration, the dosage form may be suppositories, enemas, or delivered as part of an endoscopic or colonoscopic procedure.

The bacteria, drug, or pharmaceutical composition of the present invention can be manufactured by methods well known in the art, such as microbial growth in a fermenter, followed by centrifugal concentration and washing, filtration or dialysis, conventional granulation, mixing, dissolving, encapsulation, lyophilization, or emulsification processes, and other methods. The bacteria, drug, or pharmaceutical composition of the present invention can be produced in various forms, including granules, precipitates or particulates, powders (including freeze-dried, rotary-dried, or spray-dried powders), amorphous powders, injectables, emulsions, elixirs, suspensions, or solutions. The formulation may optionally contain stabilizers, pH adjusters, surfactants, bioavailability modifiers, and combinations thereof.

The bacteria, drug, or pharmaceutical composition of the present invention can be administered alone or in combination with other compounds or compositions in the presence of a carrier. In a preferred embodiment of the present invention, the bacteria, drug, or pharmaceutical composition can be administered in combination with other malignant tumor therapies (including but not limited to radiotherapy, chemotherapy, and surgery). The bacteria, drug, or pharmaceutical composition can be used as an adjuvant in therapy in such cases.

In another aspect, the present invention provides a method for treating a tumor, comprising administering to a subject the eukaryotic expression vector in the present invention, the Gram-negative bacteria described in the present invention, or the pharmaceutical composition described in the present invention.

In one embodiment, the eukaryotic expression vector, Gram-negative bacteria, or pharmaceutical composition is used for treating a tumor. In one embodiment, the eukaryotic expression vector, Gram-negative bacteria, or pharmaceutical composition is used for inducing an anti-tumor specific immune response in a subject having a tumor. In one embodiment, the eukaryotic expression vector, Gram-negative bacteria, or pharmaceutical composition is used for inducing anti-tumor immune memory in a subject having a tumor. In one embodiment, the eukaryotic expression vector, Gram-negative bacteria, or pharmaceutical composition is used for preventing or treating metastasis or recurrence of a tumor. In one embodiment, the eukaryotic expression vector, Gram-negative bacterium, or pharmaceutical composition is used for treating a tumor that has developed resistance to prior anti-tumor treatments or where treatment has failed.

In one embodiment of the present invention, the tumor is a tumor of the nervous system, respiratory system, digestive system, urinary system, reproductive system, hematopoietic system, lymphatic system, endocrine system, or skin and mucous membranes. In one embodiment, the tumor is a sarcoma or carcinoma. In one embodiment, the tumor is a solid tumor.

In a further preferred embodiment of the present invention, the solid tumor is selected from tumors/cancers of the breast, bone, liver, lung, skin, kidney, stomach, pancreas, prostate, lymph (non-Hodgkin's lymphoma, Hodgkin's lymphoma), intestine (colon cancer, rectal cancer), pelvis (cervical cancer, ovarian malignancy, endometrial cancer, ovarian cancer), nervous system, head and neck cancer, and bladder, etc.

In a more preferred embodiment of the present invention, the solid tumor is breast cancer, osteosarcoma, liver cancer, lung cancer, melanoma, kidney cancer, gastric cancer, pancreatic cancer, prostate cancer, colon cancer, ovarian cancer, neuroblastoma, squamous cell carcinoma, and bladder cancer.

The method for treating a tumor provided by the present invention comprises administering an effective amount of the eukaryotic expression vector, Gram-negative bacteria, or pharmaceutical composition of the present invention to a subject having a tumor.

In an embodiment of the present invention, the eukaryotic expression vector, Gram-negative bacteria, or pharmaceutical composition of the present invention may be administered via the following routes: intravenous injection, intratumoral injection, intramuscular injection, subcutaneous injection, intraperitoneal injection, intracerebral administration, gastrointestinal administration, topical administration, buccal administration, nasal administration, rectal administration, or vaginal administration.

In some embodiments of the present invention, the eukaryotic expression vector, Gram-negative bacteria, or pharmaceutical composition of the present invention may be administered at intervals of, for example, about 1 minute, 2, 3, 4, 5, 6, 7, 8, 9, 10, 15, 20, 25, 30, 35, 40, 45, 50, 55 minutes, 1 hour, 2, 3, 4, 5, 6, 7, 8, 9, 10, 11, 12, 13, 14, 15, 16, 17, 18, 19, 20, 21, 22, 23 hours, 1 day, 2, 3, 4, 5, 6, 7, 8, 9, or 10 days, including ranges between any two of the listed values, for example, 1 minute-10 minutes, 1 minute-30 minutes, 1 minute-1 hour, 1 minute-2 hours, 1 minute-4 hours, 1 minute-12 hours, 1 minute-18 hours, 1 minute-1 day, 10 minutes-30 minutes, 10 minutes-1 hour, 10 minutes-2 hours, 10 minutes-4 hours, 10 minutes-12 hours, 10 minutes-18 hours, 10 minutes-1 day, 30 minutes-1 hour, 30 minutes-2 hours, 30 minutes-4 hours, 30 minutes-12 hours, 30 minutes-18 hours, 30 minutes-1 day, 30 minutes-2 days, 1 hour-2 hours, 1 hour-4 hours, 1 hour-12 hours, 1 hour-18 hours, 1 hour-1 day, 4 hours-12 hours, 4 hours-18 hours, 4 hours-1 day, 1 day-2 days, 1 day-3 days, 1 day-4 days, 1 day-5 days, 1 day-7 days, 1 day-10 days, 2 days-3 days, 2 days-4 days, 2 days-5 days, 2 days-7 days, 2 days-10 days, or 5 days-10 days. In some embodiments, the eukaryotic expression vector, Gram-negative bacteria, or pharmaceutical composition is administered once a week, once every two weeks, once every three weeks, once every four weeks, once every five weeks, once every six weeks, once every seven weeks, once every eight weeks, once every nine weeks, once every ten weeks, once every fifteen weeks, once every twenty weeks, once every twenty-five weeks, or once every twenty-six weeks. In some embodiments, the eukaryotic expression vector, Gram-negative bacteria, or pharmaceutical composition is formulated in a form that, for example, can be administered at intervals of about 1 minute, 2, 3, 4, 5, 6, 7, 8, 9, 10, 15, 20, 25, 30, 35, 40, 45, 50, 55 minutes, 1 hour, 2, 3, 4, 5, 6, 7, 8, 9, 10, 11, 12, 13, 14, 15, 16, 17, 18, 19, 20, 21, 22, 23 hours, 1 day, 2, 3, 4, 5, 6, 7, 8, 9, or 10 days, including ranges between any two of the listed values, for example, 1 minute-10 minutes, 1 minute-30 minutes, 1 minute-1 hour, 1 minute-2 hours, 1 minute-4 hours, 1 minute-12 hours, 1 minute-18 hours, 1 minute-1 day, 10 minutes-30 minutes, 10 minutes-1 hour, 10 minutes-2 hours, 10 minutes-4 hours, 10 minutes-12 hours, 10 minutes-18 hours, 10 minutes-1 day, 30 minutes-1 hour, 30 minutes-2 hours, 30 minutes-4 hours, 30 minutes-12 hours, 30 minutes-18 hours, 30 minutes-1 day, 30 minutes-2 days, 1 hour-2 hours, 1 hour-4 hours, 1 hour-12 hours, 1 hour-18 hours, 1 hour-1 day, 4 hours-12 hours, 4 hours-18 hours, 4 hours-1 day, 1 day-2 days, 1 day-3 days, 1 day-4 days, 1 day-5 days, 1 day-7 days, 1 day-10 days, 2 days-3 days, 2 days-4 days, 2 days-5 days, 2 days-7 days, 2 days-10 days, or 5 days-10 days. In some embodiments, the expression vector, bacterium, drug, or pharmaceutical composition is formulated in a form to be administered once a week, once every two weeks, once every three weeks, once every four weeks, once every five weeks, once every six weeks, once every seven weeks, once every eight weeks, once every nine weeks, once every ten weeks, once every fifteen weeks, once every twenty weeks, once every twenty-five weeks, or once every twenty-six weeks. The dosage regimen may depend on the pattern of pharmacokinetic decay expected by the medical practitioner. The progress of the therapy can be monitored by conventional techniques and assays. The administration regimen may change over time.

The present invention also provides a method for inducing an anti-tumor specific immune response in a subject having a tumor, comprising administering to the subject having a tumor an effective amount of the eukaryotic expression vector, Gram-negative bacteria, or pharmaceutical composition of the present invention.

The present invention also provides a method for inducing anti-tumor immune memory in a subject having a tumor, comprising administering to the subject having a tumor an effective amount of the eukaryotic expression vector, Gram-negative bacteria, or pharmaceutical composition of the present invention.

The present invention also provides a method for preventing or treating metastasis or recurrence of a tumor, comprising administering to a subject having a tumor an effective amount of the eukaryotic expression vector, Gram-negative bacteria, or pharmaceutical composition of the present invention.

The present invention also provides a method for preventing or treating metastasis or recurrence of a tumor, comprising administering an effective amount of the eukaryotic expression vector, Gram-negative bacteria, or pharmaceutical composition of the present invention to a subject having tumor metastasis or recurrence or a subject at high risk of tumor metastasis or recurrence.

The present invention also provides a method for treating a tumor that has developed resistance to prior anti-tumor treatments or where treatment has failed, comprising administering an effective amount of the eukaryotic expression vector, Gram-negative bacteria, or pharmaceutical composition of the present invention to a subject having a tumor that has developed resistance to prior anti-tumor treatments or where treatment has failed.

By adopting the above technical solutions, the present invention achieves the following beneficial effects:
1. The present invention, by integrating a bacteriophage-derived promoter with a specific bacteriophage RNA polymerase gene to enable their coordinated action, and utilizing a plasmid-based expression platform, achieves stable and efficient expression of therapeutic proteins specifically within tumor regions.
2. The present invention, by combining a bacteriophage-derived promoter with membrane-disrupting protein genes and a eukaryotic therapeutic gene mRNA/DNA mixed vector, enables the three components to act synergistically to achieve stable and highly efficient drug delivery, realizing a true cytoplasmic expression system, and thereby achieving highly efficient killing of tumor cells.
3. The present invention, through the rational design of a hypoxia-specific gene expression cassette, results in modified bacteria with a low overall mutation rate, achieving specific distribution within tumors, and exhibiting faster clearance from normal organs.

### Brief Description of the Drawings

Figure 1 Verification diagram of the forward hypoxia promoter *yhbU-S* and *ynfK-S* clone identified by agarose gel electrophoresis.
Figure 2 Amplification diagram of verification of essential gene library identified by agarose gel electrophoresis.
Figure 3 Verification diagram of the reverse hyperoxia promoter *cyoA-S* and *ydcI-S* clone library identified by agarose gel electrophoresis.
Figure 4 Schematic diagram of a hypoxia-specific gene expression cassette.
Figure 5 Schematic diagram of lambda RED recombinase and CRE recombinase systems.
Figure 6 Identification diagram of the *aroA* gene knockout in *Salmonella* by experiments.
   (A) Verification diagram of PCR-amplified product of the *aroA* gene knockout fragment identified by agarose gel electrophoresis;
   (B) Verification diagram of PCR-amplification product of target fragment in strain SWT003 identified by agarose gel electrophoresis.
Figure 7 Identification diagram of the *alr* gene knockout in *Salmonella* by experiments.
   (A) Verification diagram of PCR-amplified product of the *alr* gene knockout fragment identified by agarose gel electrophoresis;
   (B) Verification diagram of PCR-amplification product of target fragment in strain SWT004 identified by agarose gel electrophoresis.
Figure 8 Verification diagram of strain SWT1001 and SWT1005 with insertion of hypoxia-specific gene expression cassette (*alr* as an essential gene) identified by agarose gel electrophoresis.
Figure 9 Verification diagram of PCR-amplified products of strain SWT007 identified by agarose gel electrophoresis.
Figure 10 Verification diagram of strain SWT2009 and SWT2013 with insertion of hypoxia-specific gene expression cassette (*dadX* as an essential gene) identified by agarose gel electrophoresis.
Figure 11 Results diagram of oxygen adaptability validation tests for *Salmonella* strain SWT1001.
   (A) Anaerobic culture on LB plates without D-alanine;
   (B) Aerobic culture on LB plates without D-alanine.
Figure 12 Results diagram of oxygen adaptability validation tests for *Salmonella* strain SWT1005.
   (A) Anaerobic culture on LB plates without D-alanine;
   (B) Aerobic culture on LB plates without D-alanine.
Figure 13 Results diagram of oxygen adaptability validation tests for *Salmonella* strains SWT2009 and SWT2013.
   (A) SWT2009 cultured on LB plates without D-alanine, the upper part shows culture under anaerobic conditions, the lower part shows culture under aerobic conditions;
   (B) SWT2013 cultured on LB plates without D-alanine, the upper part shows culture under anaerobic conditions, the lower part shows culture under aerobic conditions.

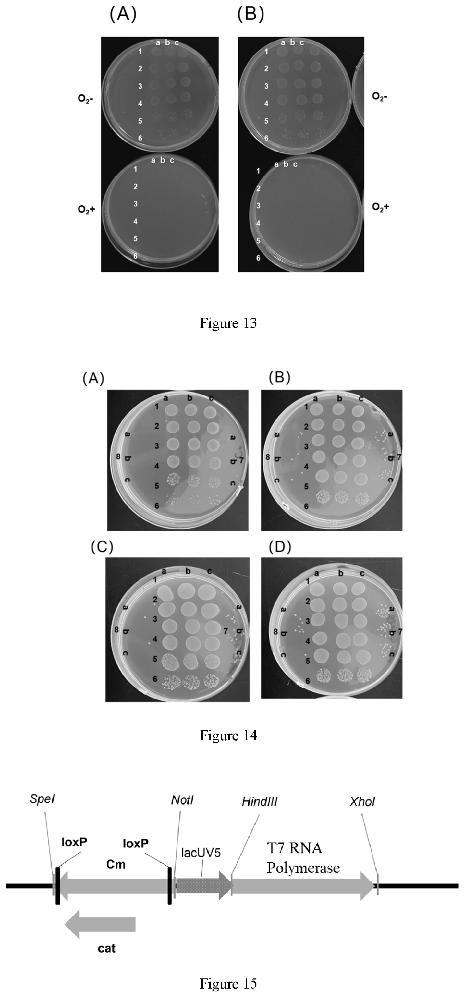

Figure 14 Results diagram of oxygen adaptability validation tests for various *Salmonella* strains under oxygen concentrations below 0.8%.
   (A) *Salmonella* SWT1001 cultured on LB plates;
   (B) *Salmonella* SWT1005 cultured on LB plates;
   (C) *Salmonella* SWT2009 cultured on LB plates;
   (D) *Salmonella* SWT2013 cultured on LB plates.
Figure 15 Schematic diagram of plasmid pPRO005, containing the chloramphenicol resistance gene, lacUV5, and T7 RNA polymerase gene cassette.

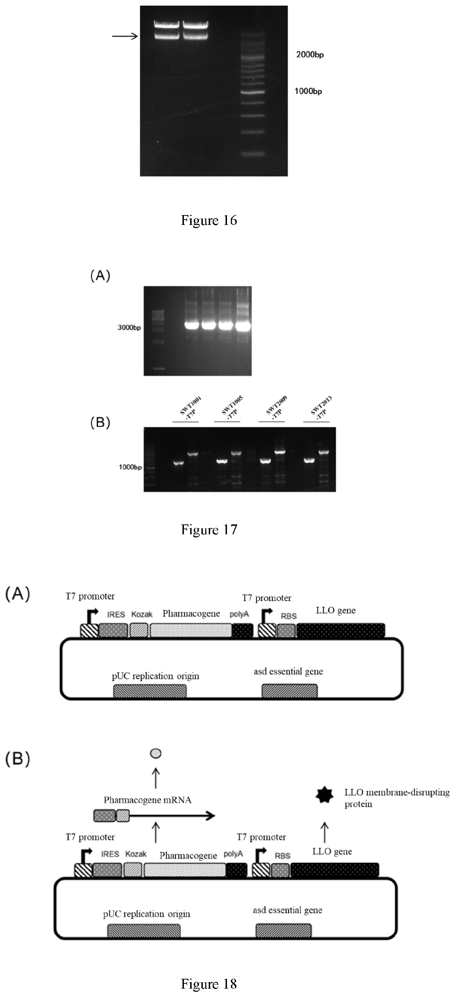

Figure 16 Verification diagram of the enzymatic digestion products of plasmid pPRO005 (The arrow indicates the excised T7 RNA polymerase gene cassette fragment).
Figure 17 Verification diagram of *lacUV5*-controlled T7 RNA polymerase constitutive expression cassette recombined into the *Salmonella* chromosome.
   (A) PCR amplification fragments used for recombination (primers SWTO102 and SWTO103);
   (B) Verification diagram of PCR-amplification for strains SWT1001-T7P, SWT1005-T7P, SWT2009-T7P, SWT2013-T7P after construction.
Figure 18 Schematic diagram of the construction and function of the plasmid vector pEU011.
   (A) Construction diagram of the plasmid vector pEU011;
   (B) Schematic diagram of the functional process of the plasmid vector pEU011.

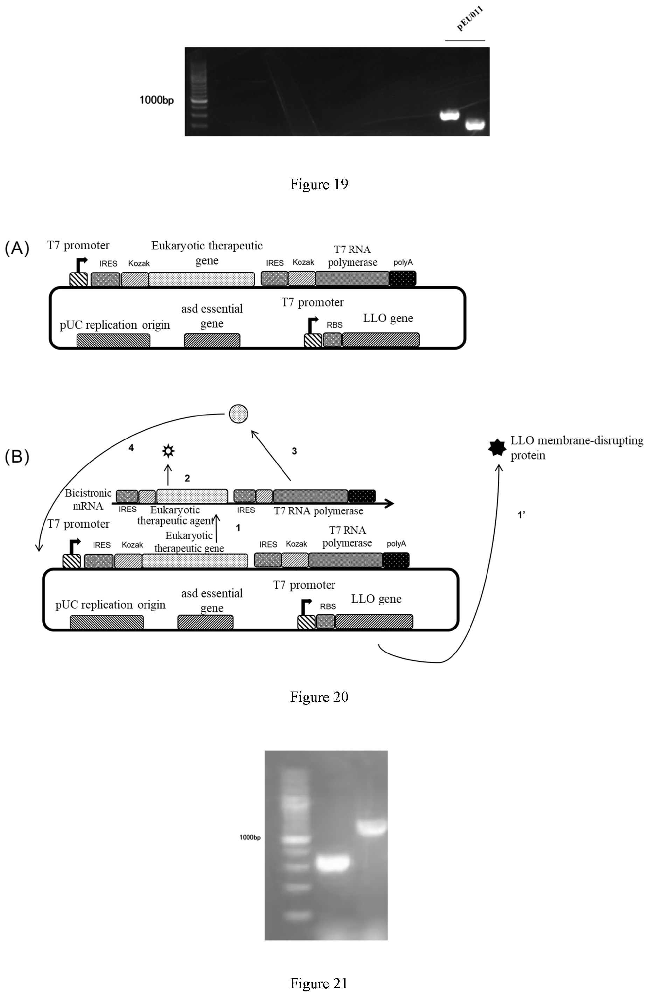

Figure 19 Verification diagram of PCR-amplification for the plasmid vector pEU011.
Figure 20 Schematic diagram of the construction and function of the plasmid vector pEU010.
   (A) Construction diagram of the plasmid vector pEU010;
   (B) Schematic diagram of the functional process of the plasmid vector pEU010.
Figure 21 Verification diagram of PCR-amplification for the plasmid vector pEU010.

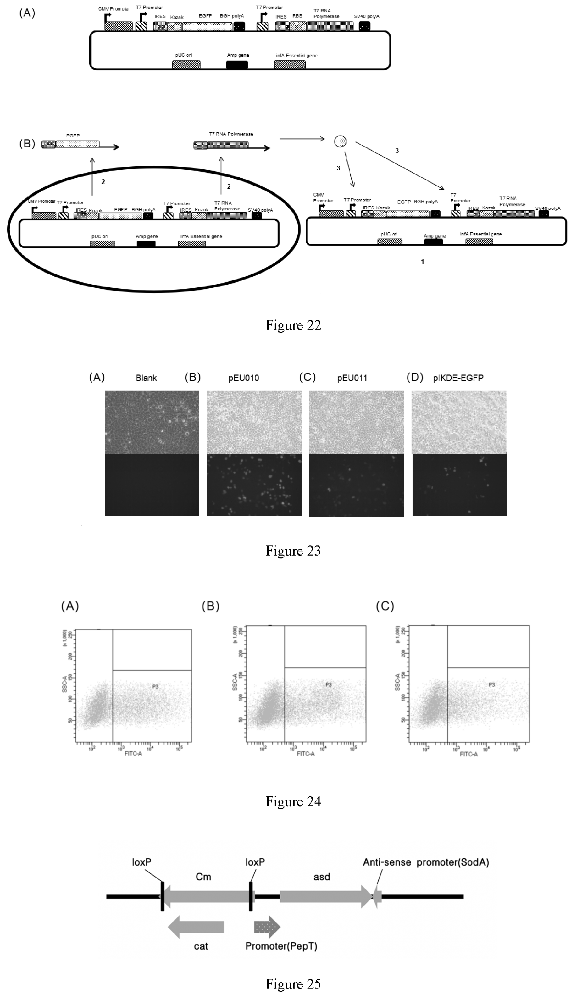

Figure 22 Schematic diagram of the construction and function of the plasmid vector pIKDE-EGFP.
   (A) Construction diagram of the plasmid vector pIKDE-EGFP;
   (B) Schematic diagram of the functional process of the plasmid vector pIKDE-EGFP.
Figure 23 The expression intensity of green fluorescence observed by fluorescence microscopy after transfecting plasmids pEU010, pEU011, and pIKDE-EGFP into BSR-T7/5 cells stably expressing T7 polymerase.
   (A) BSR-T7/5 cell control, with the white light image on top and the fluorescence image on the bottom;
   (B) BSR-T7/5 cells transfected with pEU010, with the white light image on top and the fluorescence image on the bottom;
   (C) BSR-T7/5 cells transfected with pEU011, with the white light image on top and the fluorescence image on the bottom;
   (D) BSR-T7/5 cells transfected with pIKDE-EGFP, with the white light image on top and the fluorescence image on the bottom.
Figure 24 Flow cytometry analysis of the proportion of green fluorescent cells after transfecting plasmids pEU010, pEU011, and pIKDE-EGFP into BSR-T7/5 cells stably expressing T7 polymerase.
   (A) BSR-T7/5 cells transfected with pEU010;
   (B) BSR-T7/5 cells transfected with pEU011;
   (C) BSR-T7/5 cells transfected with pIKDE-EGFP.
Figur*e* 25 Schematic diagram of the construction of the plasmid pSWT004 used to construct the YB1-like *Salmonella* SWT005.

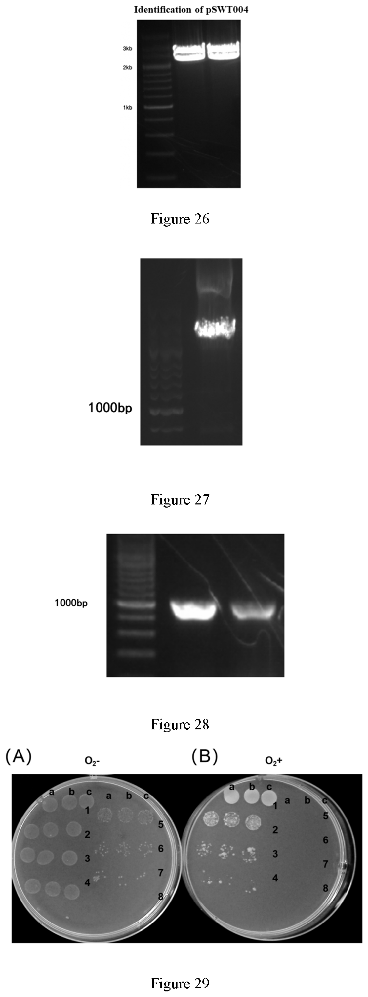

Figure 26 Verification diagram of the restriction enzyme digestion products of plasmid pSWT004 identified by agarose gel electrophoresis.
Figure 27 Verification diagram of the PCR amplification products of the cm-pepT-asd-sodA fragment identified by agarose gel electrophoresis.
Figure 28 Verification diagram of the PCR amplification products of *Salmonella* SWT005 identified by agarose gel electrophoresis.
Figure 29 Results diagram of oxygen adaptability validation tests for the YB1-like *Salmonella* SWT005.
   (A) Cultured under anaerobic conditions on an LB plate without D-alanine;
   (B) Cultured under aerobic conditions on an LB plate without D-alanine.

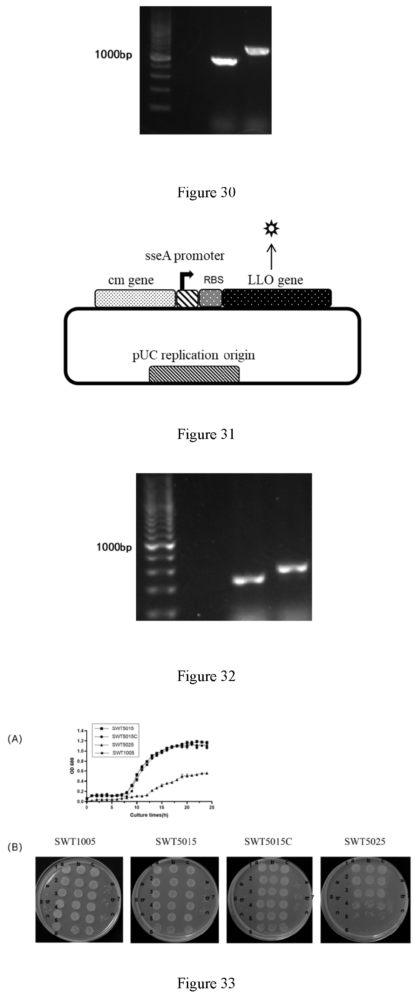

Figure 30 Verification diagram of PCR-amplification for the strain SWT008.
Figure 31 Schematic diagram of the plasmid vector pPRO013.
Figure 32 Verification diagram of PCR-amplification for the strain SWT5025.
Figure 33 Growth capabilities comparation of strains SWT5015, SWT5015C, and SWT5025 in culture media.
   (A) Growth curves of strains SWT5015, SWT5015C, and SWT5025 in liquid LB medium;
   (B) Counting analysis of strains SWT5015, SWT5015C, and SWT5025 under the same OD conditions on solid LB plates.

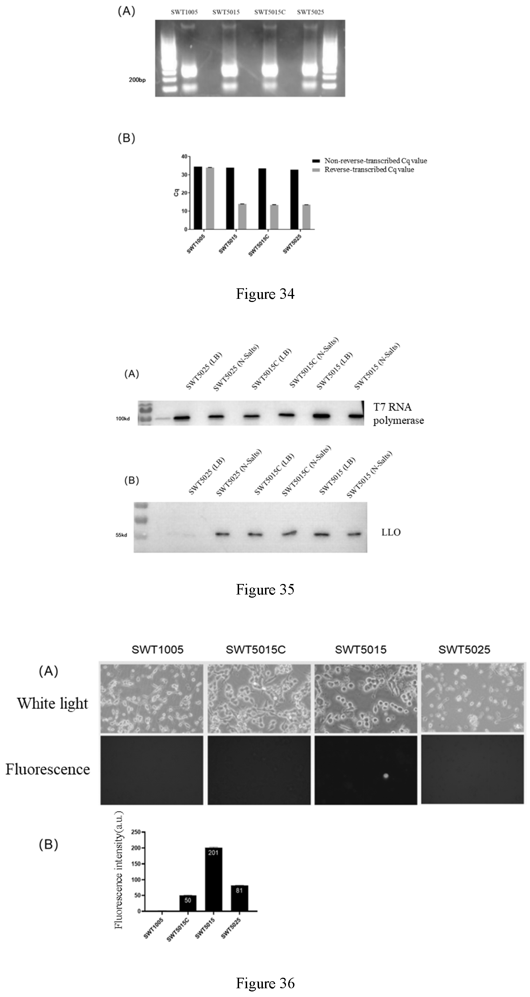

Figure 34 Verification diagram of the EGFP mRNA transcription in strains SWT5015, SWT5015C, and SWT5025.
   (A) Verification diagram of total RNA extracted from the control strain SWT1005 and strains SWT5015, SWT5015C, and SWT5025 identified by agarose gel electrophoresis;
   (B) Amplification of EGFP RNA from the control strain SWT1005 and strains SWT5015, SWT5015C, and SWT5025 using reverse transcription qPCR and non-reverse transcription qPCR, respectively.
Figure 35 Western Blot identification of the expression of T7 RNA polymerase and LLO in strains SWT5025, SWT5015C, and SWT5025.
   (A) Expression of T7 RNA polymerase in strains SWT5025, SWT5015C, and SWT5025 under LB medium and N-salts medium conditions;
   (B) Expression of LLO in strains SWT5025, SWT5015C, and SWT5025 under LB medium and N-salts medium conditions.
Figure 36 EGFP Expression efficiency comparation of strains SWT5015C, SWT5015, and SWT5025 after invading cancer cells.
   (A) Observation of EGFP expression in cells after invasion under a fluorescence microscope;
   (B) Analysis of the EGFP fluorescence signal using a microplate reader.

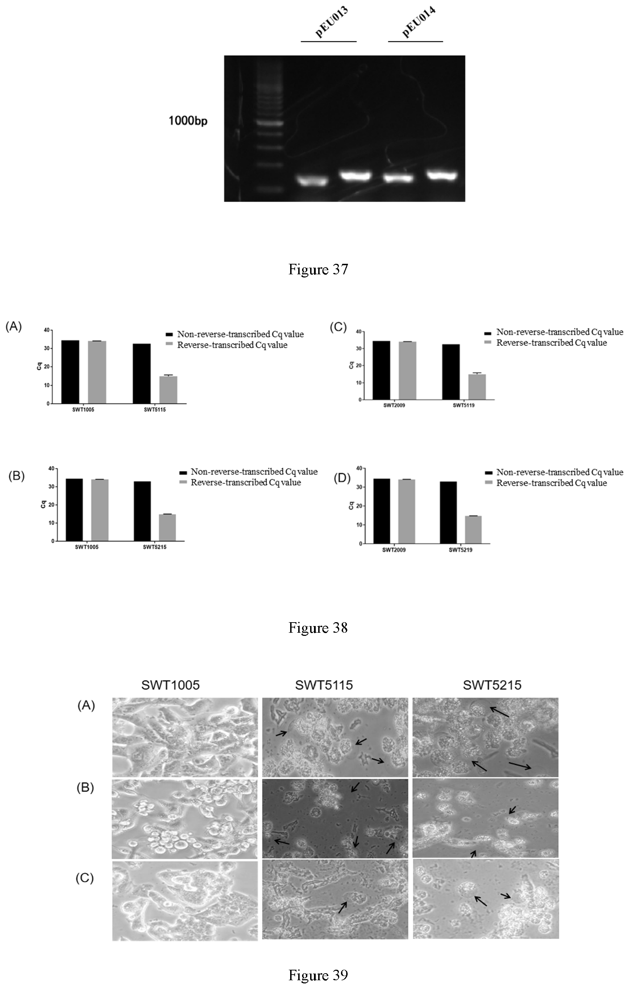

Figure 37 Verification diagram of PCR-amplification for the plasmid vectors pEU013 and pEU014.
Figure 38 qPCR identification of the mRNA transcription of target genes in SWT5115, SWT5119, SWT5215, and SWT5219, where the Cq value represents the amplification threshold.
   (A) Amplification of GSDMD-N mRNA in the control strain SWT1005 and SWT5115 using reverse transcription qPCR and non-reverse transcription qPCR, respectively;
   (B) Amplification of GSDME-N mRNA in the control strain SWT1005 and SWT5215 using reverse transcription qPCR and non-reverse transcription qPCR, respectively;
   (C) Amplification of GSDMD-N mRNA in the control strain SWT1005 and SWT5119 using reverse transcription qPCR and non-reverse transcription qPCR, respectively;
   (D) Amplification of GSDME-N mRNA in the control strain SWT1005 and SWT5219 using reverse
   transcription qPCR and non-reverse transcription qPCR, respectively.
Figure 39 Microscopic images diagram of cell killing caused by pyroptosis after co-culture of strain SWT1005(control), SWT5115 and SWT5215 with cancer cells under anaerobic conditions, respectively.
   (A) EMT6;
   (B) MFC;
   (C) RM-1.

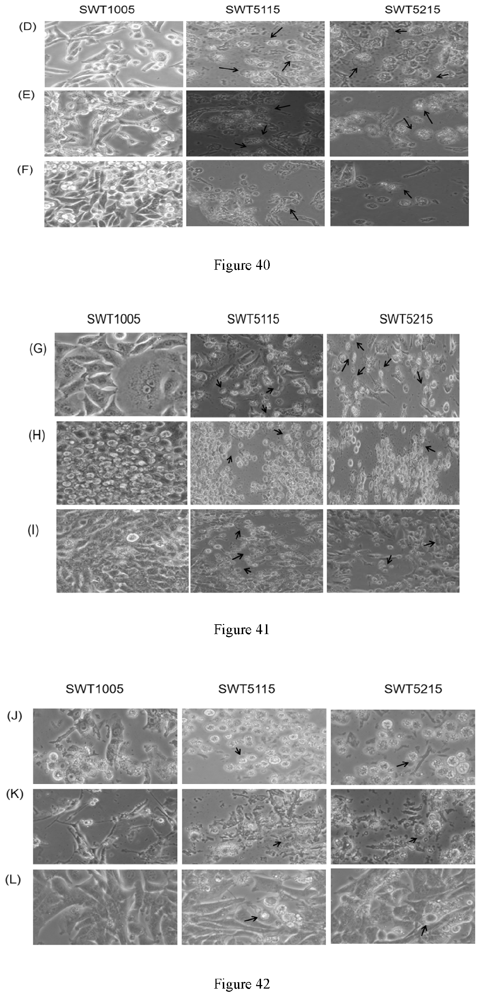

Figure 40 Microscopic images diagram of cell killing caused by pyroptosis after co-culture of strain SWT1005(control), SWT5115 and SWT5215 with cancer cells under anaerobic conditions, respectively.
   (D) SCC7;
   (E) CT26;
   (F) MB49.
Figure 41 Microscopic images diagram of cell killing caused by pyroptosis after co-culture of strain SWT1005(control), SWT5115 and SWT5215 with cancer cells under anaerobic conditions, respectively.
   (G) A549;
   (H) Neuro-2a;
   (I) ID8.
Figure 42 Microscopic images diagram of cell killing caused by pyroptosis after co-culture of strain SWT1005(control), SWT5115 and SWT5215 with cancer cells under anaerobic conditions, respectively.
   (J) Hepa1-6;
   (K) Renca;
   (L) Pan02.

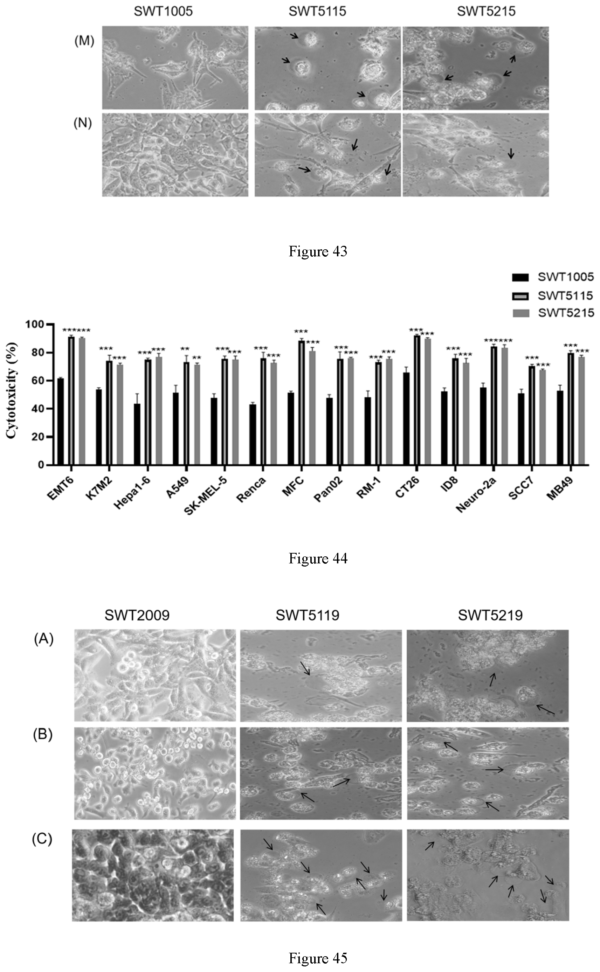

Figure 43 Microscopic images diagram of cell killing caused by pyroptosis after co-culture of strain SWT1005(control), SWT5115 and SWT5215 with cancer cells under anaerobic conditions, respectively.
   (M) SK-MEL-5;
   (N) K7M2.
Figure 44 Killing effect diagram of CCK8 assay identifying for control strain SWT1005 and expression vector strains SWT5115, SWT5215 against cancer cell lines EMT6, K7M2, Hepa1-6, A549, SK-MEL-5, Renca, MFC, Pan02, RM-1, CT26, ID8, Neuro-2a, SCC7 and MB49.
Figure 45 Microscopic images diagram of cell killing caused by pyroptosis after co-culture of strain SWT2009 (control), SWT5119 and SWT5219 with cancer cells under anaerobic conditions, respectively.
   (A) EMT6;
   (B) MFC;
   (C) RM-1.

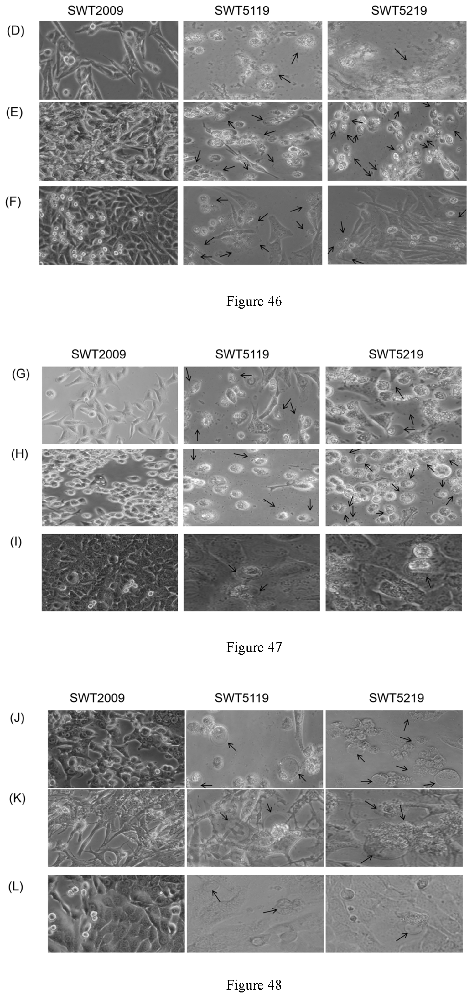

Figure 46 Microscopic images diagram of cell killing caused by pyroptosis after co-culture of strain SWT2009 (control), SWT5119 and SWT5219 with cancer cells under anaerobic conditions, respectively.
   (D) SCC7;
   (E) CT26;
   (F) MB49.
Figure 47 Microscopic images diagram of cell killing caused by pyroptosis after co-culture of strain SWT2009 (control), SWT5119 and SWT5219 with cancer cells under anaerobic conditions, respectively.
   (G) A549;
   (H) Neuro-2a;
   (I) ID8.
Figure 48 Microscopic images diagram of cell killing caused by pyroptosis after co-culture of strain SWT2009 (control), SWT5119 and SWT5219 with cancer cells under anaerobic conditions, respectively.
   (J) Hepa1-6;
   (K) Renca;
   (L) Pan02.

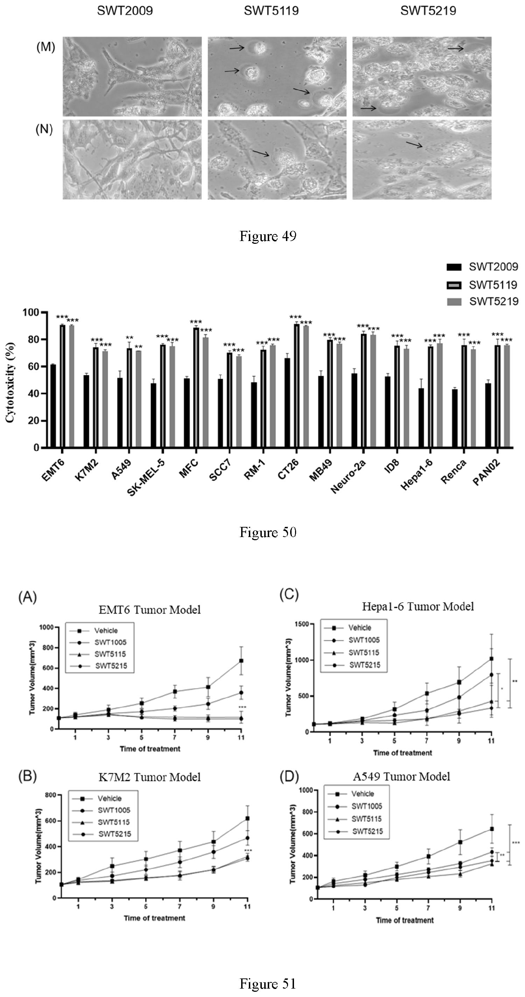

Figure 49 Microscopic images diagram of cell killing caused by pyroptosis after co-culture of strain SWT2009 (control), SWT5119 and SWT5219 with cancer cells under anaerobic conditions, respectively.
   (M) SK-MEL-5;
   (N) K7M2.
Figure 50 Killing effect diagram of CCK8 assay identifying for control strain SWT2009 and expression vector strains SWT5119, SWT5219 against cancer cell lines EMT6, K7M2, A549, SK-MEL-5, MFC and SCC7.
Figure 51 Identification diagram of inhibitory effects for expression vector strains SWT5115, SWT5215 and control strain SWT1005 in different mouse tumor models (vehicle as blank control without drug treatment).
   (A) EMT6;
   (B) K7M2;
   (C) Hepa1-6;
   (D) A549.

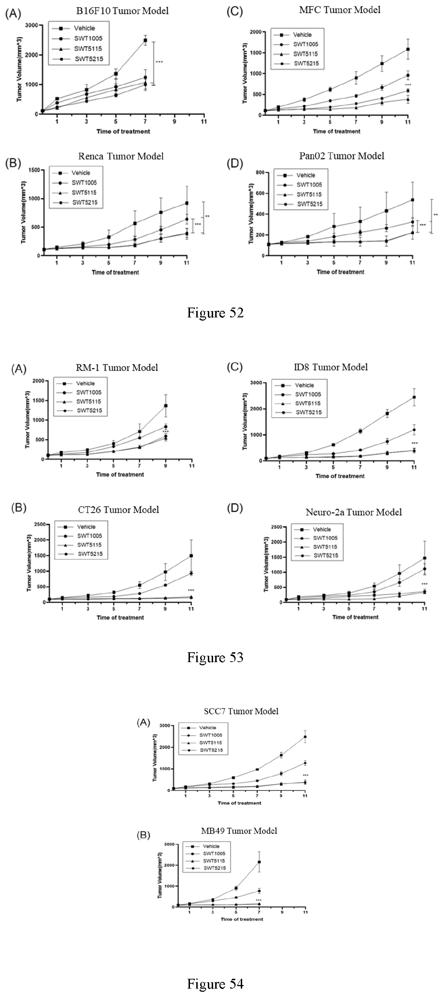

Figure 52 Identification diagram of inhibitory effects for expression vector strains SWT5115, SWT5215 and control strain SWT1005 in different mouse tumor models (vehicle as blank control without drug treatment).
   (A) B16F10;
   (B) Renca;
   (C) MFC;
   (D) Pan02.
Figure 53 Identification diagram of inhibitory effects for expression vector strains SWT5115, SWT5215 and control strain SWT1005 in different mouse tumor models (vehicle as blank control without drug treatment).
   (A) RM-1;
   (B) CT26;
   (C) ID8;
   (D) Neuro-2a.
Figure 54 Identification diagram of inhibitory effects for expression vector strains SWT5115, SWT5215 and control strain SWT1005 in different mouse tumor models (vehicle as blank control without drug treatment).
   (A) SCC7;
   (B) MB49.

### Detailed Description of the Embodiments

The present application will be further described in detail below with reference to the accompanying drawings and embodiments. Through these descriptions, the features and advantages of the present application will become clearer and more apparent.

### Example 1: Construction of hypoxia-specific gene expression cassette using the alr gene and dadX gene as survival -ssential gene

In this embodiment, hypoxia-specific gene expression cassettes were constructed by using the following combinations: The forward hypoxia promoter employed was the *Salmonella yhbU* (*yhbU*-*S*) (SEQ ID No. 31), the survival-essential gene was the *Salmonella alr* gene (SEQ ID No. 27), and the reverse hyperoxia promoters were, respectively, the *Salmonella cyoA* (*cyoA-S*) (SEQ ID No. 33) and the *Salmonella ydcI* (*ydcI*-S) (SEQ ID No. 34). Additionally, the forward hypoxia promoter employed was the *Escherichia coli ynfK* (*ynfK-E*) (SEQ ID No. 32), the survival-essential gene was the *Salmonella dadX* gene (SEQ ID No. 29), and the reverse hyperoxia promoters were, respectively, the *Salmonella cyoA* (*cyoA-*S) (SEQ ID No. 33) and the *Salmonella ydcI* (*ydcI-S*) (SEQ ID No. 34).

The combinations for the hypoxia-specific gene expression cassette are shown in Table 1.

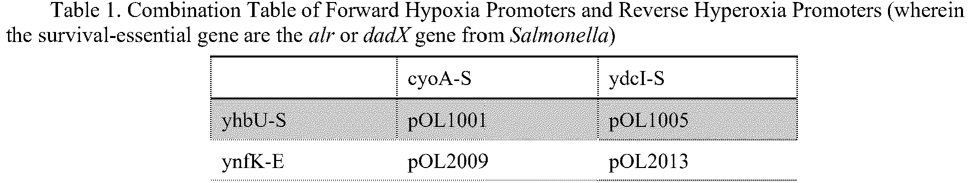

### (I) Construction of the Forward Hypoxia Promoter Clone Library

1. A single colony of wild-type *Salmonella Typhimurium* (strain SWT001, purchased from the China Center for Industrial Culture Collection, CICC) *or Escherichia coli* DH10B (purchased from Shanghai Weidi Biotechnology Co., Ltd.) was picked and inoculated into 5 ml of LB broth. The culture was then incubated in a shaking incubator at 37°C and 220 rpm for 16 hours, until the OD₆₀₀ was between 2 and 3.
2. Two microliters (2 µl) of the corresponding bacterial culture were added to a reaction mixture containing the corresponding primers and a high-fidelity PCR enzyme (purchased from Takara), and the mixture was placed in a PCR amplification equipment.
   The PCR program was as follows: 95°C for 2 minutes; followed by 30 cycles of 95°C for 30 seconds, 60°C for 30 seconds, and 72°C for 60 seconds; 72°C for 10 minutes; and holding at 4°C for 5 minutes.
3. The amplified PCR product was purified and recovered using a DNA gel purification kit.
4. The recovered product was mixed with a restriction digestion mixture containing restriction enzymes (purchased from NEB) and incubated at 37°C for 1 hour.
5. The digestion product was then purified and recovered using a DNA gel purification kit.

The primers and restriction enzymes used for the different forward hypoxia promoters were as follows:
1. yhbU-S (SEQ ID NO: 31): The promoter was amplified from strain SWT001 using primers SWTO19 and SWTO20. The product was then double-digested with NotI and HindIII and recovered.
2. ynfK-E (SEQ ID NO: 32): The promoter was amplified from *Escherichia coli* strain DH10B using primers SWTO21 and SWTO22. The product was then double-digested with NotI and HindIII and recovered.

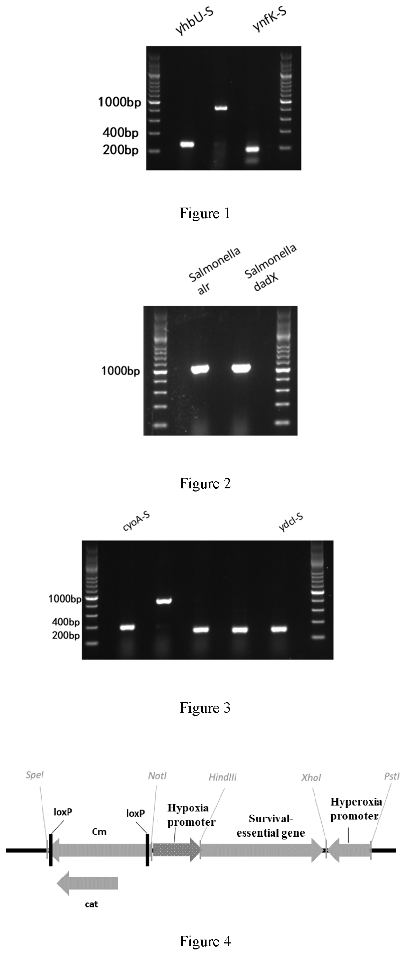

The results of the verification of the forward hypoxia promoter clone library by agarose gel electrophoresis are shown in Figure 1.

### (II) Construction of the Survival-Essential Gene Library

1. A single colony of wild-type *Salmonella Typhimurium* (strain SWT001, purchased from the China Center for Industrial Culture Collection, CICC) was picked and inoculated into 5 ml of LB broth. The culture was then incubated in a shaking incubator at 37°C and 220 rpm for 16 hours.
2. Two microliters (2 µl) of the corresponding bacterial culture were added to a reaction mixture containing the corresponding primers and a high-fidelity PCR enzyme, and the mixture was placed in a PCR amplification equipment.

The PCR program was as follows: 95°C for 2 minutes; followed by 30 cycles of 95°C for 30 seconds, 60°C for 30 seconds, and 72°C for 60 seconds; 72°C for 10 minutes; and holding at 4°C for 5 minutes.

3. The amplified PCR product was purified and recovered using a DNA gel purification kit.

4. The recovered product was mixed with a restriction digestion mixture containing restriction enzymes and incubated at 37°C for 1 hour.

5. The digestion product was then purified and recovered using a DNA gel purification kit.

The different survival-essential genes were as follows:
1. *Salmonella alr* gene (SEQ ID NO: 27): The gene was amplified from strain SWT001 using primers SWTO35 and SWTO36 The product was then double-digested with HindIII and XhoI and recovered.
2. *Salmonella dadX* gene (SEQ ID NO: 29): The gene was amplified from strain SWT001 using primers SWTO39 and SWTO40. The product was then double-digested with HindIII and XhoI and recovered.

The result of the verification of the survival-essential gene library by agarose gel electrophoresis is shown in Figure 2.

### (III) Construction of the Reverse Hyperoxia Promoter Library

1. A single colony of wild-type *Salmonella Typhimurium* (strain SWT001, purchased from the China Center for Industrial Culture Collection, CICC) was picked and inoculated into 5 ml of LB broth. The culture was then incubated in a shaking incubator at 37°C and 220 rpm for 16 hours.
2. Two microliters (2 µl) of the corresponding bacterial culture were added to a reaction mixture containing the corresponding primers and a high-fidelity PCR enzyme, and the mixture was placed in a PCR amplification equipment.

The PCR program was as follows: 95°C for 2 minutes; followed by 30 cycles of 95°C for 30 seconds, 60°C for 30 seconds, and 72°C for 60 seconds; 72°C for 10 minutes; and holding at 4°C for 5 minutes.

3. The amplified PCR product was purified and recovered using a DNA gel purification kit.

4. The recovered product was mixed with a restriction digestion mixture containing restriction enzymes and incubated at 37°C for 1 hour.

5. The digestion product was then purified and recovered using a DNA gel purification kit.

The primers and restriction enzymes used for the different reverse hyperoxia promoters were as follows:
1. cyoA-S (SEQ ID NO: 33): The promoter was amplified from strain SWT001 using primers SWTO43 and SWTO44. The product was then double-digested with XhoI and PstI and recovered.
2. ydcI-S (SEQ ID NO: 34): The promoter was amplified from strain SWT001 using primers SWTO51 and SWTO52. The product was then double-digested with XhoI and PstI and recovered.

The results of the verification of the reverse hyperoxia promoter clone library by agarose gel electrophoresis are shown in Figure 3.

### (IV) Construction of a Combinatorial Library of Forward Hypoxia Promoters, Survival-Essential Genes, and Reverse Hyperoxia Promoters

The composition of the hypoxia-specific gene expression cassette is shown in Figure 4. It is composed of a forward hypoxia promoter, an survival-essential gene, and a reverse hyperoxia promoter, arranged in sequence.

As shown in Table 1, the cloned library was formed by combining the following sequences: the forward hypoxia promoter library digested with NotI and HindIII; the survival-essential *Salmonella alr* gene (SEQ ID NO: 27) or *Salmonella dadX* gene (SEQ ID NO: 29) digested with HindIII and XhoI; and the reverse hyperoxia promoter library digested with XhoI and PstI. These were ligated into the plasmid pSWT003 vector (vector backbone: pBlueScript SK(+), purchased from Biofeng), which had been digested with SpeI and PstI, and the plasmid pSWT007 vector (containing bilateral co-directional *loxP* sequences and a DNA fragment of the chloramphenicol resistance gene (SEQ ID NO: 37)), which had been digested with SpeI and NotI.

The products mentioned above obtained by primer amplification from *Salmonella* SWT001 or *Escherichia coli* DH10B followed by enzymatic digestion and recovery, were subjected to combinatorial ligation via an enzymatic ligation reaction. The ligation products were transformed into DH10B and spread onto LB plates containing 25 µg/mL chloramphenicol to obtain the corresponding plasmids shown in Table 1.

### (V) Construction of the strain corresponding to this invention

### 1. Wild-type Salmonella typhimurium (SWT001)

Wild-type *Salmonella typhimurium* (SWT001) was purchased from the China Center of Industrial Culture Collection (CICC).

### 2. Construction of Salmonella (SWT002) containing a temperature-inducible Lambda-RED recombinase and a loxP-Cre enzyme system

The Lambda-RED recombination system is widely used for homologous recombination in Gram-negative bacteria. In the present invention, this system consists of plasmid pSWT001.

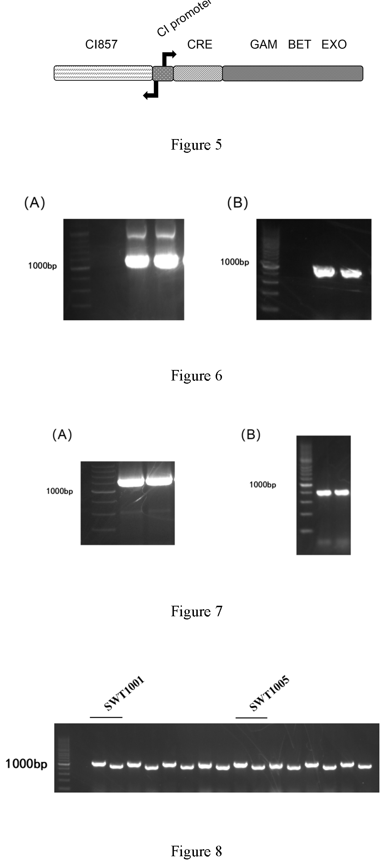

As shown in Figure 5, plasmid pSWT001 contains a Lambda-RED recombinase module (SEQ ID NO: 38) (functionally similar to the plasmid vector *p*sim6 from Biofeng) and a *loxP*-Cre recombinase module (SEQ ID NO: 38) (functionally similar to the plasmid vector 705-Cre from Gene Bridges). The Lambda-RED recombinase module is composed of three recombinases: EXO, BET, and GAM, and the recombinases are controlled by the CI857 temperature-sensitive regulator. Therefore, they cannot be expressed under culture conditions at 32°C and are expressed only at temperatures higher than 37°C. Under temperature-induced conditions, the EXO recombinase cleaves the 5' end of double-stranded linear DNA, creating single-stranded DNA with a protruding 3' end. The single-stranded DNA is bound by the BET protein and protected from degradation by other nucleases. The function of GAM is to inhibit bacterial endogenous nuclease activity. The homology arms for homologous recombination are approximately 35-50 bp in length and are added to both sides of the DNA fragment to be recombined via PCR. The advantage of this technology is that it precisely targets the region of interest in the bacterial chromosome without causing extra mutations. The outermost sides of the recombined double-stranded DNA fragment include the homology arm sequences for the recombination site, while the interior contains two *loxP* sequences in the same orientation, totaling 34 bp. Located between the two co-directional *loxP* sites is a chloramphenicol resistance gene used for screening recombinant bacteria. After successful recombination, the CRE enzyme system carried on the pSWT001 plasmid (also controlled by the CI857 temperature-sensitive regulator) specifically recognizes the *loxP* sequences and creates a cut within the sequence between the co-directional *loxP* sites, leaving one *loxP* sequence behind, thereby eliminating the chloramphenicol resistance gene. Repeating the above operations allows for continuous gene knockout and knock-in.

The specific operational steps are as follows:
(1) Strain SWT001 was streaked onto an LB plate and cultured overnight in a constant temperature incubator at 37°C;
(2) A single colony was picked and inoculated into 5 mL of LB liquid medium, then placed in a constant temperature shaker at 37°C and 220 rpm for 16 hours;
(3) The culture was inoculated into fresh LB liquid medium at a ratio of 1:100 and cultured for a further 2-3 hours until the bacterial density reached OD₆₀₀ = 0.3, then placed on ice for 1 hour;
(4) The bacterial cells were washed 3 times with sterilized purified water;
(5) The recovered bacterial cells were mixed with 10 ng of plasmid pSWT001 and electroporated at a voltage of 1.8 kV;
(6) The electroporated bacterial cells were spread onto LB plates containing 100 µg/mL ampicillin sodium and cultured overnight in a constant temperature incubator at 32°C until single colonies grew. The resulting strain was named SWT002.

### 3. Construction of aroA Gene-Knockout Attenuated Salmonella (SWT003)

(1) A single colony of SWT002 was picked and inoculated into 5 mL of LB liquid medium containing 100 µg/mL ampicillin sodium, and cultured in a constant temperature shaker at 32°C and 220 rpm for 16 hours.
(2) The culture was inoculated at a ratio of 1:100 into fresh LB liquid medium containing 100 µg/mL ampicillin sodium and cultured for a further 2-3 hours. When the bacterial density reached an OD₆₀₀ of 0.3, the culture flask was placed in a 42°C water bath and incubated with shaking for 15 minutes, then placed on ice for 1 hour.
(3) The bacterial cells were washed 3 times with sterilized purified water.
(4) Preparation of PCR products using primers SWTO1 and SWTO2:
   Primers SWTO1 and SWTO2 were mixed with plasmid pSWT002 (SEQ ID No.39, containing bilateral co-directional *loxP* sequences with a chloramphenicol resistance gene in between) and a high-fidelity PCR amplification enzyme system, and placed into PCR amplification equipment.

The amplification program was: 95°C for 2 minutes; 30 cycles of 95°C for 30 seconds, 60°C for 30 seconds, and 72°C for 60 seconds; followed by 72°C for 10 minutes; and 4°C for 5 minutes.

The amplified PCR products were verified by agarose gel electrophoresis. The verification results of the PCR amplification products of the *aroA* gene knockout fragment using SWTO1 and SWTO2 are shown in Figure 6A.

The products were purified and recovered using a DNA gel extraction system. The concentration and purity of the recovered PCR products were determined using a nanodrop.

(5) The recovered bacterial cells were mixed with 100 ng of the PCR products generated by SWTO1 and SWTO2, and electroporated at a voltage of 1.8 kV.

(6) The electroporated bacterial cells were spread onto plates containing 25 µg/mL chloramphenicol and cultured overnight in a constant temperature incubator at 32°C until single colonies grew.

(7) Positive clones were identified by colony PCR. Primers SWTO4 and SWTO6, and SWTO3 and SWTO5 were used to verify the insertion of the chloramphenicol resistance gene.

The PCR amplification products of the target fragment of strain SWT003 were verified by agarose gel electrophoresis, and the verification results are shown in Figure 6B.

(8) The positive single colonies were inoculated into 5 mL of LB medium and cultured in a constant temperature shaker at 37°C and 220 rpm for 16 hours to allow the CRE enzyme to function and excise the chloramphenicol resistance gene.

### 4. Construction of alr Gene-Knockout Attenuated Salmonella (SWT004)

(1) A single colony of SWT003 was picked and inoculated into 5 mL of LB liquid medium, and cultured in a constant temperature shaker at 32°C and 220 rpm for 16 hours.
(2) The culture was inoculated at a ratio of 1:100 into fresh LB liquid medium and cultured for a further 2-3 hours. When the bacterial density reached an OD₆₀₀ of 0.3, the culture flask was placed in a 42°C water bath and incubated with shaking for 15 minutes, then placed on ice for 1 hour.
(3) The bacterial cells were washed 3 times with sterilized purified water.
(4) Preparation of PCR products using primers SWTO70 and SWTO71:

Primers SWTO70 and SWTO71 were mixed with plasmid pSWT002 and a high-fidelity PCR amplification enzyme system, and placed into PCR amplification equipment.

The amplification program was: 95°C for 2 minutes; 30 cycles of 95°C for 30 seconds, 60°C for 30 seconds, and 72°C for 60 seconds; followed by 72°C for 10 minutes; and 4°C for 5 minutes.

The amplified PCR products were purified and recovered using a DNA gel extraction system. The concentration and purity of the recovered PCR products were determined using a nanodrop.

(5) The recovered bacterial cells were mixed with 100 ng of the PCR products generated by SWTO70 and SWTO71, and electroporated at a voltage of 1.8 kV.

The PCR amplification products of the *alr* gene knockout fragment using SWTO70 and SWTO71 were verified by agarose gel electrophoresis, and the verification results are shown in Figure 7A.

(6) The electroporated bacterial cells were spread onto plates containing 25 µg/mL chloramphenicol and cultured overnight in a constant temperature incubator at 32°C until single colonies grew.

(7) Positive clones were identified by colony PCR. Primers SWTO72 and SWTO5, and SWTO6 and SWTO73 were used to verify the insertion of the chloramphenicol resistance gene.

The PCR amplification products of the target fragment of strain SWT004 were verified by agarose gel electrophoresis, and the verification results are shown in Figure 7B (primers used: SWTO72, SWTO5, SWTO6, SWTO73).

(8) The positive single colonies were inoculated into 5 mL of LB medium and cultured in a constant temperature shaker at 37°C and 220 rpm for 16 hours to allow the CRE enzyme to function and excise the chloramphenicol resistance gene.

### Example 2: Integration of the Hypoxia-Specific Gene Expression Cassette into the dadX Gene Locus on the Chromosome of Strain SWT004 (Disrupting its Function)

1. A single colony of the *alr* gene-knockout attenuated bacteria SWT004 was picked and inoculated into 5 mL of fresh LB liquid medium, and cultured in a constant temperature shaker at 32°C and 220 rpm for 16 hours.
2. The culture was inoculated at a ratio of 1:100 into fresh LB liquid medium and cultured for a further 2-3 hours until the bacterial density reached an OD₆₀₀ of 0.3. The culture flask was then placed in a 42°C water bath and incubated with shaking for 15 minutes, followed by resting on ice for 1 hour.
3. The bacterial cells were washed 3 times with sterilized purified water.
4. Preparation of PCR products for constructing strains containing the hypoxia-specific expression cassette: Primers SWTO78 and SWTO79 were mixed with the hypoxia-specific gene expression cassette plasmid (pOL1001, pOL1005) and a high-fidelity PCR amplification enzyme system, and placed into PCR amplification equipment.

The amplification program was: 95°C for 2 minutes; 30 cycles of 95°C for 30 seconds, 60°C for 30 seconds, and 72°C for 120 seconds; followed by 72°C for 10 minutes; and 4°C for 5 minutes.

The amplified PCR products were purified and recovered using a DNA gel extraction system. The concentration and purity of the recovered PCR products were determined using a nanodrop.

5. The recovered bacterial cells were mixed with 100 ng of the PCR product and electroporated at a voltage of 1.8 kV.

6. The electroporated bacterial cells were spread onto plates containing 25 µg/mL chloramphenicol and 100 µg/mL D-alanine, and cultured overnight in a constant temperature incubator at 32°C until single colonies grew and the generated strains are SWT1001 and SWT1005, respectively..

7. A single colony was inoculated into 5 mL of fresh LB liquid medium containing 100 µg/mL D-alanine and cultured in a constant temperature shaker at 37°C and 220 rpm for 16 hours to allow the CRE enzyme to function and excise the chloramphenicol resistance gene.

8. Clones were identified by colony PCR using the corresponding primers SWTO61, 20 and SWTO75, 20. The identification results of strain SWT1001 (yhbU-S + alr + cyoA-S combination) and strain SWT1005 (yhbU-S + alr + ydcI-S combination) are shown in Figure 8.

### Example 3: Construction of the Hypoxia-Specific Gene Expression Cassette with dadX as the Survival-essential Gene

As alanine racemase, the genomes of bacteria such as *Salmonella* and *E. coli* contain two isoenzyme genes, namely the *alr* gene and the *dadX* gene. To verify the effect, the survival-essential gene in the aforementioned hypoxia-specific gene expression cassettes was accordingly replaced with the homologous *dadX* gene in plasmids pOL2009 and pOL2013.

### 1. Construction of the dadX Gene Knockout Attenuated Bacterium (SWT007)

(1) A single colony of SWT003 was picked and inoculated into 5 ml of LB liquid medium, and placed in a constant temperature shaker at 32°C and 220 rpm for 16 hours of cultivation.
(2) The culture was inoculated into fresh LB liquid medium at a ratio of 1:100 and cultivation were continued for 2-3 hours until the bacterial density grew to an OD₆₀₀=0.3, at which point the culture flask was placed in a 42°C water bath and incubated with shaking for 15 minutes, then placed on ice to stand for 1 hour.
(3) The bacterial cells were washed 3 times with sterilized pure water.
(4) Preparation of the PCR product of SWTO78 and SWTO79.

Primers SWTO78 and SWTO79 were mixed with plasmid pSWT002 (as described above) and a high-fidelity PCR amplification enzyme system, and placed into a PCR amplification instrument.

The amplification program was: 95°C for 2 minutes; 30 cycles of 95°C for 30 seconds, 60°C for 30 seconds, 72°C for 60 seconds; 72°C for 10 minutes; 4°C for 5 minutes.

The amplified PCR product was purified and recovered using a DNA gel recovery system, and the concentration and purity of the recovered PCR product DNA were measured using a nanodrop.

(5) The recovered bacterial cells were mixed with 100 ng of the PCR product of SWTO78 and SWTO79, and electroporated at a voltage of 1.8 kV.

(6) The electroporated bacterial cells were spread onto plates containing 25 µg/mL chloramphenicol and cultured overnight in a constant temperature incubator at 32°C until single colonies grew.

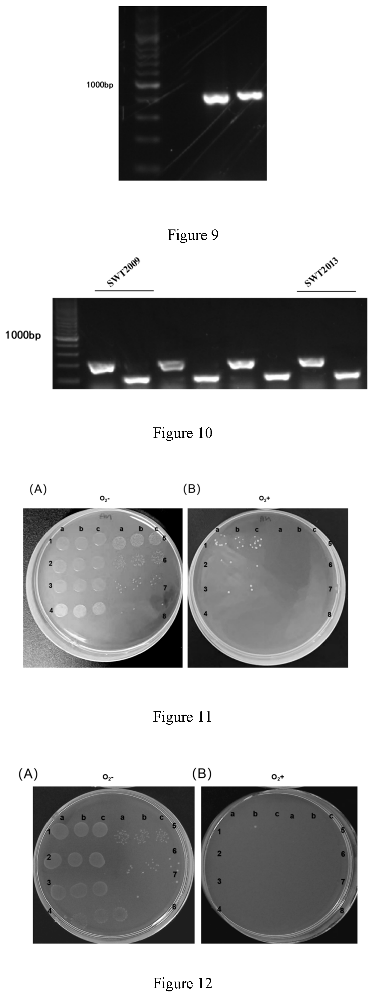

(7) Positive clones were identified through colony PCR using primers SWTO59, SWTO5, SWTO6, and SWTO87 to verify the insertion of the chloramphenicol resistance gene, as shown in figure 9.

(8) The positive single clones were inoculated into 5 ml of LB medium and cultured for 16 hours in a constant temperature shaker at 37°C and 220 rpm, allowing the Cre recombinase to act and excise the chloramphenicol resistance gene.

### 2. Construction of a Deficient and Attenuated Bacterial Strain Containing the Hypoxia-Specific Gene Expression Cassette and with the Salmonella dadX Gene as the Survival-Essential Gene

(1) A single colony of the *dadX* gene knockout attenuated bacterium, SWT007, was picked and inoculated into 5 ml of LB liquid medium, and cultured for 16 hours in a constant temperature shaker at 32°C and 220 rpm.
(2) The culture was inoculated into fresh LB liquid medium at a ratio of 1:100, and cultivation was continued for 2-3 hours until the bacterial density reached an OD₆₀₀ of 0.3, at which point the culture flask was placed in a 42°C water bath and incubated with shaking for 15 minutes, then placed on ice to stand for 1 hour.
(3) The bacterial cells were washed 3 times with sterilized pure water.
(4) To prepare the PCR product for constructing the strain containing the hypoxia-specific gene expression cassette, primers SWTO85 and SWTO86 were mixed with the hypoxia-specific gene expression cassette plasmid (pOL2009, pOL2013) and a high-fidelity PCR amplification enzyme system, and the mixture was placed into a PCR amplification instrument.

The amplification program was: 95°C for 2 minutes; 30 cycles of 95°C for 30 seconds, 60°C for 30 seconds, and 72°C for 120 seconds; followed by 72°C for 10 minutes; and 4°C for 5 minutes.

The amplified PCR product was purified and recovered using a DNA gel recovery system, and the concentration and purity of the recovered PCR product DNA were measured using a Nanodrop spectrophotometer.

(5) The recovered bacterial cells were mixed with 100 ng of the PCR product, and electroporation was performed at a voltage of 1.8 kV.

(6) The electroporated cells were plated on plates containing 25 µg/ml chloramphenicol and 100 µg/ml D-alanine, and incubated overnight in a constant temperature incubator at 32°C until single colonies grew.

(7) The positive single colony was inoculated into 5 ml of LB medium and cultured for 16 hours in a constant temperature shaker at 37°C and 220 rpm, allowing the CRE recombinase to act and excise the chloramphenicol resistance gene.

(8) Clones were identified by colony PCR using the corresponding primers SWTO98, 22 and SWTO99, 22. The identification results of strain SWT2013 (ynfK-E+dadX+ydcI-S) and strain SWT2009 (ynfK-E+dadX+cyoA-S) are shown in Figure 10.

### Example 4: Verification of Oxygen Adaptability of the Salmonella Strain SWT1001, SWT1005, SWT2009, SWT2013 Containing Hypoxia-Specific Gene Expression Cassette

1. Single colonies of strains SWT1001, SWT1005, SWT2009, SWT2013 containing the hypoxia-specific expression cassette were picked and inoculated into 5 mL of fresh LB liquid medium containing 100 µg/mL D-alanine, and cultured in a constant temperature shaker at 37°C and 200 rpm for 16 hours.
2. After culturing was completed, the strains were diluted 10-fold, and the absorbance (OD value) at 600 nm was measured.
3. The volume of bacterial culture corresponding to 1 OD of bacteria was calculated according to the following formula, and deionized water was added to reach 1 mL.$Volume of bacterial culture for 1 OD = 1 / \left(Absorbance\times 10\right) \times 1000$
4. 10 µL of the above bacterial suspension was taken and spotted onto two LB plates without D-alanine for culture, labeled as "1". This was repeated three times, labeled as "a", "b", and "c".
5. After performing a 10-fold serial dilution, 10 µL of the diluted solution was taken and spotted onto the above medium, labeled as "2".
6. This 10-fold serial dilution was continued in the same manner until labeled "8".
7. One of the plates was placed in an anaerobic environment at 37°C for culture, and the other plate was placed in an atmospheric environment (21% oxygen concentration) at 37°C for culture.

The verification test results of the oxygen adaptability of the strains containing the hypoxia-specific expression cassette are shown in Figures 11-13. The results demonstrate that *Salmonella* strains SWT1001, SWT1005, SWT2009, and SWT1023-each harboring the described hypoxia-specific gene expression cassette-exhibit hypoxia-regulated capability.

### Example 5: Oxygen Concentration Simulation for the Strain Library Containing Hypoxia-Specific Gene Expression Cassette

Oxygen in the sealed culture jar is consumed by using an anaerobic gas-generating bag, and the oxygen concentration in the sealed culture jar is determined using an oxygen meter. After using the anaerobic gas-generating bag for a certain period, the oxygen concentration in the sealed culture jar can be fixed within a specific concentration range. The specific method is as follows:
An anaerobic gas-generating bag (Brand: Mitsubishi, Japan; Cat. No.: D-119) was placed into a 7.0 L sealed culture jar (Brand: Mitsubishi, Japan; Cat. No.: D-112), and an oxygen meter (Brand: Meicheng Electronics; Cat. No.: OX-100A) was placed inside simultaneously.

The above-mentioned anaerobic gas-generating bag combination was used to verify the growth status of the genetically modified strains under an oxygen concentration below 0.8%.

Since the range of the pathological hypoxia zone in tumors corresponds to an oxygen concentration of less than 1%, the present invention intends to demonstrate that *Salmonella* modified with the hypoxia-specific gene expression cassette can still grow normally when the oxygen concentration is less than 1% (or approaches 1%).

The regulation of oxygen concentration was achieved using an anaerobic gas-generating bag. After consumption by the anaerobic gas-generating bag for 1 hour, the reading on the oxygen meter indicated that the oxygen concentration was between 0.8% and 1%. Therefore, this experiment simulated the intratumoral hypoxic environment to verify the growth of *Salmonella* on culture plates after the anaerobic gas-generating bag had consumed oxygen in the sealed culture jar for 1 hour, thereby simulating the growth conditions of *Salmonella* strains containing the hypoxia-specific gene expression cassette within the intratumoral hypoxic environment.

The operational procedure for the spotting assay is as follows:
(1) Single colonies of strains containing the hypoxia-specific expression cassette were picked and inoculated into 5 mL of fresh LB liquid medium containing 100 µg/mL D-alanine, and cultured in a constant temperature shaker at 37°C and 200 rpm for 16 hours.
(2) After culturing was completed, the strains were diluted 10-fold, and the absorbance (OD value) at 600 nm was measured.
(3) The volume of bacterial culture corresponding to 1 OD of bacteria was calculated according to the following formula, and deionized water was added to reach 1 mL.$Volume of bacterial culture for 1 OD = 1 / \left(Absorbance\times 10\right) \times 1000$
(4) 10 µL of the above bacterial suspension was taken and spotted onto an LB plate containing D-alanine for culture, labeled as "1". This was repeated three times, labeled as "a", "b", and "c".
(5) Simultaneously, 10 µL of the above bacterial suspension was taken and spotted onto another LB plate without D-alanine for culture, labeled as "1" with three replicates labeled as "a", "b", and "c".
(6) After performing a 10-fold serial dilution, 10 µL of the diluted solution was taken and spotted onto the above media, labeled as "2".
(7) This 10-fold serial dilution was continued in the same manner until labeled "8".
(8) The two plates were placed in an environment with 0.8% oxygen at 37°C for culture.

As shown in Figure 14, the present invention compared the growth of different strains (Figure 14(A) SWT1001, Figure 14(B) SWT1005, Figure 14(C) SWT2009, Figure 14(D) SWT2013) on LB plates without supplemented D-alanine to judge the growth status of each strain under an oxygen concentration below 0.8%. Since the medium was not supplemented with D-alanine, the corresponding *Salmonella* strains relied on their own oxygen regulation systems to grow normally. The results confirmed that strains containing the hypoxia-specific expression cassette, which can grow normally in an anaerobic environment, can all grow normally under an oxygen concentration below 0.8%.

### Example 6: Construction of Salmonella Strains (SWT1001-T7P, SWT1005-T7P, SWT2009-T7P, SWT2013-T7P) with T7 RNA Polymerase Homologously Replaced at the asd Gene Locus

### (I) Construction of Plasmid pPRO005 for Constitutive Expression of T7 RNA Polymerase Controlled by lacUV5

To construct a constitutively expressed T7 RNA polymerase, the plasmid vector pPRO005 was generated. The process is illustrated in Figure 15. The constitutive expression promoter lacUV5 was positioned upstream of the ribosome binding site (RBS) sequence (SEQ ID No. 35) and the T7 RNA polymerase gene (SEQ ID No. 4). Furthermore, a chloramphenicol resistance gene (*cm*) (SEQ ID No. 37) flanked by *loxp* sequences on both sides was inserted upstream of *lacUV5*. This was done to facilitate subsequent homologous recombination into the *Salmonella* chromosome and for selection purposes.
1. Plasmid pSWT007 (synthesized by Beijing Liuhe Huada Gene Technology Co., Ltd.) was digested with SpeI and NotI restriction enzymes. An approximately 1100 bp fragment containing the chloramphenicol resistance gene (*cm*) flanked by *loxp* sequences was recovered.
2. Primers SWTO100 and SWTO101 were directly annealed to generate NotI and HindIII restriction sites, and an approximately 70 bp fragment comprising the lacUV5 promoter was recovered.
3. Plasmid pPRO004 (synthesized by Beijing Liuhe Huada Gene Technology Co., Ltd.) was digested with HindIII and XhoI restriction enzymes. An approximately 2690 bp fragment containing the T7 RNA polymerase gene was recovered.
4. Plasmid pSWT003 (plasmid backbone) was digested with SpeI and XhoI restriction enzymes. An approximately 3000 bp fragment was recovered.
5. The aforementioned five fragments were ligated using T4 DNA ligase. The ligation product was then transformed into DH10B competent cells (purchased from Shanghai Weidi Biotechnology Co., Ltd.). The transformed cells were plated onto LB agar plates containing both ampicillin and chloramphenicol for double antibiotic selection, and single colonies were screened and designated pPRO005.
6. Plasmid pPRO005 was extracted, and 100 ng of the extracted plasmid was mixed with NotI and XhoI restriction enzymes. The mixture was incubated at 37 °C for 1 hour and subsequently identified by agarose gel electrophoresis, as shown in Figure 16.

### (II) Recombination-Mediated Integration of the lacUV5-Controlled Constitutive T7 RNA Polymerase Expression Cassette into the asd gene locus on the Salmonella Chromosome

1. Single colonies of SWT1001, SWT1005, SWT2009, and SWT2013 were separately picked and inoculated into 5 mL LB liquid medium containing 100 µg/mL D-alanine, and cultured for 16 hours in a constant temperature shaker at 32°C and 220 rpm.
2. The culture was inoculated into fresh LB liquid medium at a ratio of 1:100, and cultivation was continued for 2-3 hours until the bacterial density reached an OD₆₀₀ of 0.3, at which point the culture flask was placed in a 42°C water bath and incubated with shaking for 15 minutes, then placed on ice to stand for 1 hour.
3. The bacterial cells were washed 3 times with sterilized pure water.
4. Prepare the PCR product of SWTO102 and SWTO103.

Primers SWTO102 and SWTO103 were mixed with the plasmid pPRO005 and a high-fidelity PCR amplification enzyme system, and the mixture was placed into a PCR amplification instrument.

The amplification program was: 95°C for 2 minutes; 30 cycles of 95°C for 30 seconds, 60°C for 30 seconds, and 72°C for 120 seconds; followed by 72°C for 10 minutes; and 4°C for 5 minutes.

The amplified PCR products were verified by agarose gel electrophoresis. The verification results of the PCR amplification products using SWTO102 and SWTO103 are shown in Figure 17A.

The amplified PCR product was purified and recovered using a DNA gel recovery system, and the concentration and purity of the recovered PCR product DNA were measured using a Nanodrop spectrophotometer.

5. The recovered bacterial cells were mixed with 100 ng of the PCR product of SWTO102 and SWTO103, and electroporation was performed at a voltage of 1.8 kV.

6. The electroporated bacterial cells were spread onto plates containing 25 µg/mL chloramphenicol, 100µg/ml D-alanine and 100 µg/mL DAP (diaminopimelic acid), and cultured overnight in a constant temperature incubator at 32°C until single colonies grew.

7. The single colonies were inoculated into 5 mL of LB medium containing 100 µg/mL DAP and 100µg/ml D-alanine and cultured in a constant temperature shaker at 37°C and 220 rpm for 16 hours to allow the CRE enzyme to function and excise the chloramphenicol resistance gene.

8. Positive clones were identified by colony PCR using primers SWT1O4, SWT1O6, and SWT1O5, SWT1O7.

The PCR amplification products of the target fragment of strains SWT1001-T7P, SWT1005-T7P, SWT2009-T7P, SWT2013-T7P were verified by agarose gel electrophoresis, and the verification results are shown in Figure 17B.

### Example 7: Construction of a Eukaryotic Therapeutic mRNA/DNA Mixed Dual-Delivery Cytoplasmic Enhanced Green Fluorescent Protein (EGFP) Expression Vector pEU010 and Control Vector pEU011

### 1. Construction of the control vector pEU011 for eukaryotic therapeutic mRNA single-delivery to the cytoplasm

In this example, to verify the system for eukaryotic therapeutic mRNA single-delivery to the cytoplasm, a control plasmid vector pEU011 was constructed. It carries a eukaryotic expression EGFP as an example, does not contain self-amplifying regulation, and lacks antibiotic resistance selection, as shown in FIG. 18A.

The pUC replicon (SEQ ID NO: 36) and the *Salmonella asd* gene (SEQ ID NO: 11) were adopted as the balanced-lethal control mechanism. Additionally, dual bacteriophage-derived T7 promoters were utilized to respectively control the expression of the therapeutic protein (in plasmid pEU011, this element is replaced by EGFP, SEQ ID NO: 17) and the membrane-disrupting protein genes LLO (SEQ ID NO: 13).

The specific operational steps are as follows:
(1) Plasmid pPRO006 (synthesized by Beijing Liuhe BGI Tech Co., Ltd.) was digested with restriction endonucleases *Xho*I and *Not*I, and an approximately 820 bp fragment containing the pUC replicon (SEQ ID No. 36) was recovered.
(2) Plasmid pEU001 (synthesized by Beijing Liuhe BGI Tech Co., Ltd.) was digested with *Spe*I and *Xho*I, and a 2831 bp fragment containing the *Salmonella asd* gene and its regulatory region, as well as the LLO gene and RBS site (SEQ ID No. 15), was recovered.
(3) Plasmid pEU002 (synthesized by Beijing Liuhe BGI Tech Co., Ltd.) was digested with *Not*I and *Nco*I, and an approximately 654 bp fragment containing the first IRES sequence and Kozak sequence under the control of a T7 promoter (SEQ ID No. 16) was recovered.
(4) Plasmid pEGFP-N1 (purchased from Biofeng) was digested with *Nco*I and *Xba*I, and an approximately 731 bp fragment containing EGFP (SEQ ID No. 17) was recovered.
(5) Plasmid pEU002 (synthesized by Beijing Liuhe BGI Tech Co., Ltd.) was digested with *Xba*I and *Spe*I, and an approximately 281 bp fragment containing the 3'-UTR regions, a 50 bp polyA sequence, a T7 terminator, and a second T7 promoter (SEQ ID No. 18) was recovered.
(6) The above five fragments were ligated using T4 DNA ligase, transformed into competent SWT1005-T7P cells, and spread onto LB agar plates supplemented with 100 µg/mL D-alanine. Positive monoclonal colonies were screened and designated SWT5015C.
(7) Single colonies were picked and amplified using primer pairs SWTO129/SWTO130 and SWTO131/SWTO132, followed by identification via agarose gel electrophoresis. As shown in Figure 19, the resulting plasmid was designated pEU011.

### 2. Construction of Expression Vector pEU010 for Mixed Dual-Delivery of Eukaryotic Drug mRNA/DNA into the Cytoplasm

In this example, to validate the system for mixed dual-delivery of eukaryotic drug mRNA/DNA into the cytoplasm, plasmid vector pEU010 was constructed, which carries EGFP for eukaryotic expression as a model and contains no antibiotic resistance selection markers, as shown in Figure 20.

The pUC replicon (SEQ ID No. 36) and the *Salmonella asd* gene (SEQ ID No. 11) were used as the balanced-lethal selection system. Dual bacteriophage-derived T7 promoters were used to separately control the transcription of the therapeutic protein (replaced by EGFP in plasmid pEU010, SEQ ID No. 17) and T7 RNA polymerase (SEQ ID No. 20), as well as the expression of the membrane-disrupting protein gene LLO (SEQ ID No. 13).

The detailed procedures are as follows:
(1) Plasmid pEU011 was digested with *Xba*I and *Sac*I, and an approximately 5322 bp fragment was recovered.
(2) Plasmid pEU002 (synthesized by Beijing Liuhe BGI Tech Co., Ltd.) was amplified using primer pair SWTO133/SWTO134, and an approximately 613 bp fragment containing an IRES sequence was recovered. This fragment, designated the second IRES sequence (SEQ ID No. 19), was retrieved by digestion with *Xba*I and *Eco*RI.
(3) Plasmid pPRO004 (synthesized by Beijing Liuhe BGI Tech Co., Ltd.) was amplified using primer pair SWTO135/SWTO136, and an approximately 2670 bp fragment containing the T7 RNA polymerase coding sequence was recovered. This fragment, corresponding to the T7 RNA polymerase sequence driven by the second IRES sequence (SEQ ID No. 20), was retrieved by digestion with *Eco*RI and *Sac*I.
(4) The above three fragments were ligated using T4 DNA ligase, transformed into competent SWT1005-T7P cells, and spread onto LB agar plates supplemented with 100 µg/mL D-alanine. Positive monoclonal colonies were screened and designated SWT5015.
(5) Single colonies were picked and amplified using primer pairs SWTO137/SWTO134 and SWTO137/SWTO138, followed by identification via agarose gel electrophoresis. As shown in Figure 21, the resulting plasmid was designated pEU010, with the nucleotide sequence set forth in SEQ ID No. 21.

### 3. Construction of the Cytoplasmic Expression Vector from Reference Patent US10987432B2

The structure of the cytoplasmic expression vector disclosed in Patent US10987432B2 is shown in Figure 22. Plasmid pIKDE-EGFP (SEQ ID No. 22) was synthesized according to the sequence provided in the patent by Beijing Liuhe BGI Tech Co., Ltd.

### Example 8: Comparison of Expression Intensity of Expression Vector pEU010 and Control Vectors pEU011 and pIKDE-EGFP in BSR-T7/5 Golden Hamster Kidney Cells Stably Expressing T7 Polymerase

In this example, to verify the difference in EGFP expression intensity among the eukaryotic therapeutic mRNA/DNA mixed dual-delivery cytoplasmic expression vector pEU010, the eukaryotic therapeutic mRNA single-delivery cytoplasmic control vector pEU011, and the cytoplasmic expression vector pIKDE-EGFP from patent US10987432B2, these three plasmids were respectively transfected into BSR-T7/5 golden hamster kidney cells stably expressing T7 RNA polymerase (purchased from Shanghai Qiansi Biotechnology Co., Ltd.). Because BSR-T7/5 endogenously carries T7 RNA polymerase, it can directly transcribe the genes on the plasmids in the cytoplasm. The advantages and disadvantages of the different systems were verified by comparing the intensity of EGFP green fluorescence expression.

The specific operational steps are as follows:
1. BSR-T7/5 cell culture. A 24-well plate was used for culturing. The culture medium was DMEM (purchased from Shanghai Yuanpei Biotechnology Co., Ltd., Cat. No. L110KJ) supplemented with 10% FBS (purchased from US EVERBRIGHT, Cat. No. H9043). One day (18-24 hours) prior to transfection, cells were seeded at approximately 100,000 to 300,000 cells per well into the 24-well plate and cultured to allow the cell density to reach approximately 70-80% the following day. Two hours before transfection, the medium in each well of the 24-well plate containing the cells was replaced with 1 ml of fresh complete culture medium containing serum and penicillin-streptomycin antibiotics (purchased from Shanghai Yuanpei Biotechnology Co., Ltd., Cat. No. S110JV).
2. A clean centrifuge tube was taken. For the cells in each well of the 24-well plate to be transfected, 25 µl of Opti-MEM (purchased from Gibco, Cat. No. 31985-070) was added, along with 500 ng of plasmid pEU10, pEU11, or pIKDE-EGFP, respectively, with a DNA concentration of 0.5-5 µg/µl. The mixture was gently pipetted to ensure uniform mixing. Next, 0.8 µl of Lipo8000^{™} transfection reagent (purchased from Shanghai Beyotime Biotechnology Co., Ltd., Cat. No. C0533) was added and gently pipetted to mix. The resulting DNA and Lipo8000 mixture were uniformly added dropwise into each well according to the aforementioned dosage, and culturing was continued.
3. After continuing the culture for approximately 48 hours, the expression intensity of green fluorescence was observed using a fluorescence microscope, and the proportion of fluorescent cells was analyzed via flow cytometry.

The fluorescence microscopy results are shown in Figure 23. Figure 23A is the BSR-T7/5 cell control, with the white light image on top and the fluorescence image on the bottom, showing no fluorescence signal. Figure 23B shows BSR-T7/5 cells transfected with pEU010, revealing a large number of cells with fluorescence signals. Figure 23C shows BSR-T7/5 cells transfected with pEU011, revealing cells with fluorescence signals, but the number is significantly lower than that in Figure 23B. Figure 23D shows BSR-T7/5 cells transfected with pIKDE-EGFP, revealing cells with fluorescence signals, but the number is significantly lower than that in Figure 23B.

The flow cytometry analysis results are shown in Figure 24, where the FITC channel indicates green fluorescent protein positivity, and the P3 gate is defined as green fluorescent protein-positive cells. Figure 24A shows BSR-T7/5 cells transfected with pEU010, exhibiting a positive cell proportion of 44.1%. Figure 24B shows BSR-T7/5 cells transfected with pEU011, exhibiting a positive cell proportion of 35.7%. Figure 24C shows BSR-T7/5 cells transfected with pIKDE-EGFP, exhibiting a positive cell proportion of 34.8%.

According to the two experiments described above, the plasmids of all three systems can achieve the synthesis of the target protein in background cells expressing T7 RNA polymerase. The eukaryotic therapeutic mRNA/DNA mixed dual-delivery cytoplasmic expression vector pEU010 demonstrates a significant advantage compared to the other two control vectors.

### Example 9: Construction of Strain SWT5025 Carrying the Control Vector pIKDE-EGFP as a Control Strain According to Patent US10987432B2

### 1. Construction of YB1-like Salmonella (SWT005)

In order to reconstruct the strain carrying the control vector pIKDE-EGFP from patent US10987432B2, the first step is to construct the chassis strain of the original YB1.

The YB1-like *Salmonella* strain includes an *aroA* gene defect and an oxygen regulation system constructed by the forward hypoxic promoter *pepT*, the essential gene *asd*, and the reverse hyperoxic promoter *sodA*.

### (1)Construction of Plasmid pSWT004

① Construction of plasmid pSWT004 is shown in Figure 25. It contains a chloramphenicol resistance gene (*cm*) flanked by *loxP* sequence*s* (SEQ ID NO: 39), the *pepT* promoter (SEQ ID NO: 41), the *asd* gene (SEQ ID NO: 42), and the reverse *sodA* promoter (SEQ ID NO: 43).
② Plasmid pSWT002 was digested with NotI, and a 1130 bp fragment was recovered. Strain SWT001 was amplified using primers SWTO11 and SWTO12; the product was double-digested with NotI and HindIII and recovered.

Strain SWT001 was amplified using primers SWTO13 and SWTO14; the product was double-digested with XhoI and HindIII and recovered.

Primers SWTO15 and SWTO16 were directly annealed to generate XhoI and PstI overhangs and recovered.
③ Plasmid pSWT003 (vector backbone) was double-digested with NotI and PstI. The five fragments were ligated using T4 DNA ligase. The product was spread onto LB plates containing both ampicillin and chloramphenicol. Single colonies were screened and named pSWT004 (YB1-like construction plasmid, including *cm-pepT-asd-sodA*).
④ Plasmid pSWT004 was extracted, and 100 ng was mixed with SacI and KpnI endonuclease systems and incubated at 37°C for 1 hour. Identification was performed via agarose gel electrophoresis, as shown in Figure 26.

### (2)Construction of Recombinant YB1-like salmonella SWT005(YB1-like)

Plasmid pSWT004 was amplified using primers SWTO17 and SWTO18 and recovered. This fragment was recombined into SWT003 to construct the YB1-like *Salmonella* SWT005.
① A single colony of SWT003 was picked and inoculated into 5 mL of LB liquid medium and cultured in a constant temperature shaker at 32°C and 220 rpm for 16 hours.
② The culture was inoculated at a ratio of 1:100 into fresh LB liquid medium and cultured for a further 2-3 hours until the bacterial density reached OD₆₀₀ = 0.3. The culture flask was placed in a 42°C water bath and incubated with shaking for 15 minutes, then placed on ice to rest for 1 hour.
③ The bacterial cells were washed 3 times with sterilized purified water and set aside.
④ pSWT004 was amplifi*e*d using primers SWTO17 and SWTO18, purified, and recovered. The recovered PCR product of the *cm*-*pepT*-*asd*-*sodA* fragment generated by SWTO17 and SWTO18 was verified by agarose gel electrophoresis. The verification results are shown in Figure 27. The DNA concentration and purity were determined using a nanodrop.
⑤ The recovered bacterial cells were mixed with 100 ng of the PCR product and electroporated at a voltage of 1.8 kV.
⑥ The electroporated bacterial cells were spread onto plates containing 25 µg/mL chloramphenicol and 100 µg/mL DAP (diaminopimelic acid), and cultured overnight in a constant temperature incubator at 32°C until single colonies grew.
⑦ Positive clones were identified by colony PCR. Primers SWTO93 and SWTO5, and SWTO94 and SWTO95 were used to verify the insertion of the chloramphenicol resistance gene. The results of the PCR amplification product verification for *Salmonella* SWT005 (using SWTO93 and SWTO5, SWTO94 and SWTO95) by agarose gel electrophoresis are shown in Figure 28.

### (3)Oxygen Adaptability Verification of YB1-like Salmonella SWT005

① A single colony of YB1-like *Salmonella* SWT005 was picked and inoculated into 5 mL of LB liquid medium containing 100 µg/mL DAP (diaminopimelic acid) and cultured in a constant temperature shaker at 37°C and 200 rpm for 16 hours.
② After culturing, the strain was diluted 10-fold, and the absorbance (OD value) at 600 nm was measured.
③ The volume of bacterial liquid (in microliters) corresponding to 1 OD of bacteria was calculated according to the following formula, and deionized water was added to reach 1 mL:
   $Volume of bacterial culture for 1 OD = 1 / \left(Absorbance\times 10\right) \times 1000$
④ 10 µL of the above bacterial suspension was spotted onto two LB plates without DAP, labeled as "1". This was repeated three times, labeled "a", "b", and "c".
⑤ After a 10-fold serial dilution, 10 µL of the diluted solution was spotted onto the medium, labeled as "2".
⑥ This 10-fold serial dilution was continued until label "8".
⑦ One plate was placed in an anaerobic jar at 37°C. The operation involved placing an anaerobic gas-generating bag (Brand: Mitsubishi, Japan, Cat. No.: D-119) into a 7.0 L sealed culture jar (Brand: Mitsubishi, Japan, Cat. No.: D-112) and culturing for 24 hours in an anoxic environment (oxygen concentration less than 0.01%). The other plate was cultured for 24 hours in an atmospheric environment (21% oxygen concentration) at 37°C.

The results of the oxygen adaptability verification test for YB1-like *Salmonella* SWT005 are shown in Figure 29. From the oxygen adaptability verification data of YB1-like *Salmonella* SWT005, it can be concluded that: YB1-like *Salmonella* SWT005 grew normally on LB plates without DAP under anoxic conditions (as shown in Figure 29(A)). Under aerobic (atmospheric) conditions, distinct strain growth was observed at concentration 1 (10⁻² OD), concentration 2 (10⁻³ OD), concentration 3 (10⁻⁴ OD), and concentration 4 (10⁻⁵ OD) (as shown in Figure 29(B)).

Therefore, it can be seen that YB1-like *Salmonella* SWT005 achieves the regulation of strain growth in an aerobic environment, which is consistent with previous reports regarding YB1.

### 2. Construction of strain SWT008 by introducing a T7 RNA polymerase expression system controlled by a placUV5 promoter at the chromosomal gmd locus on the chassis of YB1-like Salmonella (SWT005)

Since the *asd* gene locus in strain SWT005 is occupied by the hypoxia-regulated gene cassette, the T7 RNA polymerase gene under the control of the *plac*UV5 promoter was integrated at the *gmd* locus with reference to Patent US10987432B2.

The detailed procedures are as follows:
(1) A single colony of SWT005 was inoculated into 5 mL of liquid LB medium supplemented with 100 µg/mL DAP, and cultured in a constant-temperature shaker at 32 °C, 220 rpm for 16 h.
(2) The culture was inoculated at a ratio of 1:100 into fresh liquid LB medium supplemented with 100 µg/mL DAP and cultured for an additional 2-3 h until OD₆₀₀ = 0.3. The culture flask was transferred to a 42 °C water bath, incubated with shaking for 15 min, then placed on ice for 1 h.
(3) The bacterial cells were washed three times with sterile purified water.
(4) Preparation of PCR product using primers SWTO139 and SWTO140.

Primers SWTO139 and SWTO140, plasmid pPRO005, and a high-fidelity PCR amplification enzyme mixture were mixed and subjected to PCR amplification.

The amplification program is 95°C for 2 minutes; 30 cycles of 95°C for 30 seconds, 60°C for 30 seconds, 72°C for 60 seconds; 72°C for 10 minutes; 4°C for 5 minutes.

The amplified PCR product is purified and recovered through a DNA gel recovery system, and the concentration and purity of the recovered PCR product DNA are determined via NanoDrop.

(5) The recovered bacterial cells were mixed with 100 ng of the PCR product amplified with SWTO139 and SWTO140, and electroporation was performed at 1.8 kV.

(6) The electroporated cells were spread onto agar plates supplemented with 25 µg/mL chloramphenicol and 100 µg/mL DAP, and cultured overnight in a 32 °C constant-temperature incubator until single colonies formed.

(7) Single colonies were inoculated into 5 mL of liquid LB medium supplemented with 100 µg/mL DAP and cultured at 37 °C, 220 rpm for 16 h. The chloramphenicol resistance gene was excised via Cre recombinase activity to generate strain SWT008.

(8) Positive clones were identified by colony PCR using primer pairs SWTO141/SWTO106 and SWTO142/SWTO107.

The target PCR amplicons of strain SWT008 were verified by agarose gel electrophoresis as shown in Figure 30.

### 3. Construction of strain SWT5025 carrying the control vector pIKDE-EGFP

With reference to Patent US10987432B2, to ensure stable plasmid maintenance in *Salmonella*, pIKDE-EGFP contains the *infA* gene as an essential gene. The *infA* gene encodes one of the protein translation initiation factors and is critical for viability. Therefore, construction of strain SWT5025 required transformation of plasmid pIKDE-EGFP into strain SWT008, followed by knockout of the chromosomal *infA* gene to maintain a balanced-lethal system.

In addition, in Patent US10987432B2, expression of the LLO gene is driven chromosomally by the promoter of the *sseA* gene (SEQ ID No. 40) from *Salmonella* pathogenicity island II. The *sseA* promoter is a strong promoter that is induced upon bacterial invasion into host cells. Accordingly, vector pPRO013 was constructed in the present invention, which contains the LLO gene driven by the *sseA* promoter, as well as a chloramphenicol resistance gene flanked by direct repeat *loxp* sequences. This fragment was PCR-amplified and recombined into the chromosomal *infA* locus of SWT008 to generate strain SWT5025.

The detailed procedures are as follows:
(1) Plasmid pIKDE-EGFP was transformed into competent SWT008 cells, which were spread onto LB agar plates supplemented with 100 µg/mL DAP and 100 µg/mL ampicillin sodium. Single colonies were screened and designated SWT009.
(2) A single colony of SWT009 was inoculated into 5 mL of liquid LB medium supplemented with 100 µg/mL DAP and 100 µg/mL ampicillin sodium, and cultured in a constant-temperature shaker at 32 °C, 220 rpm for 16 h.
(3) The culture was inoculated at a ratio of 1:100 into fresh liquid LB medium supplemented with 100 µg/mL DAP and cultured for an additional 2-3 h until OD₆₀₀ = 0.3. The culture flask was transferred to a 42 °C water bath, incubated with shaking for 15 min, then placed on ice for 1 h.
(4) The bacterial cells were washed three times with sterile purified water.
(5) Preparation of PCR product amplified from pPRO013 using primers SWTO143 and SWTO144.

Plasmid pPRO013 was constructed as shown in Figure 31,
① Plasmid pPRO009 (synthesized by Beijing Liuhe Huada Gene Technology Co., Ltd.) was double-digested with *Nde*I and *Pst*I, and an approximately 1600-bp fragment containing the *Salmonella*-codon-optimized LLO gene was recovered.
② Plasmid pPRO011 (synthesized by Beijing Liuhe Huada Gene Technology Co., Ltd.) was double-digested with *Not*I and *Nde*I, and an approximately 425-bp fragment containing the *sseA* promoter and RBS sequence was recovered as the promoter for LLO.
③ Plasmid pSWT002 was digested with *Not*I, and a 1130-bp fragment was recovered.
④ Plasmid pSWT003 (vector backbone) was double-digested with *Not*I and *Pst*I. Four fragments were ligated using T4 DNA ligase. The ligation product was spread onto LB agar plates supplemented with ampicillin and chloramphenicol for double selection, and single colonies were screened and designated pPRO013.

(6) Primers SWTO143 and SWTO144, plasmid pPRO013, and a high-fidelity PCR amplification enzyme mixture were combined and subjected to PCR amplification.

Amplification program: 95 °C for 2 min; 30 cycles of 95 °C for 30 s, 60 °C for 30 s, 72 °C for 60 s; 72 °C for 10 min; 4 °C for 5 min.

The amplified PCR product was purified using a DNA gel recovery kit. DNA concentration and purity were determined using a NanoDrop spectrophotometer.

(7) The recovered competent cells were mixed with 100 ng of the PCR product amplified with SWTO143 and SWTO144, and electroporation was performed at 1.8 kV.

(8) The electroporated cells were spread onto agar plates supplemented with 25 µg/mL chloramphenicol and 100 µg/mL DAP, and cultured overnight in a 32 °C constant-temperature incubator until single colonies formed.

(9) Single colonies were inoculated into 5 mL of liquid LB medium supplemented with 100 µg/mL DAP and cultured at 37 °C, 220 rpm for 16 h. The chloramphenicol resistance gene was excised via Cre recombinase activity to generate strain SWT5025.

(10) Positive clones were identified by colony PCR using primer pairs SWTO145/SWTO5 and SWTO146/SWTO116.

The target PCR amplicons of strain SWT5025 were verified by agarose gel electrophoresis as shown in Figure 32.

### Example 10: Comparison of Various Properties and Cellular Presentation Efficiency Among Strain SWT5015, SWT5015C and SWT5025

In the present example, the properties of strain SWT5015 harboring the eukaryotic therapeutic mRNA/DNA mixed dual-delivery cytoplasmic enhanced green fluorescent protein (EGFP) expression vector pEU010, strain SWT5015C harboring the control vector pEU011, and strain SWT5025 harboring the control vector pIKDE-EGFP were compared in terms of strain stability, membrane-disrupting protein genes expression rate, and EGFP expression efficiency. The characteristics of these three systems are described in Table 2.

**Table 2 Comparison of Characteristics Among Strains SWT5015, SWT5015C and SWT5025**

| | SWT5015 | SWT5015C | SWT5025 |
|---|---|---|---|
| Target gene expression pattern | mRNA/DNA mixed dual delivery into cytoplasm | mRNA delivery into cytoplasm | mRNA/DNA mixed dual delivery into cytoplasm and nucleus |

| Nuclear entry requirement | Not required | Not required | Required; contains CMV promoter and polyA signal, necessitating nuclear regulation |
|---|---|---|---|
| Self-amplifying regulatory mode | Self-amplification via single mRNA bicistron | No self-amplification | Self-amplification via dual mRNA monocistrons |
| LLO expression mode | Controlled by T7 promoter, co-expressed with mRNA | Controlled by T7 promoter, co-expressed with mRNA | Controlled by *sseA* promoter, induced upon bacterial invasion into host cells |
| LLO expression locus | Multi-copy plasmid | Multi-copy plasmid | Chromosome |
| Oxygen regulation mode | Combined regulation of *yhbU-S + alr + ydcI-S* | Combined regulation of *yhbU-S + alr + ydcI-S* | YB1-like *pepT-asd-sodA* regulation |
| Gene expression process | As shown in Figure 20B: | As shown in Figure | As shown in Figure 22B: |
| | 1. Within the bacterium, constitutively expressed T7 RNA polymerase drives transcription of a single mRNA encoding LLO and the eukaryotic therapeutic molecule; | 18B: | 1. Within the bacterium, constitutively expressed T7 RNA polymerase drives transcription of the eukaryotic therapeutic mRNA and the eukaryotic T7 RNA polymerase mRNA; |
| | | 1. Within the bacterium, constitutively expressed T7 RNA polymerase drives transcription of a single mRNA encoding LLO and the eukaryotic therapeutic molecule; | |
| | | | 2. Upon bacterial invasion into host cells, chromosomally encoded LLO under control of the *sseA* promoter is induced. Expressed LLO causes phagosomal rupture, releasing monocistronic eukaryotic therapeutic mRNA, eukaryotic T7 RNA polymerase mRNA, and plasmid DNA into the cytoplasm. However, bacterially synthesized mRNAs lack a polyA tail and exhibit extremely short half-lives, thus exerting no significant effect; |
| | 2. Upon bacterial invasion into host cells, LLO induces phagosomal rupture, releasing the bicistronic mRNA and plasmid DNA into the cytoplasm, where the eukaryotic therapeutic molecule is concurrently translated into protein; | | |
| | | 2. Upon bacterial invasion into host cells, LLO induces phagosomal rupture, releasing the mRNA, and the eukaryotic therapeutic molecule is concurrently translated into protein. | |
| | 3. T7 RNA polymerase encoded by the mRNA is translated into mature polymerase protein; | | |
| | | | 3. A portion of plasmid DNA enters the nucleus, activates the CMV promoter, and drives synthesis of mature therapeutic mRNA and T7 RNA polymerase mRNA, which are exported to the cytoplasm for translation; Mature T7 RNA polymerase mRNA is translated into protein that directly acts on cytoplasmic plasmid DNA to generate polyA-deficient mRNA, which supplements EGFP expression from the nucleus. |
| | 4. T7 RNA polymerase acts on DNA delivered into the cytoplasm to further promote transcription, establishing a cytoplasmic self-amplification cascade. | | |

### 1. Comparison of Stability Among Strains SWT5015, SWT5015C and SWT5025

Both strain SWT5015 and strain SWT5025 employ a self-amplifying regulatory mode based on eukaryotically expressed T7 RNA polymerase carried on plasmids. Although the expression level of eukaryotically expressed T7 RNA polymerase is very low in host bacteria, the constitutively expressed T7 RNA polymerase on the chromosome tends to trigger cyclic self-amplifying transcription in the host bacterium, thereby depleting intracellular resources and leading to bacterial death.

In the present example, using SWT1005 as a control strain, the stability of SWT5015, SWT5015C and SWT5025 was evaluated by comparing their growth and viability in normal culture medium.

The detailed procedures are as follows:
(1) Single colonies of SWT1005, SWT5015 and SWT5015C were picked and inoculated into 5 mL of LB liquid medium supplemented with 100 µg/mL D-alanine, respectively. A single colony of SWT5025 was picked and inoculated into 5 mL of LB liquid medium supplemented with 100 µg/mL DAP and 100 µg/mL ampicillin sodium. All cultures were incubated in a constant-temperature shaker at 37 °C, 220 rpm for 16 hours.
(2) The cultures of SWT1005, SWT5015, SWT5015C and SWT5025 were inoculated at a ratio of 1:1000 into fresh liquid medium and transferred to a 96-well cell culture plate. The plate was incubated continuously at 37 °C, 220 rpm for 24 hours. Bacterial density (OD₆₀₀) was measured and recorded hourly using a microplate reader. The growth profiles are shown in Figure 33A.
(3) After incubation, each strain was diluted 10-fold, and the absorbance at 600 nm (OD value) was measured. The volume of bacterial suspension corresponding to 1 OD unit was calculated according to the following formula, and deionized water was added to a final volume of 1 mL.$Volume of bacterial culture for 1 OD = 1 / \left(Absorbance\times 10\right) \times 1000$
(4) Ten microliters of the above bacterial suspensions of SWT1005, SWT5015 and SWT5015C were spotted onto an LB plate supplemented with D-alanine, and 10 µL of SWT5025 suspension was spotted onto an LB plate supplemented with DAP, labeled as "1". The spotting was performed in triplicate, labeled as "a", "b" and "c". After 10-fold serial dilution, 10 µL of each dilution was spotted onto the corresponding medium and labeled as "2". Serial 10-fold dilutions were continued in this manner up to label "8". The growth status is shown in Figure 33B.

Results show: No significant differences were observed between SWT5015, SWT5015C and the control strain SWT1005 in either the growth rate in liquid culture or colony counts by spot plating. This demonstrates that the self-amplifying regulatory mode of SWT5015 exerts no adverse effect on strain stability. In contrast, SWT5025 exhibited severely retarded growth in liquid culture, accompanied by flocculent precipitates in the medium consisting of dead bacterial cells, resulting in a significantly lower overall OD reading in the late culture stage compared with SWT5015, SWT5015C and the control strain SWT1005.

In addition, at equivalent OD values, the viable cell count of SWT5025 was approximately 100-fold lower (only 1% of the viable cell count) than that of the other strains in spot plating assays. These results indicate that the self-amplifying regulatory mode of SWT5025 severely impairs bacterial viability.

### 2. Comparison of EGFP mRNA Transcription in Strains SWT5015, SWT5015C and SWT5025

Since strains SWT5015, SWT5015C and SWT5025 are all capable of delivering mRNA and constitutively express T7 RNA polymerase, mRNA encoding the target protein EGFP is present in the bacterial cells. In the present example, therefore, the mRNA levels of eukaryotically expressed EGFP in these three strains were determined by reverse transcription quantitative real-time PCR (RT-qPCR) using SWT1005 as a control strain.

The detailed procedures are as follows:
(1) Single colonies of SWT1005, SWT5015 and SWT5015C were picked and inoculated into 5 mL of LB liquid medium supplemented with 100 µg/mL D-alanine, respectively. A single colony of SWT5025 was picked and inoculated into 5 mL of LB liquid medium supplemented with 100 µg/mL DAP and 100 µg/mL ampicillin sodium. All cultures were incubated in a constant-temperature shaker at 37 °C, 220 rpm for 16 hours.
(2) Each culture was transferred at a ratio of 1:100 into a centrifuge tube containing 1 mL of the corresponding fresh medium, and cultured at 32 °C, 200 rpm for 4-6 hours until the OD₆₀₀ reached 0.5-1.0.
(3) The bacterial cultures were centrifuged at 12,000 rpm for 2 minutes at 4 °C, and all supernatants were carefully removed.
(4) The cell pellets were thoroughly resuspended (by pipetting) in 100 µL of TE buffer containing lysozyme (purchased from Shanghai Beyotime Biotechnology Co., Ltd., Cat. No. ST206), and incubated at room temperature for 5 minutes.
(5) To each tube, 300 µL of Lysis Buffer (GeneJET RNA Purification Kit, purchased from Thermo, Cat. No. K0731) was added, followed by vortexing to homogeneity.
(6) 180 µL of absolute ethanol was added to each tube and mixed by pipetting. The entire liquid volume was transferred to an RNA purification column (GeneJET RNA Purification Kit, Thermo, Cat. No. K0731), centrifuged at 12,000 rpm for 1 minute, and the flow-through was discarded. The column was transferred to a new 2 mL collection tube.
(7) 700 µL of Wash Buffer 1 (GeneJET RNA Purification Kit, Thermo, Cat. No. K0731) was added to the column, which was centrifuged at 12,000 rpm for 1 minute, and the flow-through was discarded.
(8) 600 µL of Wash Buffer 2 (GeneJET RNA Purification Kit, Thermo, Cat. No. K0731) was added to the column, which was centrifuged at 12,000 rpm for 1 minute, and the flow-through was discarded.
(9) The column was transferred to a new 1.5 mL centrifuge tube, and 50 µL of DEPC-treated water (purchased from Shanghai Beyotime Biotechnology Co., Ltd., Cat. No. R0022) was added. After standing at room temperature for 1 minute, the column was centrifuged at 12,000 rpm for 1 minute, and the column was discarded.
(10) The RNA tubes were placed on ice. After measuring RNA concentration and purity using a NanoDrop spectrophotometer, 500 ng of RNA was subjected to 1% agarose gel electrophoresis to assess integrity. The identification results are shown in Figure 34A.
(11) Residual DNA was removed from the RNA samples. 1 µL of the extracted RNA was mixed with 1 µL of DNase I (purchased from Thermo, Cat. No. EN0521) and 1 µL of DNase I buffer, adjusted to 10 µL with DEPC-treated water, incubated at 37 °C for 30 minutes, and inactivated at 65 °C for 10 minutes.
(12) 1 µL of RNA from the previous step was mixed with primers SWTO147 and SWTO148 and Luna Universal qPCR Master Mix (purchased from NEB, Cat. No. M3003S) in a qPCR reaction mixture, and subjected to real-time fluorescence PCR.
   Amplification program: 95 °C for 60 seconds; 40 cycles of 95 °C for 15 seconds, 60 °C for 30 seconds.
(13) No reverse transcription step was performed in this step, in order to exclude interference from residual DNA. The results are shown in Figure 34B.
(14) 1 µL of RNA from Step (11) was mixed with primers SWTO147 and SWTO148 and Luna Universal One-Step RT-qPCR Kit (purchased from NEB, Cat. No. E3005S) in a reaction mixture, and subjected to real-time fluorescence PCR.

Amplification program: 55 °C for 10 minutes; 95 °C for 60 seconds; 40 cycles of 95 °C for 15 seconds, 60 °C for 30 seconds. This procedure first converts mRNA to cDNA via reverse transcription, followed by amplification of the target gene. The results are shown in Figure 34B.

The above experimental results show that mRNA encoding the target gene EGFP was present in strains SWT5015, SWT5015C and SWT5025, with no significant difference in mRNA levels among the three strains.

### 3. Comparison of Expression Efficiencies of T7 RNA Polymerase and LLO (a Membrane-Disrupting Protein) in Strains SWT5015, SWT5015C and SWT5025

T7 RNA polymerase in strains SWT5015, SWT5015C and SWT5025 is all chromosomally expressed under the control of the constitutive *lac*UV5 promoter. However, expression of LLO in strains SWT5015 and SWT5015C is regulated by the T7 promoter and located on plasmids of expression vectors. This configuration enables synchronous expression with mRNA of the target protein, and sufficient LLO protein accumulated in bacterial cells can maximize the overall delivery *e*fficiency of the bacteria. In contrast, LLO in SWT5025 is chromosomally controlled by the promoter of the *sseA* gene from *Salmonella* pathogenicity island II (SEQ ID No. 40) and is induced upon bacterial invasion into host cells.

In the present example, expression of T7 RNA polymerase and LLO protein in strains SWT5015, SWT5015C and SWT5025 was examined in conventional LB medium and in the *sseA* promoter-inducing medium N-salts (which contains low-concentration magnesium ions to simulate the intracellular environment after bacterial invasion and induce expression from the *sseA* promoter; composition: 5 mM KCl, 7.5 mM (NH₄)₂SO₄, 0.5 mM K₂SO₄, 100 mM Bis-Tris/HCl (pH 7.0), 38 mM glycerol, and 0.1% Casamino Acids, supplemented with 30 µM MgCl₂).

The detailed procedures are as follows:
(1) Single colonies of SWT5015 and SWT5015C were picked and inoculated into 5 mL of LB liquid medium supplemented with 100 µg/mL D-alanine, respectively. A single colony of SWT5025 was picked and inoculated into 5 mL of LB liquid medium supplemented with 100 µg/mL DAP and 100 µg/mL ampicillin sodium. All cultures were incubated in a constant-temperature shaker at 37 °C, 220 rpm for 16 hours.
(2) A 1 mL aliquot of each culture was centrifuged at 12,000 rpm for 1 minute, the supernatant was discarded, and the pellet was collected for later use.
(3) Another 1 mL aliquot of each culture was centrifuged at 4,000 rpm for 5 minutes, the supernatant was removed, and the pellet was resuspended in 5 mL of freshly prepared N-salts induction medium. The medium for SWT5015 and SWT5015C was supplemented with 100 µg/mL D-alanine; the medium for SWT5025 was supplemented with 100 µg/mL DAP and 100 µg/mL ampicillin sodium. Each suspension was incubated in a constant-temperature shaker at 37 °C, 220 rpm for 24 hours.
(4) A 5 mL aliquot of each culture grown in N-salts induction medium was centrifuged at 12,000 rpm for 1 minute, the supernatant was discarded, and the pellet was collected for later use.
(5) Samples from Steps (2) and (4) were resuspended in lysis buffer (protein loading buffer, purchased from CST, Cat. No. 7722S), heated at 95 °C for 10 minutes, centrifuged at 12,000 rpm again, and the supernatant was collected for later use.
(6) A 5 µL aliquot of the supernatant collected above was loaded onto an SDS-PAGE gel (SDS-PAGE Gel Preparation Kit, purchased from Sangon Biotech, Cat. No. C631100-0200), and electrophoresis was performed at 120 V for 60 minutes.
(7) The resolved SDS-PAGE gel was transferred onto a PVDF membrane (purchased from Roche, Cat. No. 03010040001) at a constant current of 250 mA for 30 minutes.
(8) The PVDF membrane after transfer was blocked in 5% skimmed milk on an orbital shaker at 80 rpm for 1 hour at room temperature.
(9) The blocked PVDF membrane was incubated separately with either Anti-T7 RNA Polymerase antibody (anti-T7 RNA polymerase antibody, purchased from Creative Biomart, Cat. No. CABT-B8990) or Anti-listeriolysin antibody (anti-LLO antibody, purchased from Abcam, Cat. No. ab200538) as the primary antibody, diluted in 5% skimmed milk, at 4 °C for 18 hours (one antibody per membrane).
(10) The secondary antibody was diluted in 5% skimmed milk. The PVDF membrane after primary antibody incubation was transferred to the secondary antibody dilution and incubated on an orbital shaker at 80 rpm for 1 hour at room temperature.
(11) The PVDF membrane after secondary antibody incubation was washed three times with TBST Buffer (purchased from Sangon Biotech, Cat. No. C520009-0005), 5 minutes each time. ECL developer (SignalFire^{™} ECL Reagent, purchased from CST, Cat. No. 6883P) was added dropwise onto the washed PVDF membrane, followed by incubation in the dark for 1 minute. After incubation, imaging was performed on a chemiluminescence imager.

Western blot analysis showed that under both LB and N-salts culture conditions, strains SWT5015, SWT5015C and SWT5025 all expressed T7 RNA polymerase at high levels, as shown in Figure 35A. Strains SWT5015 and SWT5015C expressed LLO at high levels under both LB and N-salts conditions, as shown in Figure 35B, whereas SWT5025 expressed LLO only under N-salts culture conditions, as shown in Figure 35B. The spatiotemporal difference in LLO expression may affect the efficiency of mRNA/DNA delivery after bacterial invasion.

### 4. Comparison of EGFP Expression Efficiency in Cancer Cells Following Invasion by Strains SWT5015, SWT5015C and SWT5025

In the above examples, the stability and the expression efficiencies of T7 RNA polymerase and LLO in strains SWT5015, SWT5015C and SWT5025 were verified respectively. Although differences were observed, further cell invasion assays are required to evaluate the drug delivery efficiency.

In the present example, SWT1005 was used as a negative control, and strains SWT5015, SWT5015C and SWT5025 were co-cultured with CT26 cancer cells respectively, so as to analyze the differences in delivery efficiency based on the expression of fluorescent protein.

The detailed procedures are as follows:
(1) Single colonies of SWT1005, SWT5015 and SWT5015C were picked and inoculated into 5 mL of LB liquid medium supplemented with 100 µg/mL D-alanine, respectively. A single colony of SWT5025 was picked and inoculated into 5 mL of LB liquid medium supplemented with 100 µg/mL DAP and 100 µg/mL ampicillin sodium. All cultures were incubated in a constant-temperature shaker at 37 °C, 220 rpm for 16 hours.
(2) A volume of culture corresponding to 1 OD unit was collected and washed three times with PBS.
(3) 2.5×10⁶ CT26 cancer cells were seeded into each well of a 6-well cell culture plate, and cultured overnight in complete medium (DMEM basic medium supplemented with 10% fetal bovine serum and double antibiotics) in a 37 °C incubator with 5% CO₂.
(4) After cell counting in two wells, strains SWT1005, SWT5015, SWT5015C and SWT5025 were added respectively at a multiplicity of infection (MOI) of 200:1. The plates were placed in an anaerobic jar with oxygen concentration below 0.8% and co-cultured for 2 hours to allow bacterial invasion. The cells were then carefully washed with an equal volume of PBS, the supernatant was aspirated, and DMEM medium containing 100 µg/mL gentamicin was added. The cells were further cultured for 24 hours in an anaerobic jar with oxygen concentration below 0.8%, and photographed under a fluorescence microscope as shown in Figure 36A.
(5) The invaded cells were digested with 0.25% trypsin (purchased from Shanghai Yuanpei Biotechnology Co., Ltd., Cat. No. S310JV), resuspended in 100 µL PBS, transferred to a black flat-bottom 96-well plate, and subjected to green fluorescence signal measurement using a microplate reader at room temperature (emission wavelength: 518 nm, excitation wavelength: 518 nm, reading time fixed at 800 ms). The results are shown in Figure 36B.

The above results show that no fluorescence signal was detected in the negative control group SWT1005 by microplate reader analysis. EGFP signals were detected in strains SWT5015, SWT5015C and SWT5025, among which SWT5015 exhibited the strongest signal. Owing to the relatively low efficiency of eukaryotic system delivery by bacteria, only the mRNA/DNA mixed dual cytoplasmic delivery system of SWT5015 achieved sufficiently high efficiency to enable obvious observation of fluorescence signals under a fluorescence microscope. Therefore, it can be concluded that SWT5015 exhibits the optimal delivery efficiency.

### Example 11: Construction of Vector pEU013 for Eukaryotic Therapeutic mRNA/DNA Mixed Dual Delivery into Cytoplasm with GSDMD-N and Delivery Strains SWT5115 and SWT5119, Construction of Vector pEU014 with GSDME-N and Delivery Strains SWT5215 and SWT5219, and Verification of mRNA Transcription in the Above Strains

In the present example, to verify the ability of the eukaryotic therapeutic mRNA/DNA mixed dual delivery system for cytoplasmic delivery of GSDM-N, vector pEU013 carrying GSDMD-N and delivery strains SWT5115 and SWT5119 were constructed, as well as vector pEU014 carrying GSDME-N and delivery strains SWT5215 and SWT5219.

### 1. Construction of Plasmid Vector pEU013 and Strains SWT5115 and SWT5119

The pUC origin of replication (SEQ ID No. 36) and the *Salmonella asd* gene (SEQ ID No. 11) were adopted as the balanced-lethal control mechanism, and dual phage-derived T7 promoters were used to separately control the transcription of the therapeutic protein GSDMD-N (SEQ ID No. 23) and T7 RNA polymerase (SEQ ID No. 20), as well as the expression of membrane-disrupting protein gene LLO (SEQ ID No. 13).

The detailed procedures are as follows:
(1) Plasmid pEU010 was digested with NcoI and XbaI, and a fragment of approximately 7869 bp was recovered.
(2) Plasmid pEU003 (synthesized by Beijing Liuhe Huada Gene Technology Co., Ltd.) was digested with NcoI and XbaI, and a fragment of approximately 828 bp containing the GSDMD-N sequence was recovered.
(3) The above two fragments were ligated using T4 DNA ligase, transformed into competent SWT1005-T7P cells, spread onto LB plates supplemented with 100 µg/mL D-alanine, and single colonies were screened and designated SWT5115.
(4) Single colonies were picked and amplified using primer pairs SWTO129/SWTO149 and SWTO150/SWTO151, followed by identification via agarose gel electrophoresis as shown in Figure 37. The resulting plasmid was designated pEU013.
(5) Plasmid pEU013 was extracted, transformed into competent SWT2009-T7P cells, spread onto LB plates supplemented with 100 µg/mL D-alanine, and single colonies were screened and designated SWT5119.

### 2. Construction of Plasmid Vector pEU014 and Strains SWT5215 and SWT5219

The pUC origin of replication (SEQ ID No. 36) and the *Salmonella asd* gene (SEQ ID No. 11) were adopted as the balanced-lethal control mechanism, and dual phage-derived T7 promoters were used to separately control the transcription of the therapeutic protein GSDME-N (SEQ ID No. 24) and T7 RNA polymerase (SEQ ID No. 20), as well as the expression of membrane-disrupting protein gene *LLO* (SEQ ID No. 13).

The detailed procedures are as follows:
(1) Plasmid pEU010 was digested with *Nco*I and *Xba*I, and a fragment of approximately 7869 bp was recovered.
(2) Plasmid pEU004 (synthesized by Beijing Liuhe Huada Gene Technology Co., Ltd.) was digested with *Nco*I and *Xba*I, and a fragment of approximately 816 bp containing the GSDME-N sequence was recovered.
(3) The above two fragments were ligated using T4 DNA ligase, transformed into competent SWT1005-T7P cells, spread onto LB plates supplemented with 100 µg/mL D-alanine, and single colonies were screened and designated SWT5215.
(4) Single colonies were picked and amplified using primer pairs SWTO129/SWTO152 and SWTO150 /SWTO153, followed by identification via agarose gel electrophoresis as shown in Figure 37. The resulting plasmid was designated pEU014.
(5) Plasmid pEU014 was extracted, transformed into competent SWT2009-T7P cells, spread onto LB plates supplemented with 100 µg/mL D-alanine, and single colonies were screened and designated SWT5219.

### 3. Verification of GSDMD-N mRNA Transcription in Strains SWT5115 and SWT5119 and GSDME-N mRNA Transcription in Strains SWT5215 and SWT5219

Strains SWT5115, SWT5119, SWT5215 and SWT5219 are all capable of mRNA delivery and constitutively express T7 RNA polymerase, resulting in the presence of mRNA encoding the eukaryotically expressed target proteins GSDMD-N and GSDME-N in bacterial cells. In the present example, using SWT1005 as a control, the mRNA levels of eukaryotically expressed GSDMD-N and GSDME-N in strains SWT5115 and SWT5215 were determined by reverse transcription quantitative real-time PCR (RT-qPCR). Meanwhile, using SWT2009 as a control, the mRNA levels of eukaryotically expressed GSDMD-N and GSDME-N in strains SWT5119 and SWT5219 were also determined by RT-qPCR.

The detailed procedures are as follows:
(1) Single colonies of SWT1005, SWT5115, SWT5215, SWT2009, SWT5119 and SWT5219 were picked and inoculated into 5 mL of LB liquid medium supplemented with 100 µg/mL D-alanine, respectively, and cultured in a constant-temperature shaker at 37 °C, 220 rpm for 16 hours.
(2) Each culture was transferred at a ratio of 1:100 into a centrifuge tube containing 1 mL of corresponding fresh medium, and cultured at 32 °C, 200 rpm for 4-6 hours until the OD₆₀₀ reached 0.5-1.0.
(3) The bacterial cultures were centrifuged at 12,000 rpm for 2 minutes at 4 °C, and all supernatants were carefully removed.
(4) The cell pellets were thoroughly resuspended (by pipetting) in 100 µL of TE buffer containing lysozyme (purchased from Shanghai Beyotime Biotechnology Co., Ltd., Cat. No. ST206), and incubated at room temperature for 5 minutes.
(5) To each tube, 300 µL of Lysis Buffer (GeneJET RNA Purification Kit, purchased from Thermo, Cat. No. K0731) was added, followed by vortexing to homogeneity.
(6) 180 µL of absolute ethanol was added to each tube and mixed by pipetting. The entire liquid volume was transferred to an RNA purification column (GeneJET RNA Purification Kit, Thermo, Cat. No. K0731), centrifuged at 12,000 rpm for 1 minute, and the flow-through was discarded. The column was transferred to a new 2 mL collection tube.
(7) 700 µL of Wash Buffer 1 (GeneJET RNA Purification Kit, Thermo, Cat. No. K0731) was added to the column, which was centrifuged at 12,000 rpm for 1 minute, and the flow-through was discarded.
(8) 600 µL of Wash Buffer 2 (GeneJET RNA Purification Kit, Thermo, Cat. No. K0731) was added to the column, which was centrifuged at 12,000 rpm for 1 minute, and the flow-through was discarded.
(9) The column was transferred to a new 1.5 mL centrifuge tube, and 50 µL of DEPC-treated water (purchased from Shanghai Beyotime Biotechnology Co., Ltd., Cat. No. R0022) was added. After standing at room temperature for 1 minute, the column was centrifuged at 12,000 rpm for 1 minute, and the column was discarded.
(10) The RNA tubes were placed on ice. After measuring RNA concentration and purity using a NanoDrop spectrophotometer, 500 ng of RNA was subjected to 1% agarose gel electrophoresis to assess integrity. The identification results are shown in Figure 34A.
(11) Residual DNA was removed from the RNA samples. 1 µL of the extracted RNA was mixed with 1 µL of DNase I (purchased from Thermo, Cat. No. EN0521) and 1 µL of DNase I buffer, adjusted to 10 µL with DEPC-treated water, incubated at 37 °C for 30 minutes, and inactivated at 65 °C for 10 minutes.
(12) 1 µL of RNA from the previous step was mixed with primers SWTO154 & SWTO155 (for identification of GSDMD-N mRNA) and SWTO156 & SWTO157 (for identification of GSDME-N mRNA) and Luna Universal qPCR Master Mix (purchased from NEB, Cat. No. M3003S) in a qPCR reaction mixture, and subjected to real-time fluorescence PCR.
   Amplification program: 95 °C for 60 seconds; 40 cycles of 95 °C for 15 seconds, 60 °C for 30 seconds.
(13) No reverse transcription step was performed in this step to exclude interference from residual DNA. The results are shown in Figure 38.
(14) 1 µL of RNA from Step (11) was mixed with primers SWTO154 & SWTO155 (for identification of GSDMD-N mRNA) and SWTO156 & SWTO157 (for identification of GSDME-N mRNA) and Luna Universal One-Step RT-qPCR Kit (purchased from NEB, Cat. No. E3005S) in a reaction mixture, and subjected to real-time fluorescence PCR.

Amplification program: 55 °C for 10 minutes; 95 °C for 60 seconds; 40 cycles of 95 °C for 15 seconds, 60 °C for 30 seconds. This procedure first converts mRNA to cDNA via reverse transcription, followed by amplification of the target gene. The results are shown in Figure 38.

The above experimental results show that strains SWT5115 and SWT5215 contained mRNA transcribed from the first expression cassette of target genes GSDMD-N (Figure 38A) and GSDME-N (Figure 38B), respectively, with no significant difference in mRNA levels. Strains SWT5119 and SWT5219 contained mRNA transcribed from the first expression cassette of target genes GSDMD-N (Figure 38C) and GSDME-N (Figure 38D), respectively, with no significant difference in mRNA levels.

The results of the present example demonstrate that strains carrying different hypoxia-specific expression cassettes exhibit no effect on the mRNA transcribed from their first expression cassette.

### Example 12: Verification of Pyroptosis and Cytotoxic Effects of Strains SWT5115 and SWT5215 on Various Cancer Cell Lines

To verify the cytotoxic effects of strains SWT5115 and SWT5215 on various cancer cell lines under in vitro hypoxic conditions, co-culture with cancer cells was performed, with strain SWT1005 used as a control strain.

The detailed procedures are as follows:
1. Strains SWT1005, SWT5115 and SWT5215 were streaked onto LB agar plates supplemented with 100 µg/mL D-alanine, and statically cultured overnight in a 37 °C constant-temperature incubator.
2. Single colonies of SWT1005, SWT5115 and SWT5215 were picked and inoculated into 5 mL of fresh LB liquid medium containing 100 µg/mL D-alanine, and cultured in a constant-temperature shaker at 37 °C, 220 rpm for 16 hours.
3. The absorbance of each culture at 600 nm was measured. A volume of culture corresponding to an OD₆₀₀ of 1.0 was collected, washed twice with PBS and once with the corresponding cell culture medium, then resuspended in cell culture medium at a multiplicity of infection (MOI) of 200:1.
4. The above bacterial strains were separately co-cultured with the following cancer cell lines for 2 hours under an oxygen concentration below 0.8%: breast cancer (EMT6), hepatocellular carcinoma (Hepa1-6), lung cancer (A549), melanoma (SK-MEL-5), renal cancer (Renca), gastric cancer (MFC), pancreatic cancer (Pan02), prostate cancer (RM-1), colon cancer (CT26), ovarian cancer (ID8), neuroblastoma (Neuro-2a), squamous cell carcinoma (SCC7), bladder cancer (MB49), and osteosarcoma (K7M2). Cells were then washed three times with PBS containing gentamicin, and further cultured for 24 hours in an anaerobic jar with oxygen concentration below 0.8%. Photographs were taken under bright-field microscopy using a 40× objective lens, as shown in Figures 39-43.
5. CCK8 reagent was mixed with the above cell suspensions and incubated for 1 hour.
6. Absorbance was measured using a microplate reader at a detection wavelength of 450 nm, and the percentage of cytotoxicity was calculated.

The cytotoxic effects of strains SWT1005, SWT5115 and SWT5215 on EMT6, Hepa1-6, A549, SK-MEL-5, Renca, MFC, Pan02, RM-1, CT26, ID8, Neuro-2a, SCC7, MB49 and K7M2 cells are shown in Figure 44.

Statistical analysis was performed using Student's t-test. Significance levels were defined as follows: *p*<0.05 (*) , *p*<0.01 (**) , *p*<=0.001 (***) .

The above results show that *Salmonella* strains SWT5115 and SWT5215 harboring the expression vectors both exert significant cytotoxicity against various cancer cell lines. Significant cellular pyroptosis (indicated by arrows) was observed under bright-field microscopy, whereas no obvious pyroptosis was observed in the SWT1005 control group.

In addition, CCK8 viability assay revealed that the cytotoxicity of SWT5115 and SWT5215 was significantly stronger than that of the control strain SWT1005.These results indicate that Salmonella strains utilizing the eukaryotic therapeutic mRNA/DNA mixed dual cytoplasmic delivery system for GSDM-N exhibit broad-spectrum pyroptosis-inducing and cytotoxic activity against cancer cells.

### Example 13: Verification of Pyroptosis and Cytotoxic Effects of Strains SWT5119 and SWT5219 on Various Cancer Cell Lines

To verify the cytotoxic effects of strains SWT5119 and SWT5219 on various cancer cell lines under in vitro hypoxic conditions, co-culture with cancer cells was performed, with strain SWT2009 used as a control strain.

The detailed procedures are as follows:
1. Strains SWT2009, SWT5119 and SWT5219 were streaked onto LB agar plates supplemented with 100 µg/mL D-alanine, and statically cultured overnight in a 37 °C constant-temperature incubator.
2. Single colonies of SWT2009, SWT5119 and SWT5219 were picked and inoculated into 5 mL of fresh LB liquid medium containing 100 µg/mL D-alanine, and cultured in a constant-temperature shaker at 37 °C, 220 rpm for 16 hours.
3. The absorbance of each culture at 600 nm was measured. A volume of culture corresponding to an OD₆₀₀ of 1.0 was collected, washed twice with PBS and once with the corresponding cell culture medium, then resuspended in cell culture medium at a multiplicity of infection (MOI) of 200:1.
4. The above bacterial strains were separately co-cultured with the following cancer cell lines for 2 hours under an oxygen concentration below 0.8%: breast cancer (EMT6), hepatocellular carcinoma (Hepa1-6), lung cancer (A549), melanoma (SK-MEL-5), renal cancer (Renca), gastric cancer (MFC), pancreatic cancer (Pan02), prostate cancer (RM-1), colon cancer (CT26), ovarian cancer (ID8), neuroblastoma (Neuro-2a), squamous cell carcinoma (SCC7), bladder cancer (MB49), and osteosarcoma (K7M2). Cells were then washed three times with PBS containing gentamicin, and further cultured for 24 hours in an anaerobic jar with oxygen concentration below 0.8%. Photographs were taken under bright-field microscopy using a 40× objective lens, as shown in Figures 45-49.
5. CCK8 reagent was mixed with the above cell suspensions and incubated for 1 hour.
6. Absorbance was measured using a microplate reader at a detection wavelength of 450 nm, and the percentage of cytotoxicity was calculated.

The cytotoxic effects of strains SWT2009, SWT5119 and SWT5219 on EMT6, Hepa1-6, A549, SK-MEL-5, Renca, MFC, Pan02, RM-1, CT26, ID8, Neuro-2a, SCC7, MB49 and K7M2 cells are shown in Figure 50.

Statistical analysis was performed using Student's t-test. Significance levels were defined as follows: *p*<0.05 (*) , *p*<0.01 (**) , *p*<=0.001 (***) .

The above results show that Salmonella strains SWT5119 and SWT5219 harboring the expression vectors both exert significant cytotoxicity against various cancer cell lines. Significant cellular pyroptosis (indicated by arrows) was observed under bright-field microscopy, whereas no obvious pyroptosis was observed in the SWT2009 control group.

In addition, CCK8 viability assay revealed that the cytotoxicity of SWT5119 and SWT5219 was significantly stronger than that of the control strain SWT2009.These results indicate that Salmonella strains utilizing the eukaryotic therapeutic mRNA/DNA mixed dual cytoplasmic delivery system for GSDM-N exhibit broad-spectrum pyroptosis-inducing and cytotoxic activity against cancer cells.

The present example also demonstrates that strains carrying different hypoxia-specific expression cassettes exhibit no difference in cytotoxicity, and the cytotoxic effect is derived from the eukaryotic expression vector carried by the bacteria.

### Example 14: Evaluation of Tumor Suppressive Effects of Strains SWT5115, SWT5215 and Control Strain SWT1005 in Mouse Tumor Models

To verify the inhibitory effects of strains carrying the expression vector on tumor growth in mice, the present invention employed SWT5115 and SWT5215 as representatives to perform tumor inhibition assays in mouse models of EMT6, K7M2, Hepa1-6, A549, B16F10, Renca, MFC, Pan02, RM-1, CT26, ID8, Neuro-2a, SCC7 and MB49 tumors.

### 1. Establishment of Multiple Mouse Tumor Models

### (1) Establishment of the EMT6 Tumor Mouse Model

BALB/c mice (purchased from Zhejiang Vital River Laboratory Animal Technology Co., Ltd., body weight approximately 18 g, housed in a specific pathogen-free (SPF) environment) were subcutaneously inoculated with 1×10⁶ EMT6 cancer cells to establish a mouse breast cancer subcutaneous tumor model. Experiments were initiated 14-18 days after inoculation when tumor volumes reached approximately 100 mm³. Mice were randomly divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### (2) Establishment of the K7M2 Tumor Mouse Model

BALB/c mice (purchased from Zhejiang Vital River Laboratory Animal Technology Co., Ltd., body weight approximately 18 g, housed in an SPF environment) were subcutaneously inoculated with 1×10⁶ K7M2 cancer cells to establish a mouse subcutaneous tumor model. Experiments were initiated 14-18 days after inoculation when tumor volumes reached approximately 100 mm³. Mice were divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### (3) Establishment of the Hepa1-6 Tumor Mouse Model

C57BL/6 mice (purchased from Zhejiang Vital River Laboratory Animal Technology Co., Ltd., body weight approximately 18 g, housed in an SPF environment) were subcutaneously inoculated with 1×10⁶ Hepa1-6 cancer cells to establish a mouse subcutaneous tumor model. Experiments were initiated 14-18 days after inoculation when tumor volumes reached approximately 100 mm³. Mice were divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### (4) Establishment of the A549 Tumor Mouse Model

Nu/Nu nude mice (purchased from Zhejiang Vital River Laboratory Animal Technology Co., Ltd., body weight approximately 18 g, housed in an SPF environment) were subcutaneously inoculated with 1×10⁶ A549 cancer cells to establish a mouse subcutaneous tumor model. Experiments were initiated 14-18 days after inoculation when tumor volumes reached approximately 100 mm³. Mice were divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### (5) Establishment of the B16F10 Tumor Mouse Model

C57BL/6 mice (purchased from Zhejiang Vital River Laboratory Animal Technology Co., Ltd., body weight approximately 18 g, housed in an SPF environment) were subcutaneously inoculated with 1×10⁶ B16F10 cancer cells to establish a mouse subcutaneous melanoma model. Experiments were initiated 14-18 days after inoculation when tumor volumes reached approximately 100 mm³. Mice were divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### (6) Establishment of the Renca Tumor Mouse Model

BALB/c mice (purchased from Zhejiang Vital River Laboratory Animal Technology Co., Ltd., body weight approximately 18 g, housed in an SPF environment) were subcutaneously inoculated with 1×10⁶ Renca cancer cells to establish a mouse subcutaneous tumor model. Experiments were initiated 14-18 days after inoculation when tumor volumes reached approximately 100 mm³. Mice were divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### (7) Establishment of the MFC Tumor Mouse Model

BALB/c mice (purchased from Zhejiang Vital River Laboratory Animal Technology Co., Ltd., body weight approximately 18 g, housed in an SPF environment) were subcutaneously inoculated with 1×10⁶ MFC cancer cells to establish a mouse subcutaneous tumor model. Experiments were initiated 14-18 days after inoculation when tumor volumes reached approximately 100 mm³. Mice were divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### (8) Establishment of the Pan02 Tumor Mouse Model

C57BL/6 mice (purchased from Zhejiang Vital River Laboratory Animal Technology Co., Ltd., body weight approximately 18 g, housed in an SPF environment) were subcutaneously inoculated with 1×10⁶ Pan02 cancer cells to establish a mouse subcutaneous tumor model. Experiments were initiated 14-18 days after inoculation when tumor volumes reached approximately 100 mm³. Mice were divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### (9) Establishment of the RM-1 Tumor Mouse Model

C57BL/6 mice (purchased from Zhejiang Vital River Laboratory Animal Technology Co., Ltd., body weight approximately 18 g, housed in an SPF environment) were subcutaneously inoculated with 1×10⁶ RM-1 cancer cells to establish a mouse subcutaneous tumor model. Experiments were initiated 14-18 days after inoculation when tumor volumes reached approximately 100 mm³. Mice were divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### (10) Establishment of the CT26 Tumor Mouse Model

BALB/c mice (purchased from Zhejiang Vital River Laboratory Animal Technology Co., Ltd., body weight approximately 18 g, housed in an SPF environment) were subcutaneously inoculated with 1×10⁶ CT26 cancer cells to establish a mouse subcutaneous tumor model. Experiments were initiated 14-18 days after inoculation when tumor volumes reached approximately 100 mm³. Mice were divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### (11) Establishment of the ID8 Tumor Mouse Model

C57BL/6 mice (purchased from Zhejiang Vital River Laboratory Animal Technology Co., Ltd., body weight approximately 18 g, housed in an SPF environment) were subcutaneously inoculated with 1×10⁶ ID8 cancer cells to establish a mouse subcutaneous tumor model. Experiments were initiated 14-18 days after inoculation when tumor volumes reached approximately 100 mm³. Mice were divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### (12) Establishment of the Neuro-2a Tumor Mouse Model

A/J mice (purchased from Zhejiang Vital River Laboratory Animal Technology Co., Ltd., body weight approximately 18 g, housed in an SPF environment) were subcutaneously inoculated with 1×10⁶ Neuro-2a cancer cells to establish a mouse subcutaneous tumor model. Experiments were initiated 14-18 days after inoculation when tumor volumes reached approximately 100 mm³. Mice were divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### (13) Establishment of the SCC7 Tumor Mouse Model

Nu/Nu nude mice (purchased from Zhejiang Vital River Laboratory Animal Technology Co., Ltd., body weight approximately 18 g, housed in an SPF environment) were subcutaneously inoculated with 1×10⁶ SCC7 cancer cells to establish a mouse subcutaneous tumor model. Experiments were initiated 14-18 days after inoculation when tumor volumes reached approximately 100 mm³. Mice were divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### (14) Establishment of the MB49 Tumor Mouse Model

C57BL/6 mice (purchased from Zhejiang Vital River Laboratory Animal Technology Co., Ltd., body weight approximately 18 g, housed in an SPF environment) were subcutaneously inoculated with 1×10⁶ MB49 cancer cells to establish a mouse subcutaneous tumor model. Experiments were initiated 14-18 days after inoculation when tumor volumes reached approximately 100 mm³. Mice were divided into 4 groups: PBS control group, SWT1005 group, SWT5115 group, and SWT5215 group, with 5 mice per group. Tumor-bearing mice were intravenously injected via the tail vein with 1×10⁷ CFU of bacteria in a volume of 125 µL.

### 2. Preparation and Administration of Bacteria

Single colonies of the constructed *Salmonella* strains SWT1005, SWT5115 and SWT5215 were picked and inoculated into 5 mL of LB liquid medium supplemented with D-alanine, followed by incubation in a constant-temperature shaker at 37 °C, 220 rpm for 16 hours. The absorbance of each culture at 600 nm was measured, and a volume corresponding to an OD₆₀₀ of 1.0 was collected and washed three times with PBS. Model mice were administered 1×10⁷ CFU of bacteria in a volume of 125 µL via tail vein injection. The day of administration was defined as Day 0. The length and width of mouse tumors were measured on Days 1, 3, 5, 7, 9 and 11 after administration, and tumor volumes were calculated according to the following formula:$Tumor volume \left({mm}^{3}\right) = length \times {width}^{2} \times 0.52$

The results of tumor inhibition in mouse tumor models among SWT1005, SWT5115 and SWT5215 are shown in Figures 51-54.

Statistical analysis was performed using Student's t-test. Significant differences were indicated as follows: *p*<0.05 (*) , *p*<0.01 (**) , *p*<=0.001 (***) .

The results demonstrate that the *Salmonella* strains SWT5115 and SWT5215 harboring the expression vector exert significant tumor growth inhibitory effects, which are superior to those of the control strain SWT1005 and the untreated control group.

Information on the corresponding strains of the present invention is shown in Table 3.

**Table 3 List of Constructed Strains**

| **Strain Name** | **Characteristic Description** | **Source (Description)** |
|---|---|---|
| SWT001 | Wild-type *Salmonella* | Purchased from CICC |
| SWT002 | Wild-type *Salmonella* SWT001 transformed with temperature-inducible lambda-RED recombinase and loxP-Cre recombinase system (plasmid pSWT001) | Constructed |
| SWT003 | Derived from wild-type SWT001, *aroA* gene knockout | Constructed |
| SWT004 | Derived from wild-type SWT001, *aroA* gene knockout, *alr* gene knockout | Constructed |
| SWT005 | Derived from wild-type SWT001, *aroA* gene knockout, *asd* gene knockout; oxygen-regulated system *pepT-asd-sodA* (derived from plasmid pSWT004) integrated at the *asd* locus (△*asd*::*pepT-asd-sodA*), serving as a YB1-like strain | Constructed |
| SWT007 | Derived from wild-type SWT001, *aroA* gene knockout, *dadX* gene knockout | Constructed |
| SWT008 | Derived from SWT005, *gmd*::*plac*UV5-T7 RNA polymerase | Constructed |
| SWT009 | Derived from SWT005, *gmd*::*plac*UV5-T7 RNA polymerase, transformed with pIKDE-EGFP | Constructed |
| SWT5025 | Derived from SWT005, *gmd*::*plac*UV5-T7 RNA polymerase, *infA* gene knockout, transformed with plasmid pIKDE-EGFP | Constructed |
| SWT1001 | Derived from wild-type SWT001, *aroA* gene knockout, *alr* gene knockout, *dadX* gene knockout; oxygen-regulated system *yhbU-S-alr-cyoA-S* (derived from plasmid pOL1001) integrated at the *dadX* locus (△*dadX::yhbU*-*S*-*alr*-*cyoA*-*S*) | Constructed |
| SWT1005 | Derived from wild-type SWT001, *aroA* gene knockout, *alr* gene knockout, *dadX* gene knockout; oxygen-regulated system *yhbU-S-alr-ydcI-S* (derived from plasmid pOL1005) integrated at the *dadX* locus (△*dadX::yhbU*-*S*-*alr*-*ydcI*-*S*) | Constructed |
| SWT2009 | Derived from wild-type SWT001, *aroA* gene knockout, *alr* gene knockout, *dadX* gene knockout; oxygen-regulated system *ynfK-E-dadX-cyoA-S* (derived from plasmid pOL2009) integrated at the *alr* locus (△*alr*::*yhbU-S-dadX-cyoA-S*) | Constructed |
| SWT2013 | Derived from wild-type SWT001, *aroA* gene knockout, *alr* gene knockout, *dadX* gene knockout; oxygen-regulated system *ynfK-E-dadX-ydcI-S* (derived from plasmid pOL2013) integrated at the *alr* locus (△*alr*::*ynfK-E-dadX-ydcI-S*) | Constructed |
| SWT1001-T7P | Derived from SWT1001, *asd* gene knockout, T7 RNA polymerase integrated at the *asd* locus (△*asd*::*lac*UV5-T7P) | Constructed |
| SWT1005-T7P | Derived from SWT1005, *asd* gene knockout, T7 RNA polymerase integrated at the *asd* locus (△*asd*::*lac*UV5-T7P) | Constructed |
| SWT2009-T7P | Derived from SWT2009, *asd* gene knockout, T7 RNA polymerase integrated at the *asd* locus (△*asd*::*lac*UV5-T7P) | Constructed |
| SWT2013-T7P | Derived from SWT2013, *asd* gene knockout, T7 RNA polymerase integrated at the *asd* locus (△*asd*::*lac*UV5-T7P) | Constructed |
| SWT5015 | Derived from SWT1005-T7P, transformed with plasmid pEU010 | Constructed |
| SWT5015C | Derived from SWT1005-T7P, transformed with plasmid pEU011 | Constructed |
| SWT5115 | Derived from SWT1005-T7P, transformed with plasmid pEU013 | Constructed |
| SWT5215 | Derived from SWT1005-T7P, transformed with plasmid pEU014 | Constructed |
| SWT5119 | Derived from SWT2009-T7P, transformed with plasmid pEU013 | Constructed |
| SWT5219 | Derived from SWT2009-T7P, transformed with plasmid pEU0144 | Constructed |

The tool plasmids used in the present invention are shown in Table 4.

**Table 4 Tool Plasmids Used in the Present Invention**

| **Name** | **Description** | **Source** |
|---|---|---|
| pSWT001 | Temperature-inducible recombinase and Cre recombinase system | Constructed |
| PSWT002 | Vector containing two direct repeat *loxP* sequences flanking a chloramphenicol resistance gene, with *Not*I restriction sites on both sides for gene knockout after amplification | Constructed |
| pSWT003 | pBluescript cloning and construction backbone vector | Purchased from Biofeng |
| pSWT004 | pSWT1 for YB1-like application, containing cm*-pepT-asd-sodA* cassette | Constructed |
| pSWT007 | Vector containing two direct repeat *loxP* s*e*quences flanking a chloramphenicol resistance gene, with *Spe*I and *Not*I restriction sites on both sides for construction of hypoxia-specific gene expression cassette library | Constructed |
| pPRO004 | Vector containing codon-optimized T7 RNA polymerase gene | Constructed |
| pPRO005 | Vector containing T7 RNA polymerase under control of the *lac*UV5 promoter | Constructed |
| pPRO006 | Vector containing pUC origin of replication | Constructed |
| pPRO007 | Vector containing *asd* balanced-lethal control gene | Constructed |
| pPRO009 | Vector containing codon-optimized LLO | Constructed |
| pPRO011 | Vector containing *sseA* promoter and RBS | Constructed |
| pPRO013 | Expression vector with LLO under control of *sseA* promoter, containing two direct repeat *loxP* sequences flanking a chloramphenicol resistance gene, with *Not*I restriction sites on both sides for gene knockout after amplification | Constructed |
| pOL1001 | Plasmid containing *loxP*-flanked cm resistance gene sequence and *yhbU-S-alr-cyoA-S* regulatory cassette | Constructed |
| pOL1005 | Plasmid containing *loxP*-flanked cm resistance gene sequence and *yhbU-S-alr-ydcI-S* regulatory cassette | Constructed |
| pOL2009 | Plasmid containing *loxP*-flanked cm resistance gene sequence and *ynfK-E-dadX-cyoA-S* regulatory cassette | Constructed |
| pOL2013 | Plasmid containing *loxP*-flanked cm resistance gene sequence and *ynfK-E-dadX-ydcI-S* regulatory cassette | Constructed |
| pEGFP-N1 | Eukaryotic expression vector for EGFP | Purchased from Biofeng |
| pEU001 | Vector containing *asd* balanced-lethal control gene and LLO | Constructed |
| pEU002 | Vector containing first T7 promoter, first IRES sequence, 3'-UTR, polyA, T7 terminator and second T7 promoter sequence | Constructed |
| pEU003 | Vector containing synthetic GSDMD-N coding sequence | Constructed |
| pEU004 | Vector containing synthetic GSDME-N coding sequence | Constructed |
| pEU010 | Plasmid for eukaryotic EGFP expression under T7 promoter with self-enhancing T7 RNA polymerase and prokaryotic LLO expression under T7 promoter, containing T7-IRES-kozak-EGFP-IRES-kozak-T7 RNA polymerase-polyA cassette and T7-RBS-LLO | Constructed |
| pEU011 | Plasmid for eukaryotic EGFP expression under T7 promoter and prokaryotic LLO expression under T7 promoter, containing T7-IRES-kozak-EGFP-polyA cassette and T7-RBS-LLO | Constructed |
| pEU012 | pIKDE-EGFP plasmid, derived from Patent US10987432B2 | Constructed |
| pEU013 | Plasmid for eukaryotic GSDMD-N expression under T7 promoter with self-enhancing T7 RNA polymerase and prokaryotic LLO expression under T7 promoter, containing T7-IRES-kozak-GSDMD-N-IRES-kozak-T7 RNA polymerase-polyA cassette and T7-RBS-LLO | Constructed |
| pEU014 | Plasmid for eukaryotic GSDME-N expression under T7 promoter with self-enhancing T7 RNA polymerase and prokaryotic LLO expression under T7 promoter, containing T7-IRES-kozak-GSDME-N-IRES-kozak-T7 RNA polymerase-polyA cassette and T7-RBS-LLO | Constructed |

The sequences of promoters, coding regions, proteins used in the present invention are shown in Table 5.

**Table 5 Detailed sequences of promoters, gene coding regions, proteins and the like used in the present invention**

| **SEQ ID No.** | **Description** | **Sequence** |
|---|---|---|
| SEQ ID No.1 | T7 promoter | TAATACGACTCACTATAG |
| SEQ ID No.2 | T3 promoter | AATTAACCCTCACTAAAG |
| SEQ ID No.3 | SP6 promoter | ATTTAGGTGACACTATAG |
| SEQ ID No.4 | T7 RNA polymerase coding region | ATGAACACCATCAACATTGCCAAAAACGATTTTAGCGATATTGAACTGGCAG CGATTCCGTTTAATACCCTGGCAGATCATTATGGTGAACGTCTGGCACGTGA ACAGCTGGCACTGGAACATGAAAGCTATGAAATGGGTGAAGCACGTTTTCG CAAAATGTTTGAACGTCAGCTGAAAGCCGGTGAAGTTGCAGATAATGCAGC AGCAAAACCGCTGATTACCACACTGCTGCCGAAAATGATTGCACGTATTAAC GATTGGTTCGAAGAGGTTAAAGCCAAACGTGGTAAACGTCCGACCGCATTTC AGTTTCTGCAAGAAATTAAACCGGAAGCCGTTGCCTATATTACCATTAAAAC CACACTGGCATGTCTGACCAGCGCAGATAATACCACCGTTCAGGCAGTTGCA AGCGCAATTGGTCGTGCAATTGAAGATGAAGCCCGTTTTGGTCGTATTCGTG ATCTGGAAGCAAAACACTTTAAGAAAAACGTGGAAGAACAGCTGAATAAAC GTGTTGGTCACGTTTATAAGAAAGCCTTTATGCAGGTTGTTGAAGCAGATAT GCTGAGCAAAGGTCTGTTAGGTGGTGAAGCATGGTCAAGCTGGCATAAAGA AGATAGCATTCACGTTGGTGTTCGCTGTATTGAAATGCTGATTGAAAGCACC GGTATGGTTAGCCTGCATCGTCAGAATGCCGGTGTTGTTGGTCAGGATAGCG AAACCATCGAACTGGCACCGGAATATGCCGAAGCAATTGCAACCCGTGCCG GTGCACTGGCAGGTATTAGCCCGATGTTTCAGCCGTGTGTTGTTCCGCCTAA ACCGTGGACCGGCATTACCGGTGGTGGTTATTGGGCAAATGGTCGTCGTCCG CTGGCGCTGGTTCGTACCCATAGCAAAAAGGCACTGATGCGTTATGAAGATG TTTATATGCCGGAAGTGTACAAAGCCATTAACATTGCACAGAATACCGCCTG GAAAATCAATAAAAAGGTTCTGGCCGTTGCCAACGTTATCACCAAATGGAA ACATTGTCCGGTTGAAGATATTCCGGCAATTGAACGTGAAGAACTGCCGATG AAGCCGGAAGATATTGATATGAATCCGGAAGCACTGACCGCATGGAAACGT GCAGCAGCCGCAGTTTATCGTAAAGATAAAGCACGTAAAAGCCGTCGTATT AGCCTGGAATTTATGCTGGAACAGGCAAACAAATTTGCCAACCATAAAGCA ATCTGGTTCCCGTATAATATGGATTGGCGTGGTCGTGTTTATGCCGTTAGCAT GTTTAATCCGCAGGGTAATGATATGACCAAAGGCCTGCTGACCCTGGCAAAA GGTAAACCGATTGGTAAAGAAGGTTACTATTGGCTGAAAATTCATGGTGCAA ATTGTGCCGGTGTGGATAAAGTTCCGTTTCCGGAACGCATTAAATTCATCGA AGAAAACCACGAAAACATTATGGCCTGTGCAAAAAGTCCGCTGGAAAATAC CTGGTGGGCAGAACAGGATAGTCCGTTTTGTTTTCTGGCCTTTTGTTTTGAAT ATGCGGGTGTTCAGCATCATGGTCTGAGCTATAATTGTAGCCTGCCGCTGGC ATTTGATGGTAGCTGTAGCGGTATTCAGCATTTTAGCGCAATGCTGCGTGAT GAAGTTGGTGGTCGTGCCGTTAATCTGCTGCCGTCAGAAACCGTGCAGGATA TTTATGGTATTGTTGCCAAGAAAGTGAACGAAATTCTGCAAGCAGATGCAAT TAATGGCACCGATAATGAAGTTGTTACCGTGACCGATGAAAACACCGGTGA AATTAGCGAAAAAGTTAAACTGGGCACCAAAGCGCTGGCAGGTCAGTGGCT GGCCTATGGTGTTACCCGTAGCGTTACCAAACGTAGCGTGATGACACTGGCG TATGGTAGCAAAGAATTTGGTTTTCGTCAGCAGGTTCTGGAAGATACAATTC AGCCTGCAATTGATAGCGGTAAAGGTCTGATGTTTACCCAGCCGAATCAGGC AGCAGGTTATATGGCAAAACTGATTTGGGAAAGCGTTAGCGTGACCGTTGTT GCAGCAGTTGAAGCAATGAACTGGCTGAAATCAGCAGCCAAACTGCTGGCA GCAGAAGTTAAAGATAAGAAAACGGGTGAAATCCTGCGTAAACGTTGTGCC GTTCATTGGGTTACACCGGATGGTTTTCCGGTTTGGCAAGAATATAAGAAAC CGATTCAGACACGCCTGAACCTGATGTTTCTGGGTCAGTTTCGTCTGCAACC GACCATTAATACCAATAAAGATTCCGAAATCGACGCCCACAAACAAGAAAG CGGTATTGCACCGAATTTTGTTCATAGCCAGGATGGTAGCCATCTGCGTAAA ACCGTTGTTTGGGCACATGAAAAATATGGCATTGAATCCTTTGCGCTGATCC ATGATAGCTTTGGCACCATTCCGGCAGATGCAGCAAACCTGTTTAAAGCAGT TCGTGAAACAATGGTGGATACCTATGAAAGCTGTGATGTTCTGGCGGATTTT TATGATCAGTTTGCAGATCAGCTGCATGAAAGCCAGCTGGATAAAATGCCTG CACTGCCTGCCAAAGGTAATCTGAATCTGCGTGATATTCTGGAAAGCGATTT TGCCTTTGCCTAA |
| SEQ ID No.5 | T7 RNA polymerase protein sequence | MNTINIAKNDFSDIELAAIPFNTLADHYGERLAREQLALEHESYEMGEARFRKM FERQLKAGEVADNAAAKPLITTLLPKMIARINDWFEEVKAKRGKRPTAFQFLQE IKPEAVAYITIKTTLACLTSADNTTVQAVASAIGRAIEDEARFGRIRDLEAKHFKK NVEEQLNKRVGHVYKKAFMQVVEADMLSKGLLGGEAWSSWHKEDSIHVGVR CIEMLIESTGMVSLHRQNAGVVGQDSETIELAPEYAEAIATRAGALAGISPMFQP CVVPPKPWTGITGGGYWANGRRPLALVRTHSKKALMRYEDVYMPEVYKAINI AQNTAWKINKKVLAVANVITKWKHCPVEDIPAIEREELPMKPEDIDMNPEALTA WKRAAAAVYRKDKARKSRRISLEFMLEQANKFANHKAIWFPYNMDWRGRVY AVSMFNPQGNDMTKGLLTLAKGKPIGKEGYYWLKIHGANCAGVDKVPFPERIK FIEENHENIMACAKSPLENTWWAEQDSPFCFLAFCFEYAGVQHHGLSYNCSLPL AFDGSCSGIQHFSAMLRDEVGGRAVNLLPSETVQDIYGIVAKKVNEILQADAIN GTDNEVVTVTDENTGEISEKVKLGTKALAGQWLAYGVTRSVTKRSVMTLAYG SKEFGFRQQVLEDTIQPAIDSGKGLMFTQPNQAAGYMAKLIWESVSVTVVAAV EAMNWLKSAAKLLAAEVKDKKTGEILRKRCAVHWVTPDGFPVWQEYKKPIQT RLNLMFLGQFRLQPTINTNKDSEIDAHKQESGIAPNFVHSQDGSHLRKTVVWAH EKYGIESFALIHDSFGTIPADAANLFKAVRETMVDTYESCDVLADFYDQFADQL HESQLDKMPALPAKGNLNLRDILESDFAFA |
| SEQ ID No.6 | T3 RNA polymerase coding region | ATGAACATCATCGAAAACATCGAAAAGAATGACTTCTCAGAAATCGAACTG GCTGCTATCCCGTTCAACACACTGGCTGACCACTACGGAAGCGCCTTGGCTA AAGAGCAGTTGGCTTTAGAACATGAGTCTTATGAGCTAGGCGAGCGCCGCTT CCTCAAGATGCTTGAGCGTCAAGCGAAAGCTGGTGAGATTGCAGACAACGC AGCCGCTAAGCCGTTACTCGCTACGCTTCTCCCTAAGTTAACCACACGTATC GTCGAGTGGCTCGAAGAGTACGCATCGAAGAAAGGCCGCAAGCCTAGCGCA TACGCACCGCTCCAGTTACTCAAGCCGGAGGCCTCCGCGTTTATCACCCTGA AAGTTATCCTTGCGTCACTAACCAGTACGAACATGACAACCATTCAGGCCGC TGCTGGTATGCTGGGGAAAGCCATTGAGGACGAGGCACGATTTGGGCGCAT CCGTGACCTAGAAGCGAAGCACTTCAAGAAGCACGTTGAGGAACAGCTTAA CAAGCGCCACGGGCAAGTCTACAAGAAAGCATTTATGCAGGTGGTCGAGGC CGATATGATTGGTCGAGGTCTGCTTGGTGGCGAGGCGTGGTCTAGCTGGGAT AAAGAAACCACGATGCACGTAGGGATTCGCCTGATTGAAATGCTGATTGAA TCCACGGGTCTGGTGGAATTACAGCGCCACAACGCAGGTAACGCAGGCTCT GACCATGAGGCACTGCAACTGGCCCAAGAGTACGTGGACGTATTAGCGAAG CGTGCAGGCGCTCTGGCGGGTATCTCTCCGATGTTCCAGCCGTGTGTCGTAC CGCCGAAACCTTGGGTAGCAATCACAGGGGGCGGCTATTGGGCTAACGGTC GCAGACCTTTGGCACTCGTTCGCACTCACTCTAAGAAGGGCTTGATGCGCTA CGAAGACGTTTACATGCCAGAAGTCTACAAGGCTGTGAACCTCGCGCAAAA CACCGCATGGAAAATCAACAAGAAAGTTCTTGCTGTTGTCAATGAGATTGTT AACTGGAAGAATTGCCCGGTAGCAGACATTCCATCGCTGGAGCGCCAAGAG TTACCGCCTAAGCCTGACGACATTGACACCAACGAGGCAGCGCTCAAGGAG TGGAAGAAAGCCGCTGCTGGTATCTATCGCTTGGACAAGGCACGAGTGTCTC GCCGTATCAGCTTAGAGTTCATGCTGGAGCAGGCCAACAAGTTCGCAAGTAA GAAAGCAATCTGGTTCCCTTACAACATGGACTGGCGCGGTCGTGTGTACGCT GTGCCGATGTTCAACCCGCAAGGCAACGACATGACGAAAGGTCTGCTGACC CTTGCTAAAGGCAAGCCAATCGGTGAGGAAGGTTTCTACTGGCTGAAAATCC ACGGTGCGAACTGTGCGGGTGTTGATAAGGTTCCATTCCCGGAGCGCATCGC GTTCATTGAGAAGCACGTAGACGACATTCTGGCTTGCGCTAAAGACCCAATC AATAACACTTGGTGGGCTGAGCAGGATTCACCGTTCTGTTTCCTCGCGTTTTG CTTCGAGTATGCAGGCGTTACGCACCACGGTCTGAGCTACAATTGCTCTCTG CCGCTGGCGTTCGACGGGTCTTGCTCTGGTATCCAGCACTTCTCCGCGATGCT CCGCGATGAGGTAGGCGGTCGTGCGGTTAACCTGCTGCCAAGCGAAACCGT GCAGGACATTTACGGCATCGTTGCACAGAAAGTAAACGAGATTCTCAAACA GGATGCAATCAACGGCACGCCTAACGAGATGATTACCGTGACCGACAAGGA CACCGGGGAAATCTCAGAGAAGCTCAAACTTGGAACCTCAACGCTGGCGCA ACAGTGGCTGGCATATGGTGTAACCCGTAGCGTAACTAAACGTTCGGTCATG ACGCTGGCTTACGGTTCCAAGGAGTTCGGCTTTCGTCAACAGGTATTGGATG ACACCATTCAGCCTGCAATTGACAGCGGTAAGGGCTTGATGTTCACCCAACC GAACCAAGCGGCTGGCTATATGGCTAAGCTGATTTGGGATGCGGTAAGCGT GACCGTAGTTGCAGCGGTTGAGGCGATGAACTGGCTCAAATCTGCCGCTAAG CTGCTGGCTGCTGAGGTCAAGGACAAGAAGACCAAGGAGATTCTGCGCCAC CGTTGCGCGGTTCACTGGACTACGCCGGACGGCTTCCCGGTCTGGCAGGAAT ACCGCAAGCCACTCCAGAAGCGTCTCGATATGATTTTCTTAGGGCAATTCCG TCTGCAACCGACGATTAATACCCTCAAGGATTCAGGCATTGACGCACACAAG CAGGAGTCTGGCATCGCTCCTAACTTTGTTCACTCACAGGACGGTAGCCACC TCCGCATGACAGTCGTTTATGCTCACGAGAAGTATGGCATTGAGTCCTTTGC GCTCATCCATGACAGCTTTGGGACTATCCCGGCAGACGCTGGTAAGCTCTTT AAGGCTGTGCGTGAAACGATGGTTATCACCTATGAGAACAACGATGTGCTG GCAGACTTCTACTCTCAGTTTGCCGACCAGCTACACGAGACCCAACTGGACA AGATGCCTCCGCTTCCGAAGAAAGGAAACCTGAACCTGCAAGACATTCTCA AGTCTGACTTTGCCTTTGCATAA |
| SEQ ID No.7 | T3 RNA polymerase protein sequence | MNIIENIEKNDFSEIELAAIPFNTLADHYGSALAKEQLALEHESYELGERRFLKML ERQAKAGEIADNAAAKPLLATLLPKLTTRIVEWLEEYASKKGRKPSAYAPLQLL KPEASAFITLKVILASLTSTNMTTIQAAAGMLGKAIEDEARFGRIRDLEAKHFKK HVEEQLNKRHGQVYKKAFMQVVEADMIGRGLLGGEAWSSWDKETTMHVGIR LIEMLIESTGLVELQRHNAGNAGSDHEALQLAQEYVDVLAKRAGALAGISPMFQ PCVVPPKPWVAITGGGYWANGRRPLALVRTHSKKGLMRYEDVYMPEVYKAV NLAQNTAWKINKKVLAVVNEIVNWKNCPVADIPSLERQELPPKPDDIDTNEAAL KEWKKAAAGIYRLDKARVSRRISLEFMLEQANKFASKKAIWFPYNMDWRGRV YAVPMFNPQGNDMTKGLLTLAKGKPIGEEGFYWLKIHGANCAGVDKVPFPERI AFIEKHVDDILACAKDPINNTWWAEQDSPFCFLAFCFEYAGVTHHGLSYNCSLP LAFDGSCSGIQHFSAMLRDEVGGRAVNLLPSETVQDIYGIVAQKVNEILKQDAIN GTPNEMITVTDKDTGEISEKLKLGTSTLAQQWLAYGVTRSVTKRSVMTLAYGS KEFGFRQQVLDDTIQPAIDSGKGLMFTQPNQAAGYMAKLIWDAVSVTVVAAVE AMNWLKSAAKLLAAEVKDKKTKEILRHRCAVHWTTPDGFPVWQEYRKPLQKR LDMIFLGQFRLQPTINTLKDSGIDAHKQESGIAPNFVHSQDGSHLRMTVVYAHE KYGIESFALIHDSFGTIPADAGKLFKAVRETMVITYENNDVLADFYSQFADQLHE TQLDKMPPLPKKGNLNLQDILKSDFAFA |
| SEQ ID No.8 | SP6 RNA polymerase coding region | ATGCAAGATTTACACGCTATCCAGCTTCAATTAGAAGAAGAGATGTTTAATG GTGGCATTCGTCGCTTCGAAGCAGATCAACAACGCCAGATTGCAGCAGGTA GCGAGAGCGACACAGCATGGAACCGCCGCCTGTTGTCAGAACTTATTGCACC TATGGCTGAAGGCATTCAGGCTTATAAAGAAGAGTACGAAGGTAAGAAAGG TCGTGCACCTCGCGCATTGGCTTTCTTACAATGTGTAGAAAATGAAGTTGCA GCATACATCACTATGAAAGTTGTTATGGATATGCTGAATACGGATGCTACCC TTCAGGCTATTGCAATGAGTGTAGCAGAACGCATTGAAGACCAAGTGCGCTT TTCTAAGCTAGAAGGTCACGCCGCTAAATACTTTGAGAAGGTTAAGAAGTCA CTCAAGGCTAGCCGTACTAAGTCATATCGTCACGCTCATAACGTAGCTGTAG TTGCTGAAAAATCAGTTGCAGAAAAGGACGCGGACTTTGACCGTTGGGAGG CGTGGCCAAAAGAAACTCAATTGCAGATTGGTACTACCTTGCTTGAAATCTT AGAAGGTAGCGTTTTCTATAATGGTGAACCTGTATTTATGCGTGCTATGCGC ACTTATGGCGGAAAGACTATTTACTACTTACAAACTTCTGAAAGTGTAGGCC AGTGGATTAGCGCATTCAAAGAGCACGTAGCGCAATTAAGCCCAGCTTATGC CCCTTGCGTAATCCCTCCTCGTCCTTGGAGAACTCCATTTAATGGAGGGTTCC ATACTGAGAAGGTAGCTAGCCGTATCCGTCTTGTAAAAGGTAACCGTGAGCA TGTACGCAAGTTGACTCAAAAGCAAATGCCAAAGGTTTATAAGGCTATCAAC GCATTACAAAATACACAATGGCAAATCAACAAGGATGTATTAGCAGTTATTG AAGAAGTAATCCGCTTAGACCTTGGTTATGGTGTACCTTCCTTCAAGCCACT GATTGACAAGGAGAACAAGCCAGCTAACCCGGTACCTGTTGAATTCCAACA CCTGCGCGGTCGTGAACTGAAAGAGATGCTATCACCTGAGCAGTGGCAACA ATTCATTAACTGGAAAGGCGAATGCGCGCGCCTATATACCGCAGAAACTAA GCGCGGTTCAAAGTCCGCCGCCGTTGTTCGCATGGTAGGACAGGCCCGTAAA TATAGCGCCTTTGAATCCATTTACTTCGTGTACGCAATGGATAGCCGCAGCC GTGTCTATGTGCAATCTAGCACGCTCTCTCCGCAGTCTAACGACTTAGGTAA GGCATTACTCCGCTTTACCGAGGGACGCCCTGTGAATGGCGTAGAAGCGCTT AAATGGTTCTGCATCAATGGTGCTAACCTTTGGGGATGGGACAAGAAAACTT TTGATGTGCGCGTGTCTAACGTATTAGATGAGGAATTCCAAGATATGTGTCG AGACATCGCCGCAGACCCTCTCACATTCACCCAATGGGCTAAAGCTGATGCA CCTTATGAATTCCTCGCTTGGTGCTTTGAGTATGCTCAATACCTTGATTTGGT GGATGAAGGAAGGGCCGACGAATTCCGCACTCACCTACCAGTACATCAGGA CGGGTCTTGTTCAGGCATTCAGCACTATAGTGCTATGCTTCGCGACGAAGTA GGGGCCAAAGCTGTTAACCTGAAACCCTCCGATGCACCGCAGGATATCTATG GGGCGGTGGCGCAAGTGGTTATCAAGAAGAATGCGCTATATATGGATGCGG ACGATGCAACCACGTTTACTTCTGGTAGCGTCACGCTGTCCGGTACAGAACT GCGAGCAATGGCTAGCGCATGGGATAGTATTGGTATTACCCGTAGCTTAACC AAAAAGCCCGTGATGACCTTGCCATATGGTTCTACTCGCTTAACTTGCCGTG AATCTGTGATTGATTACATCGTAGACTTAGAGGAAAAAGAGGCGCAGAAGG CAGTAGCAGAAGGGCGGACGGCAAACAAGGTACATCCTTTTGAAGACGATC GTCAAGATTACTTGACTCCGGGCGCAGCTTACAACTACATGACGGCACTAAT CTGGCCTTCTATTTCTGAAGTAGTTAAGGCACCGATAGTAGCTATGAAGATG ATACGCCAGCTTGCACGCTTTGCAGCGAAACGTAATGAAGGCCTGATGTACA CCCTGCCTACTGGCTTCATCTTAGAACAGAAGATCATGGCAACCGAGATGCT ACGCGTGCGTACCTGTCTGATGGGTGATATCAAGATGTCCCTTCAGGTTGAA ACGGATATCGTAGATGAAGCCGCTATGATGGGAGCAGCAGCACCTAATTTC GTACACGGTCATGACGCAAGTCACCTTATCCTTACCGTATGTGAATTGGTAG ACAAGGGCGTAACTAGTATCGCTGTAATCCACGACTCTTTTGGTACTCATGC AGACAACACCCTCACTCTTAGAGTGGCACTTAAAGGGCAGATGGTTGCAATG TATATTGATGGTAATGCGCTTCAGAAACTACTGGAGGAGCATGAAGAGCGCT GGATGGTTGATACAGGTATCGAAGTACCTGAGCAAGGGGAGTTCGACCTTA ACGAAATCATGGATTCTGAATACGTATTTGCCTAA |
| SEQ ID No.9 | SP6 RNA polymerase protein sequence | MQDLHAIQLQLEEEMFNGGIRRFEADQQRQIAAGSESDTAWNRRLLSELIAPMA EGIQAYKEEYEGKKGRAPRALAFLQCVENEVAAYITMKVVMDMLNTDATLQAI AMSVAERIEDQVRFSKLEGHAAKYFEKVKKSLKASRTKSYRHAHNVAVVAEKS VAEKDADFDRWEAWPKETQLQIGTTLLEILEGSVFYNGEPVFMRAMRTYGGKT IYYLQTSESVGQWISAFKEHVAQLSPAYAPCVIPPRPWRTPFNGGFHTEKVASRI RLVKGNREHVRKLTQKQMPKVYKAINALQNTQWQINKDVLAVIEEVIRLDLGY GVPSFKPLIDKENKPANPVPVEFQHLRGRELKEMLSPEQWQQFINWKGECARLY TAETKRGSKSAAVVRMVGQARKYSAFESIYFVYAMDSRSRVYVQSSTLSPQSN DLGKALLRFTEGRPVNGVEALKWFCINGANLWGWDKKTFDVRVSNVLDEEFQ DMCRDIAADPLTFTQWAKADAPYEFLAWCFEYAQYLDLVDEGRADEFRTHLP VHQDGSCSGIQHYSAMLRDEVGAKAVNLKPSDAPQDIYGAVAQVVIKKNALY MDADDATTFTSGSVTLSGTELRAMASAWDSIGITRSLTKKPVMTLPYGSTRLTC RESVIDYIVDLEEKEAQKAVAEGRTANKVHPFEDDRQDYLTPGAAYNYMTALI WPSISEVVKAPIVAMKMIRQLARFAAKRNEGLMYTLPTGFILEQKIMATEMLRV RTCLMGDIKMSLQVETDIVDEAAMMGAAAPNFVHGHDASHLILTVCELVDKG VTSIAVIHDSFGTHADNTLTLRVALKGQMVAMYIDGNALQKLLEEHEERWMVD TGIEVPEQGEFDLNEIMDSEYVFA |
| SEQ ID No.10 | lacUV5 promoter | TCACTCATTAGGCACCCCAGGCTTTACACTTTATGCTTCCGGCTCGTATAATG TGTGGAA |
| SEQ ID No.11 | asd gene and its promoter and regulatory region | TTGCAGGAAAAAACGCTATGAAAAATGTTGGTTTATCGGCTGGCGCGGA ATGGTCGGCTCTGTTCTCATGCAACGCATGGTAGAGGAGCGCGATTTCGACG CTATTCGCCCTGTTTCTTTTCTACCTCCCAGTTTGGACAGGCGGCGCCCACC TTCGGCGACACCTCCACCGGCACGCTACAGGACGCTTTTGATCTGGATGCGC TAAAAGCGCTCGATATCATCGTGACCTGCCAGGGCGGCGATTATACCAACGA AATTTATCCAAAGCTGCGCGAAAGCGGATGGCAGGGTTACTGGATTGATGC GGCTTCTACGCTGCGCATGAAAGATGATGCCATTATTATTCTCGACCCGGTC AACCAGGACGTGATTACCGACGGCCTGAACAATGGCGTGAAGACCTTTGTG GGCGGTAACTGTACCGTTAGCCTGATGTTGATGTCGCTGGGCGGTCTCTTTG CCCATAATCTCGTTGACTGGGTATCCGTCGCGACCTATCAGGCCGCCTCCGG CGGCGGCGCGCGCCATATGCGCGAGCTGTTAACCCAGATGGGTCAGTTGTAT GGCCATGTCGCCGATGAACTGGCGACGCCGTCTTCCGCAATTCTTGATATTG AACGCAAAGTTACGGCATTGACCCGCAGCGGCGAGCTGCCGGTTGATAACTT TGGCGTACCGCTGGCGGGAAGCCTGATCCCCTGGATCGACAAACAGCTCGAT AACGGCCAGAGCCGCGAAGAGTGGAAAGGCCAGGCGGAAACCAACAAGAT TCTCAATACTGCCTCTGTGATTCCGGTTGATGGTTTGTGTGTGCGCGTCGGCG CGCTGCGCTGTCACAGCCAGGCGTTCACCATCAAGCTGAAAAAAGAGGTAT CCATTCCGACGGTGGAAGAACTGCTGGCGGCACATAATCCGTGGGCGAAAG TGGTGCCGAACGATCGTGATATCACTATGCGCGAATTAACCCCGGCGGCGGT GACCGGCACGTTGACTACGCCGGTTGGTCGTCTGCGTAAGCTGAACATGGGG CCAGAGTTCTTGTCGGCGTTTACCGTAGGCGACCAGTTGTTATGGGGCGCCG CCGAGCCGCTGCGTCGAATGCTGCGCCAGTTGGCGTAG |
| SEQ ID No.12 | protein sequence of asd | LQEKNAMKNVGFIGWRGMVGSVLMQRMVEERDFDAIRPVFFSTSQFGQAAPTF GDTSTGTLQDAFDLDALKALDIIVTCQGGDYTNEIYPKLRESGWQGYWIDAAST LRMKDDAIIILDPVNQDVITDGLNNGVKTFVGGNCTVSLMLMSLGGLFAHNLV DWVSVATYQAASGGGARHMRELLTQMGQLYGHVADELATPSSAILDIERKVT ALTRSGELPVDNFGVPLAGSLIPWIDKQLDNGQSREEWKGQAETNKILNTASVIP VDGLCVRVGALRCHSQAFTIKLKKEVSIPTVEELLAAHNPWAKVVPNDRDITMR ELTPAAVTGTLTTPVGRLRKLNMGPEFLSAFTVGDQLLWGAAEPLRRMLRQLA |
| SEQ ID No.13 | coding region of *Listeria monocytogenes* hemolysin O gene | ATGAAAAAAATCATGCTGGTTTTCATCACCCTCATACTGGTAAGCCTGCCGA TCGCGCAGCAGACCGAAGCGAAAGACGCGTCTGCGTTCAACAAAGAAAACT CTATCTCTTCTATGGCGCCGCCGGCGTCTCCGCCGGCGTCTCCGAAAACCCC GATCGAAAAAAAACACGCGGACGAAATCGACAAATACATCCAGGGCCTGGA CTACAACAAAAACAACGTTCTGGTTTACCACGGCGACGCGGTTACCAACGTT CCGCCGCGTAAAGGCTACAAAGACGGCAACGAATACATCGTTGTTGAAAAA AAAAAAAAATCTATCAACCAGAACAACGCGGACATCCAGGTTGTTAACGCG ATCTCTTCTCTGACCTACCCGGGCGCGCTGGTTAAAGCGAACTCTGAACTGG TTGAAAACCAGCCGGACGTTCTGCCGGTTAAACGTGACTCTCTGACCCTGTC TATCGACCTGCCGGGCATGACCAACCAGGACAACAAAATCGTTGTTAAAAA CGCGACCAAATCTAACGTTAACAACGCGGTTAACACCCTGGTTGAACGTTGG AACGAAAAATACGCGCAGGCGTACCCGAACGTTTCTGCGAAAATCGACTAC GACGACGAAATGGCGTACTCTGAATCTCAGCTGATCGCGAAATTCGGCACCG CGTTCAAAGCGGTTAACAACTCTCTGAACGTTAACTTCGGCGCGATCTCTGA AGGCAAAATGCAGGAAGAAGTTATCTCTTTCAAACAGATCTACTACAACGTT AACGTTAACGAACCGACCCGTCCGTCTCGTTTCTTCGGCAAAGCGGTTACCA AAGAACAGCTTCAGGCGCTGGGCGTTAACGCGGAAAACCCGCCGGCGTACA TCTCTTCTGTTGCGTACGGCCGTCAGGTTTACCTGAAACTGTCTACCAACTCT CACTCTACCAAAGTTAAAGCGGCTTTCGACGCGGCGGTAAGCGGCAAATCTG TTTCTGGCGACGTTGAACTGACCAACATCATCAAAAACTCTTCTTTCAAAGC GGTTATCTACGGCGGCTCTGCGAAAGACGAAGTTCAGATCATCGACGGCAA CCTGGGCGACCTGCGTGACATCCTGAAAAAAGGCGCGACCTTCAACCGTGA AACCCCGGGCGTTCCGATCGCGTACACCACCAACTTCCTGAAAGACAACGA ACTGGCGGTTATCAAAAACAACTCTGAATACATCGAAACCACCTCTAAAGCG TACACCGACGGCAAAATCAACATCGACCACTCTGGCGGCTACGTTGCGCAGT TCAACATCTCTTGGGACGAAGTTAACTACGACCCGGAAGGCAACGAAATCG TTCAGCACAAAAACTGGTCTGAAAACAACAAATCTAAACTGGCGCACTTCAC CTCTTCTATCTACCTGCCGGGCAACGCGCGTAACATCAACGTTTACGCGAAA GAATGCACCGGCCTGGCGTGGGAATGGTGGCGTACCGTTATCGACGACCGT AACCTGCCGCTGGTTAAAAACCGTAACATCTCTATCTGGGGCACCACCCTGT ACCCGAAATACTCTAACAAAGTTGACAACCCGATCGAATAATGA |
| SEQ ID No.14 | protein sequence of LLO gene | MKKIMLVFITLILVSLPIAQQTEAKDASAFNKENSISSMAPPASPPASPKTPIEKKH ADEIDKYIQGLDYNKNNVLVYHGDAVTNVPPRKGYKDGNEYIVVEKKKKSINQ NNADIQVVNAISSLTYPGALVKANSELVENQPDVLPVKRDSLTLSIDLPGMTNQ DNKIVVKNATKSNVNNAVNTLVERWNEKYAQAYPNVSAKIDYDDEMAYSESQ LIAKFGTAFKAVNNSLNVNFGAISEGKMQEEVISFKQIYYNVNVNEPTRPSRFFG KAVTKEQLQALGVNAENPPAYISSVAYGRQVYLKLSTNSHSTKVKAAFDAAVS GKSVSGDVELTNIIKNSSFKAVIYGGSAKDEVQIIDGNLGDLRDILKKGATFNRE TPGVPIAYTTNFLKDNELAVIKNNSEYIETTSKAYTDGKINIDHSGGYVAQFNIS WDEVNYDPEGNEIVQHKNWSENNKSKLAHFTSSIYLPGNARNINVYAKECTGL AWEWWRTVIDDRNLPLVKNRNISIWGTTLYPKYSNKVDNPIE |
| SEQ ID No.15 | derived from plasmid pEU001, containing *Salmonella* asd gene and its regulatory region, as well as LLO gene and RBS site | AGTAAGAAGGAGATATACATATGAAAAAAATCATGCTGGTTTTCATCACCCT CATACTGGTAAGCCTGCCGATCGCGCAGCAGACCGAAGCGAAAGACGCGTC TGCGTTCAACAAAGAAAACTCTATCTCTTCTATGGCGCCGCCGGCGTCTCCG CCGGCGTCTCCGAAAACCCCGATCGAAAAAAAACACGCGGACGAAATCGAC AAATACATCCAGGGCCTGGACTACAACAAAAACAACGTTCTGGTTTACCACG GCGACGCGGTTACCAACGTTCCGCCGCGTAAAGGCTACAAAGACGGCAACG AATACATCGTTGTTGAAAAAAAAAAAAAATCTATCAACCAGAACAACGCGG ACATCCAGGTTGTTAACGCGATCTCTTCTCTGACCTACCCGGGCGCGCTGGTT AAAGCGAACTCTGAACTGGTTGAAAACCAGCCGGACGTTCTGCCGGTTAAA CGTGACTCTCTGACCCTGTCTATCGACCTGCCGGGCATGACCAACCAGGACA ACAAAATCGTTGTTAAAAACGCGACCAAATCTAACGTTAACAACGCGGTTA ACACCCTGGTTGAACGTTGGAACGAAAAATACGCGCAGGCGTACCCGAACG TTTCTGCGAAAATCGACTACGACGACGAAATGGCGTACTCTGAATCTCAGCT GATCGCGAAATTCGGCACCGCGTTCAAAGCGGTTAACAACTCTCTGAACGTT AACTTCGGCGCGATCTCTGAAGGCAAAATGCAGGAAGAAGTTATCTCTTTCA AACAGATCTACTACAACGTTAACGTTAACGAACCGACCCGTCCGTCTCGTTT CTTCGGCAAAGCGGTTACCAAAGAACAGCTTCAGGCGCTGGGCGTTAACGC GGAAAACCCGCCGGCGTACATCTCTTCTGTTGCGTACGGCCGTCAGGTTTAC CTGAAACTGTCTACCAACTCTCACTCTACCAAAGTTAAAGCGGCTTTCGACG CGGCGGTAAGCGGCAAATCTGTTTCTGGCGACGTTGAACTGACCAACATCAT CAAAAACTCTTCTTTCAAAGCGGTTATCTACGGCGGCTCTGCGAAAGACGAA GTTCAGATCATCGACGGCAACCTGGGCGACCTGCGTGACATCCTGAAAAAA GGCGCGACCTTCAACCGTGAAACCCCGGGCGTTCCGATCGCGTACACCACCA ACTTCCTGAAAGACAACGAACTGGCGGTTATCAAAAACAACTCTGAATACAT CGAAACCACCTCTAAAGCGTACACCGACGGCAAAATCAACATCGACCACTC TGGCGGCTACGTTGCGCAGTTCAACATCTCTTGGGACGAAGTTAACTACGAC CCGGAAGGCAACGAAATCGTTCAGCACAAAAACTGGTCTGAAAACAACAAA TCTAAACTGGCGCACTTCACCTCTTCTATCTACCTGCCGGGCAACGCGCGTA ACATCAACGTTTACGCGAAAGAATGCACCGGCCTGGCGTGGGAATGGTGGC GTACCGTTATCGACGACCGTAACCTGCCGCTGGTTAAAAACCGTAACATCTC TATCTGGGGCACCACCCTGTACCCGAAATACTCTAACAAAGTTGACAACCCG ATCGAATAATGACTGCAGACTGATGGCTTCGCTATCATTGATTAATTTCACTT GCGACTTTGGCTGCTTTTTGTATGGTGAAGGATGCGCCACAGGATACTGGCG CGCATACACAGCACATCTCTTTGCAGGAAAAAAACGCTATGAAAAATGTTG GTTTTATCGGCTGGCGCGGAATGGTCGGCTCTGTTCTCATGCAACGCATGGT AGAGGAGCGCGATTTCGACGCTATTCGCCCTGTTTTCTTTTCTACCTCCCAGT TTGGACAGGCGGCGCCCACCTTCGGCGACACCTCCACCGGCACGCTACAGG ACGCTTTTGATCTGGATGCGCTAAAAGCGCTCGATATCATCGTGACCTGCCA GGGCGGCGATTATACCAACGAAATTTATCCAAAGCTGCGCGAAAGCGGATG GCAGGGTTACTGGATTGATGCGGCTTCTACGCTGCGCATGAAAGATGATGCC ATTATTATTCTCGACCCGGTCAACCAGGACGTGATTACCGACGGCCTGAACA ATGGCGTGAAGACCTTTGTGGGCGGTAACTGTACCGTTAGCCTGATGTTGAT GTCGCTGGGCGGTCTCTTTGCCCATAATCTCGTTGACTGGGTATCCGTCGCGA CCTATCAGGCCGCCTCCGGCGGCGGCGCGCGCCATATGCGCGAGCTGTTAAC CCAGATGGGTCAGTTGTATGGCCATGTCGCCGATGAACTGGCGACGCCGTCT TCCGCAATTCTTGATATTGAACGCAAAGTTACGGCATTGACCCGCAGCGGCG AGCTGCCGGTTGATAACTTTGGCGTACCGCTGGCGGGAAGCCTGATCCCCTG GATCGACAAACAGCTCGATAACGGCCAGAGCCGCGAAGAGTGGAAAGGCCA GGCGGAAACCAACAAGATTCTCAATACTGCCTCTGTGATTCCGGTTGATGGT TTGTGTGTGCGCGTCGGCGCGCTGCGCTGTCACAGCCAGGCGTTCACCATCA AGCTGAAAAAAGAGGTATCCATTCCGACGGTGGAAGAACTGCTGGCGGCAC ATAATCCGTGGGCGAAAGTGGTGCCGAACGATCGTGATATCACTATGCGCG AATTAACCCCGGCGGCGGTGACCGGCACGTTGACTACGCCGGTTGGTCGTCT GCGTAAGCTGAACATGGGGCCAGAGTTCTTGTCGGCGTTTACCGTAGGCGAC CAGTTGTTATGGGGCGCCGCCGAGCCGCTGCGTCGAATGCTGCGCCAGTTGG CGTAGCTCGAG |
| SEQ ID No.16 | derived from plasmid pEU002, containing the first IRES sequence under the control of T7 promoter | GCGGCCGCAGATCTCGATCCCGCGAAATTAATACGACTCACTATAGACGCGT CGAGCATGCATCTAGGGCGGCCAATTCCGCCCCTCTCCCTCCCCCCCCCCTA ACGTTACTGGCCGAAGCCGCTTGGAATAAGGCCGGTGTGCGTTTGTCTATAT GTGATTTTCCACCATATTGCCGTCTTTTGGCAATGTGAGGGCCCGGAAACCT GGCCCTGTCTTCTTGACGAGCATTCCTAGGGGTCTTTCCCCTCTCGCCAAAGG AATGCAAGGTCTGTTGAATGTCGTGAAGGAAGCAGTTCCTCTGGAAGCTTCT TGAAGACAAACAACGTCTGTAGCGACCCTTTGCAGGCAGCGGAACCCCCCA CCTGGCGACAGGTGCCTCTGCGGCCAAAAGCCACGTGTATAAGATACACCTG CAAAGGCGGCACAACCCCAGTGCCACGTTGTGAGTTGGATAGTTGTGGAAA GAGTCAAATGGCTCTCCTCAAGCGTATTCAACAAGGGGCTGAAGGATGCCC AGAAGGTACCCCATTGTATGGGATCTGATCTGGGGCCTCGGTGCACATGCTT TACATGTGTTTAGTCGAGGTTAAAAAACGTCTAGGCCCCCCGAACCACGGG GACGTGGTTTTCCTTTGAAAAAGCCACCATGG |
| SEQ ID No.17 | derived from plasmid pEGFP-N1, containing eukaryotic EGFP coding sequence | CCATGGTGAGCAAGGGCGAGGAGCTGTTCACCGGGGTGGTGCCCATCCTGG TCGAGCTGGACGGCGACGTAAACGGCCACAAGTTCAGCGTGTCCGGCGAGG GCGAGGGCGATGCCACCTACGGCAAGCTGACCCTGAAGTTCATCTGCACCAC CGGCAAGCTGCCCGTGCCCTGGCCCACCCTCGTGACCACCCTGACCTACGGC GTGCAGTGCTTCAGCCGCTACCCCGACCACATGAAGCAGCACGACTTCTTCA AGTCCGCCATGCCCGAAGGCTACGTCCAGGAGCGCACCATCTTCTTCAAGGA CGACGGCAACTACAAGACCCGCGCCGAGGTGAAGTTCGAGGGCGACACCCT GGTGAACCGCATCGAGCTGAAGGGCATCGACTTCAAGGAGGACGGCAACAT CCTGGGGCACAAGCTGGAGTACAACTACAACAGCCACAACGTCTATATCAT GGCCGACAAGCAGAAGAACGGCATCAAGGTGAACTTCAAGATCCGCCACAA CATCGAGGACGGCAGCGTGCAGCTCGCCGACCACTACCAGCAGAACACCCC CATCGGCGACGGCCCCGTGCTGCTGCCCGACAACCACTACCTGAGCACCCAG TCCGCCCTGAGCAAAGACCCCAACGAGAAGCGCGATCACATGGTCCTGCTG GAGTTCGTGACCGCCGCCGGGATCACTCTCGGCATGGACGAGCTGTACAAGT AAAGCGGCCGCGACTCTAGA |
| SEQ ID No.18 | derived from plasmid pEU001, containing 3'-UTR region, 50 bp polyA sequence, T7 terminator and second T7 promoter | TCTAGAGCTCGCTTTCTTGCTGTCCAATTTCTATTAAAGGTTCCTTTGTTCCCT AAGTCCAACTACTAAACTGGGGGATATTATGAAGGGCCTTGAGCATCTGGAT TCTGCCTAATAAAAAACATTTATTTTCATTGCAAAAAAAAAAAAAAAAAAAA AAAAAAAAAAAAAAAAAAAAAAAAAAAAAAAAAAAAAACTAGCATAACCCCTTGG GGCCTCTAAACGGGTCTTGAGGGGTTTTTTAGATCTCGATCCCGCGAAATTA ATACGACTCACTATAGACTAGT |
| SEQ ID No.19 | second IRES sequence | TCTAGAACGCGTCGAGCATGCATCTAGGGCGGCCAATTCCGCCCCTCTCCCT CCCCCCCCCCTAACGTTACTGGCCGAAGCCGCTTGGAATAAGGCCGGTGTGC GTTTGTCTATATGTGATTTTCCACCATATTGCCGTCTTTTGGCAATGTGAGGG CCCGGAAACCTGGCCCTGTCTTCTTGACGAGCATTCCTAGGGGTCTTTCCCCT CTCGCCAAAGGAATGCAAGGTCTGTTGAATGTCGTGAAGGAAGCAGTTCCTC TGGAAGCTTCTTGAAGACAAACAACGTCTGTAGCGACCCTTTGCAGGCAGCG GAACCCCCCACCTGGCGACAGGTGCCTCTGCGGCCAAAAGCCACGTGTATA AGATACACCTGCAAAGGCGGCACAACCCCAGTGCCACGTTGTGAGTTGGAT AGTTGTGGAAAGAGTCAAATGGCTCTCCTCAAGCGTATTCAACAAGGGGCTG AAGGATGCCCAGAAGGTACCCCATTGTATGGGATCTGATCTGGGGCCTCGGT GCACATGCTTTACATGTGTTTAGTCGAGGTTAAAAAAACGTCTAGGCCCCCC GAACCACGGGGACGTGGTTTTCCTTTGAAAAAGCCGAATTC |
| SEQ ID No.20 | sequence of T7 RNA polymerase from plasmid pEU010 | GAATTCGCCACCATGAACACCATCAACATTGCCAAAAACGATTTTAGCGATA TTGAACTGGCAGCGATTCCGTTTAATACCCTGGCAGATCATTATGGTGAACG TCTGGCACGTGAACAGCTGGCACTGGAACATGAAAGCTATGAAATGGGTGA AGCACGTTTTCGCAAAATGTTTGAACGTCAGCTGAAAGCCGGTGAAGTTGCA GATAATGCAGCAGCAAAACCGCTGATTACCACACTGCTGCCGAAAATGATT GCACGTATTAACGATTGGTTCGAAGAGGTTAAAGCCAAACGTGGTAAACGT CCGACCGCATTTCAGTTTCTGCAAGAAATTAAACCGGAAGCCGTTGCCTATA TTACCATTAAAACCACACTGGCATGTCTGACCAGCGCAGATAATACCACCGT TCAGGCAGTTGCAAGCGCAATTGGTCGTGCAATTGAAGATGAAGCCCGTTTT GGTCGTATTCGTGATCTGGAAGCAAAACACTTTAAGAAAAACGTGGAAGAA CAGCTGAATAAACGTGTTGGTCACGTTTATAAGAAAGCCTTTATGCAGGTTG TTGAAGCAGATATGCTGAGCAAAGGTCTGTTAGGTGGTGAAGCATGGTCAA GCTGGCATAAAGAAGATAGCATTCACGTTGGTGTTCGCTGTATTGAAATGCT GATTGAAAGCACCGGTATGGTTAGCCTGCATCGTCAGAATGCCGGTGTTGTT GGTCAGGATAGCGAAACCATCGAACTGGCACCGGAATATGCCGAAGCAATT GCAACCCGTGCCGGTGCACTGGCAGGTATTAGCCCGATGTTTCAGCCGTGTG TTGTTCCGCCTAAACCGTGGACCGGCATTACCGGTGGTGGTTATTGGGCAAA TGGTCGTCGTCCGCTGGCGCTGGTTCGTACCCATAGCAAAAAGGCACTGATG CGTTATGAAGATGTTTATATGCCGGAAGTGTACAAAGCCATTAACATTGCAC AGAATACCGCCTGGAAAATCAATAAAAAGGTTCTGGCCGTTGCCAACGTTAT CACCAAATGGAAACATTGTCCGGTTGAAGATATTCCGGCAATTGAACGTGAA GAACTGCCGATGAAGCCGGAAGATATTGATATGAATCCGGAAGCACTGACC GCATGGAAACGTGCAGCAGCCGCAGTTTATCGTAAAGATAAAGCACGTAAA AGCCGTCGTATTAGCCTGGAATTTATGCTGGAACAGGCAAACAAATTTGCCA ACCATAAAGCAATCTGGTTCCCGTATAATATGGATTGGCGTGGTCGTGTTTA TGCCGTTAGCATGTTTAATCCGCAGGGTAATGATATGACCAAAGGCCTGCTG ACCCTGGCAAAAGGTAAACCGATTGGTAAAGAAGGTTACTATTGGCTGAAA ATTCATGGTGCAAATTGTGCCGGTGTGGATAAAGTTCCGTTTCCGGAACGCA TTAAATTCATCGAAGAAAACCACGAAAACATTATGGCCTGTGCAAAAAGTC CGCTGGAAAATACCTGGTGGGCAGAACAGGATAGTCCGTTTTGTTTTCTGGC CTTTTGTTTTGAATATGCGGGTGTTCAGCATCATGGTCTGAGCTATAATTGTA GCCTGCCGCTGGCATTTGATGGTAGCTGTAGCGGTATTCAGCATTTTAGCGC AATGCTGCGTGATGAAGTTGGTGGTCGTGCCGTTAATCTGCTGCCGTCAGAA ACCGTGCAGGATATTTATGGTATTGTTGCCAAGAAAGTGAACGAAATTCTGC AAGCAGATGCAATTAATGGCACCGATAATGAAGTTGTTACCGTGACCGATG AAAACACCGGTGAAATTAGCGAAAAAGTTAAACTGGGCACCAAAGCGCTGG CAGGTCAGTGGCTGGCCTATGGTGTTACCCGTAGCGTTACCAAACGTAGCGT GATGACACTGGCGTATGGTAGCAAAGAATTTGGTTTTCGTCAGCAGGTTCTG GAAGATACAATTCAGCCTGCAATTGATAGCGGTAAAGGTCTGATGTTTACCC AGCCGAATCAGGCAGCAGGTTATATGGCAAAACTGATTTGGGAAAGCGTTA GCGTGACCGTTGTTGCAGCAGTTGAAGCAATGAACTGGCTGAAATCAGCAG CCAAACTGCTGGCAGCAGAAGTTAAAGATAAGAAAACGGGTGAAATCCTGC GTAAACGTTGTGCCGTTCATTGGGTTACACCGGATGGTTTTCCGGTTTGGCA AGAATATAAGAAACCGATTCAGACACGCCTGAACCTGATGTTTCTGGGTCAG TTTCGTCTGCAACCGACCATTAATACCAATAAAGATTCCGAAATCGACGCCC ACAAACAAGAAAGCGGTATTGCACCGAATTTTGTTCATAGCCAGGATGGTA GCCATCTGCGTAAAACCGTTGTTTGGGCACATGAAAAATATGGCATTGAATC CTTTGCGCTGATCCATGATAGCTTTGGCACCATTCCGGCAGATGCAGCAAAC CTGTTTAAAGCAGTTCGTGAAACAATGGTGGATACCTATGAAAGCTGTGATG TTCTGGCGGATTTTTATGATCAGTTTGCAGATCAGCTGCATGAAAGCCAGCT GGATAAAATGCCTGCACTGCCTGCCAAAGGTAATCTGAATCTGCGTGATATT CTGGAAAGCGATTTTGCCTTTGCCTAAGAGCTC |
| SEQ ID No.21 | complete sequence of pEU010 | GCGGCCGCAGATCTCGATCCCGCGAAATTAATACGACTCACTATAGACGCGT CGAGCATGCATCTAGGGCGGCCAATTCCGCCCCTCTCCCTCCCCCCCCCCTA ACGTTACTGGCCGAAGCCGCTTGGAATAAGGCCGGTGTGCGTTTGTCTATAT GTGATTTTCCACCATATTGCCGTCTTTTGGCAATGTGAGGGCCCGGAAACCT GGCCCTGTCTTCTTGACGAGCATTCCTAGGGGTCTTTCCCCTCTCGCCAAAGG AATGCAAGGTCTGTTGAATGTCGTGAAGGAAGCAGTTCCTCTGGAAGCTTCT TGAAGACAAACAACGTCTGTAGCGACCCTTTGCAGGCAGCGGAACCCCCCA CCTGGCGACAGGTGCCTCTGCGGCCAAAAGCCACGTGTATAAGATACACCTG CAAAGGCGGCACAACCCCAGTGCCACGTTGTGAGTTGGATAGTTGTGGAAA GAGTCAAATGGCTCTCCTCAAGCGTATTCAACAAGGGGCTGAAGGATGCCC AGAAGGTACCCCATTGTATGGGATCTGATCTGGGGCCTCGGTGCACATGCTT TACATGTGTTTAGTCGAGGTTAAAAAAACGTCTAGGCCCCCCGAACCACGGG GACGTGGTTTTCCTTTGAAAAAGCCACCATGGTGAGCAAGGGCGAGGAGCT GTTCACCGGGGTGGTGCCCATCCTGGTCGAGCTGGACGGCGACGTAAACGG CCACAAGTTCAGCGTGTCCGGCGAGGGCGAGGGCGATGCCACCTACGGCAA GCTGACCCTGAAGTTCATCTGCACCACCGGCAAGCTGCCCGTGCCCTGGCCC ACCCTCGTGACCACCCTGACCTACGGCGTGCAGTGCTTCAGCCGCTACCCCG ACCACATGAAGCAGCACGACTTCTTCAAGTCCGCCATGCCCGAAGGCTACGT CCAGGAGCGCACCATCTTCTTCAAGGACGACGGCAACTACAAGACCCGCGC CGAGGTGAAGTTCGAGGGCGACACCCTGGTGAACCGCATCGAGCTGAAGGG CATCGACTTCAAGGAGGACGGCAACATCCTGGGGCACAAGCTGGAGTACAA CTACAACAGCCACAACGTCTATATCATGGCCGACAAGCAGAAGAACGGCAT CAAGGTGAACTTCAAGATCCGCCACAACATCGAGGACGGCAGCGTGCAGCT CGCCGACCACTACCAGCAGAACACCCCCATCGGCGACGGCCCCGTGCTGCTG CCCGACAACCACTACCTGAGCACCCAGTCCGCCCTGAGCAAAGACCCCAAC GAGAAGCGCGATCACATGGTCCTGCTGGAGTTCGTGACCGCCGCCGGGATC ACTCTCGGCATGGACGAGCTGTACAAGTAAAGCGGCCGCGACTCTAGAACG CGTCGAGCATGCATCTAGGGCGGCCAATTCCGCCCCTCTCCCTCCCCCCCCC CTAACGTTACTGGCCGAAGCCGCTTGGAATAAGGCCGGTGTGCGTTTGTCTA TATGTGATTTTCCACCATATTGCCGTCTTTTGGCAATGTGAGGGCCCGGAAA CCTGGCCCTGTCTTCTTGACGAGCATTCCTAGGGGTCTTTCCCCTCTCGCCAA AGGAATGCAAGGTCTGTTGAATGTCGTGAAGGAAGCAGTTCCTCTGGAAGCT TCTTGAAGACAAACAACGTCTGTAGCGACCCTTTGCAGGCAGCGGAACCCCC CACCTGGCGACAGGTGCCTCTGCGGCCAAAAGCCACGTGTATAAGATACAC CTGCAAAGGCGGCACAACCCCAGTGCCACGTTGTGAGTTGGATAGTTGTGGA AAGAGTCAAATGGCTCTCCTCAAGCGTATTCAACAAGGGGCTGAAGGATGC CCAGAAGGTACCCCATTGTATGGGATCTGATCTGGGGCCTCGGTGCACATGC TTTACATGTGTTTAGTCGAGGTTAAAAAAACGTCTAGGCCCCCCGAACCACG GGGACGTGGTTTTCCTTTGAAAAAGCCGAATTCGCCACCATGAACACCATCA ACATTGCCAAAAACGATTTTAGCGATATTGAACTGGCAGCGATTCCGTTTAA TACCCTGGCAGATCATTATGGTGAACGTCTGGCACGTGAACAGCTGGCACTG GAACATGAAAGCTATGAAATGGGTGAAGCACGTTTTCGCAAAATGTTTGAA CGTCAGCTGAAAGCCGGTGAAGTTGCAGATAATGCAGCAGCAAAACCGCTG ATTACCACACTGCTGCCGAAAATGATTGCACGTATTAACGATTGGTTCGAAG AGGTTAAAGCCAAACGTGGTAAACGTCCGACCGCATTTCAGTTTCTGCAAGA AATTAAACCGGAAGCCGTTGCCTATATTACCATTAAAACCACACTGGCATGT CTGACCAGCGCAGATAATACCACCGTTCAGGCAGTTGCAAGCGCAATTGGTC GTGCAATTGAAGATGAAGCCCGTTTTGGTCGTATTCGTGATCTGGAAGCAAA ACACTTTAAGAAAAACGTGGAAGAACAGCTGAATAAACGTGTTGGTCACGT TTATAAGAAAGCCTTTATGCAGGTTGTTGAAGCAGATATGCTGAGCAAAGGT CTGTTAGGTGGTGAAGCATGGTCAAGCTGGCATAAAGAAGATAGCATTCAC GTTGGTGTTCGCTGTATTGAAATGCTGATTGAAAGCACCGGTATGGTTAGCC TGCATCGTCAGAATGCCGGTGTTGTTGGTCAGGATAGCGAAACCATCGAACT GGCACCGGAATATGCCGAAGCAATTGCAACCCGTGCCGGTGCACTGGCAGG TATTAGCCCGATGTTTCAGCCGTGTGTTGTTCCGCCTAAACCGTGGACCGGC ATTACCGGTGGTGGTTATTGGGCAAATGGTCGTCGTCCGCTGGCGCTGGTTC GTACCCATAGCAAAAAGGCACTGATGCGTTATGAAGATGTTTATATGCCGGA AGTGTACAAAGCCATTAACATTGCACAGAATACCGCCTGGAAAATCAATAA AAAGGTTCTGGCCGTTGCCAACGTTATCACCAAATGGAAACATTGTCCGGTT GAAGATATTCCGGCAATTGAACGTGAAGAACTGCCGATGAAGCCGGAAGAT ATTGATATGAATCCGGAAGCACTGACCGCATGGAAACGTGCAGCAGCCGCA GTTTATCGTAAAGATAAAGCACGTAAAAGCCGTCGTATTAGCCTGGAATTTA TGCTGGAACAGGCAAACAAATTTGCCAACCATAAAGCAATCTGGTTCCCGTA TAATATGGATTGGCGTGGTCGTGTTTATGCCGTTAGCATGTTTAATCCGCAG GGTAATGATATGACCAAAGGCCTGCTGACCCTGGCAAAAGGTAAACCGATT GGTAAAGAAGGTTACTATTGGCTGAAAATTCATGGTGCAAATTGTGCCGGTG TGGATAAAGTTCCGTTTCCGGAACGCATTAAATTCATCGAAGAAAACCACGA AAACATTATGGCCTGTGCAAAAAGTCCGCTGGAAAATACCTGGTGGGCAGA ACAGGATAGTCCGTTTTGTTTTCTGGCCTTTTGTTTTGAATATGCGGGTGTTC AGCATCATGGTCTGAGCTATAATTGTAGCCTGCCGCTGGCATTTGATGGTAG CTGTAGCGGTATTCAGCATTTAGCGCAATGCTGCGTGATGAAGTTGGTGGT CGTGCCGTTAATCTGCTGCCGTCAGAAACCGTGCAGGATATTTATGGTATTG TTGCCAAGAAAGTGAACGAAATTCTGCAAGCAGATGCAATTAATGGCACCG ATAATGAAGTTGTTACCGTGACCGATGAAAACACCGGTGAAATTAGCGAAA AAGTTAAACTGGGCACCAAAGCGCTGGCAGGTCAGTGGCTGGCCTATGGTG TTACCCGTAGCGTTACCAAACGTAGCGTGATGACACTGGCGTATGGTAGCAA AGAATTTGGTTTCGTCAGCAGGTTCTGGAAGATACAATTCAGCCTGCAATT GATAGCGGTAAAGGTCTGATGTTTACCCAGCCGAATCAGGCAGCAGGTTATA TGGCAAAACTGATTTGGGAAAGCGTTAGCGTGACCGTTGTTGCAGCAGTTGA AGCAATGAACTGGCTGAAATCAGCAGCCAAACTGCTGGCAGCAGAAGTTAA AGATAAGAAAACGGGTGAAATCCTGCGTAAACGTTGTGCCGTTCATTGGGTT ACACCGGATGGTTTTCCGGTTTGGCAAGAATATAAGAAACCGATTCAGACAC GCCTGAACCTGATGTTTCTGGGTCAGTTTCGTCTGCAACCGACCATTAATACC AATAAAGATTCCGAAATCGACGCCCACAAACAAGAAAGCGGTATTGCACCG AATTTTGTTCATAGCCAGGATGGTAGCCATCTGCGTAAAACCGTTGTTTGGG CACATGAAAAATATGGCATTGAATCCTTTGCGCTGATCCATGATAGCTTTGG CACCATTCCGGCAGATGCAGCAAACCTGTTTAAAGCAGTTCGTGAAACAATG GTGGATACCTATGAAAGCTGTGATGTTCTGGCGGATTTTTATGATCAGTTTGC AGATCAGCTGCATGAAAGCCAGCTGGATAAAATGCCTGCACTGCCTGCCAA AGGTAATCTGAATCTGCGTGATATTCTGGAAAGCGATTTTGCCTTTGCCTAA GAGCTCGCTTTCTTGCTGTCCAATTTCTATTAAAGGTTCCTTTGTTCCCTAAG TCCAACTACTAAACTGGGGGATATTATGAAGGGCCTTGAGCATCTGGATTCT GCCTAATAAAAAACATTTATTTTCATTGCAAAAAAAAAAAAAAAAAAAAAA AAAAAAAAAAAAAAAAAAAAAAAAAAAAAAAACTAGCATAACCCCTTGGGGC CTCTAAACGGGTCTTGAGGGGTTTTTTAGATCTCGATCCCGCGAAATTAATA CGACTCACTATAGACTAGTAAGAAGGAGATATACATATGAAAAAAATCATG CTGGTTTTCATCACCCTCATACTGGTAAGCCTGCCGATCGCGCAGCAGACCG AAGCGAAAGACGCGTCTGCGTTCAACAAAGAAAACTCTATCTCTTCTATGGC GCCGCCGGCGTCTCCGCCGGCGTCTCCGAAAACCCCGATCGAAAAAAAACA CGCGGACGAAATCGACAAATACATCCAGGGCCTGGACTACAACAAAAACAA CGTTCTGGTTTACCACGGCGACGCGGTTACCAACGTTCCGCCGCGTAAAGGC TACAAAGACGGCAACGAATACATCGTTGTTGAAAAAAAAAAAAAAATCTATC AACCAGAACAACGCGGACATCCAGGTTGTTAACGCGATCTCTTCTCTGACCT ACCCGGGCGCGCTGGTTAAAGCGAACTCTGAACTGGTTGAAAACCAGCCGG ACGTTCTGCCGGTTAAACGTGACTCTCTGACCCTGTCTATCGACCTGCCGGG CATGACCAACCAGGACAACAAAATCGTTGTTAAAAACGCGACCAAATCTAA CGTTAACAACGCGGTTAACACCCTGGTTGAACGTTGGAACGAAAAATACGC GCAGGCGTACCCGAACGTTTCTGCGAAAATCGACTACGACGACGAAATGGC GTACTCTGAATCTCAGCTGATCGCGAAATTCGGCACCGCGTTCAAAGCGGTT AACAACTCTCTGAACGTTAACTTCGGCGCGATCTCTGAAGGCAAAATGCAGG AAGAAGTTATCTCTTTCAAACAGATCTACTACAACGTTAACGTTAACGAACC GACCCGTCCGTCTCGTTTCTTCGGCAAAGCGGTTACCAAAGAACAGCTTCAG GCGCTGGGCGTTAACGCGGAAAACCCGCCGGCGTACATCTCTTCTGTTGCGT ACGGCCGTCAGGTTTACCTGAAACTGTCTACCAACTCTCACTCTACCAAAGT TAAAGCGGCTTTCGACGCGGCGGTAAGCGGCAAATCTGTTTCTGGCGACGTT GAACTGACCAACATCATCAAAAACTCTTCTTTCAAAGCGGTTATCTACGGCG GCTCTGCGAAAGACGAAGTTCAGATCATCGACGGCAACCTGGGCGACCTGC GTGACATCCTGAAAAAAGGCGCGACCTTCAACCGTGAAACCCCGGGCGTTC CGATCGCGTACACCACCAACTTCCTGAAAGACAACGAACTGGCGGTTATCAA AAACAACTCTGAATACATCGAAACCACCTCTAAAGCGTACACCGACGGCAA AATCAACATCGACCACTCTGGCGGCTACGTTGCGCAGTTCAACATCTCTTGG GACGAAGTTAACTACGACCCGGAAGGCAACGAAATCGTTCAGCACAAAAAC TGGTCTGAAAACAACAAATCTAAACTGGCGCACTTCACCTCTTCTATCTACC TGCCGGGCAACGCGCGTAACATCAACGTTTACGCGAAAGAATGCACCGGCC TGGCGTGGGAATGGTGGCGTACCGTTATCGACGACCGTAACCTGCCGCTGGT TAAAAACCGTAACATCTCTATCTGGGGCACCACCCTGTACCCGAAATACTCT AACAAAGTTGACAACCCGATCGAATAATGACTGCAGACTGATGGCTTCGCTA TCATTGATTAATTTCACTTGCGACTTTGGCTGCTTTTTGTATGGTGAAGGATG CGCCACAGGATACTGGCGCGCATACACAGCACATCTCTTTGCAGGAAAAAA ACGCTATGAAAAATGTTGGTTTTATCGGCTGGCGCGGAATGGTCGGCTCTGT TCTCATGCAACGCATGGTAGAGGAGCGCGATTTCGACGCTATTCGCCCTGTT TTCTTTTCTACCTCCCAGTTTGGACAGGCGGCGCCCACCTTCGGCGACACCTC CACCGGCACGCTACAGGACGCTTTTGATCTGGATGCGCTAAAAGCGCTCGAT ATCATCGTGACCTGCCAGGGCGGCGATTATACCAACGAAATTTATCCAAAGC TGCGCGAAAGCGGATGGCAGGGTTACTGGATTGATGCGGCTTCTACGCTGCG CATGAAAGATGATGCCATTATTATTCTCGACCCGGTCAACCAGGACGTGATT ACCGACGGCCTGAACAATGGCGTGAAGACCTTTGTGGGCGGTAACTGTACC GTTAGCCTGATGTTGATGTCGCTGGGCGGTCTCTTTGCCCATAATCTCGTTGA CTGGGTATCCGTCGCGACCTATCAGGCCGCCTCCGGCGGCGGCGCGCGCCAT ATGCGCGAGCTGTTAACCCAGATGGGTCAGTTGTATGGCCATGTCGCCGATG AACTGGCGACGCCGTCTTCCGCAATTCTTGATATTGAACGCAAAGTTACGGC ATTGACCCGCAGCGGCGAGCTGCCGGTTGATAACTTTGGCGTACCGCTGGCG GGAAGCCTGATCCCCTGGATCGACAAACAGCTCGATAACGGCCAGAGCCGC GAAGAGTGGAAAGGCCAGGCGGAAACCAACAAGATTCTCAATACTGCCTCT GTGATTCCGGTTGATGGTTTGTGTGTGCGCGTCGGCGCGCTGCGCTGTCACA GCCAGGCGTTCACCATCAAGCTGAAAAAAGAGGTATCCATTCCGACGGTGG AAGAACTGCTGGCGGCACATAATCCGTGGGCGAAAGTGGTGCCGAACGATC GTGATATCACTATGCGCGAATTAACCCCGGCGGCGGTGACCGGCACGTTGAC TACGCCGGTTGGTCGTCTGCGTAAGCTGAACATGGGGCCAGAGTTCTTGTCG GCGTTTACCGTAGGCGACCAGTTGTTATGGGGCGCCGCCGAGCCGCTGCGTC GAATGCTGCGCCAGTTGGCGTAGCTCGAGCATGTGAGCAAAAGGCCAGCAA AAGGCCAGGAACCGTAAAAAGGCCGCGTTGCTGGCGTTTTTCCATAGGCTCC GCCCCCCTGACGAGCATCACAAAAATCGACGCTCAAGTCAGAGGTGGCGAA ACCCGACAGGACTATAAAGATACCAGGCGTTTCCCCCTGGAAGCTCCCTCGT GCGCTCTCCTGTTCCGACCCTGCCGCTTACCGGATACCTGTCCGCCTTTCTCC CTTCGGGAAGCGTGGCGCTTTCTCATAGCTCACGCTGTAGGTATCTCAGTTC GGTGTAGGTCGTTCGCTCCAAGCTGGGCTGTGTGCACGAACCCCCCGTTCAG CCCGACCGCTGCGCCTTATCCGGTAACTATCGTCTTGAGTCCAACCCGGTAA GACACGACTTATCGCCACTGGCAGCAGCCACTGGTAACAGGATTAGCAGAG CGAGGTATGTAGGCGGTGCTACAGAGTTCTTGAAGTGGTGGCCTAACTACGG CTACACTAGAAGGACAGTATTTGGTATCTGCGCTCTGCTGAAGCCAGTTACC TTCGGAAAAAGAGTTGGTAGCTCTTGATCCGGCAAACAAACCACCGCTGGTA GCGGTGGTTTTTTTGTTTGCAAGCAGCAGATTACGCGCAGAAAAAAAGGATC TCAAGAAGATCCTTTGATCTTTTCTACGGGGTCTGACGCTCAGTGGAACGAA AACTCACGTTAAGGGATTTTGGTCATGAGATTATCAAAAAGGATCTTCACCT AGATCCTTTTAAATTAAAAATGAAGTTTTAAATCAATCTAAAGTATATATGA GTAA |
| SEQ ID No.22 | pIKDE-EGFP | TCAATATTGGCCATTAGCCATATTATTCATTGGTTATATAGCATAAATCAATA TTGGCTATTGGCCATTGCATACGTTGTATCTATATCATAATATGTACATTTAT ATTGGCTCATGTCCAATATGACCGCCATGTTGGCATTGATTATTGACTAGTTA TTAATAGTAATCAATTACGGGGTCATTAGTTCATAGCCCATATATGGAGTTC CGCGTTACATAACTTACGGTAAATGGCCCGCCTGGCTGACCGCCCAACGACC CCCGCCCATTGACGTCAATAATGACGTATGTTCCCATAGTAACGCCAATAGG GACTTTCCATTGACGTCAATGGGTGGAGTATTTACGGTAAACTGCCCACTTG GCAGTACATCAAGTGTATCATATGCCAAGTACGCCCCCTATTGACGTCAATG ACGGTAAATGGCCCGCCTGGCATTATGCCCAGTACATGACCTTATGGGACTT TCCTACTTGGCAGTACATCTACGTATTAGTCATCGCTATTACCATGGTGATGC GGTTTTGGCAGTACATCAATGGGCGTGGATAGCGGTTTGACTCACGGGGATT TCCAAGTCTCCACCCCATTGACGTCAATGGGAGTTTGTTTTGGCACCAAAAT CAACGGGACTTTCCAAAATGTCGTAACAACTCCGCCCCATTGACGCAAATGG GCGGTAGGCGTGTACGGTGGGAGGTCTATATAAGCAGAGCTCTCTGGCTAAC TAGAGAACCCACTGCTTACTGGCTTATCGAAATTAATACGACTCACTATAGG GAGACCCAAGCTGGCTAGAGCCCTCTCCCTCCCCCCCCCCTAACGTTACTG GCCGAAGCCGCTTGGAATAAGGCCGGTGTGCGTTTGTCTATATGTTATTTTCC ACCATATTGCCGTCTTTTGGCAATGTGAGGGCCCGGAAACCTGGCCCTGTCT TCTTGACGAGCATTCCTAGGGGTCTTTCCCCTCTCGCCAAAGGAATGCAAGG TCTGTTGAATGTCGTGAAGGAAGCAGTTCCTCTGGAAGCTTCTTGAAGACAA ACAACGTCTGTAGCGACCCTTTGCAGGCAGCGGAACCCCCCACCTGGCGACA GGTGCCTCTGCGGCCAAAAGCCACGTGTATAAGATACACCTGCAAAGGCGG CACAACCCCAGTGCCACGTTGTGAGTTGGATAGTTGTGGAAAGAGTCAAATG GCTCTCCTCAAGCGTATTCAACAAGGGGCTGAAGGATGCCCAGAAGGTACC CCATTGTATGGGATCTGATCTGGGGCCTCGGTGCACATGCTTTACATGTGTTT AGTCGAGGTTAAAAAAACGTCTAGGCCCCCCGAACCACGGGGACGTGGTTT TCCTTTGAAAAACACGATGATAATATGCTAGCATATGGTGAGCAAGGGCGA GGAGCTGTTCACCGGGGTGGTGCCCATCCTGGTCGAGCTGGACGGCGACGTA AACGGCCACAAGTTCAGCGTGTCCGGCGAGGGCGAGGGCGATGCCACCTAC GGCAAGCTGACCCTGAAGTTCATCTGCACCACCGGCAAGCTGCCCGTGCCCT GGCCCACCCTCGTGACCACCCTGACCTACGGCGTGCAGTGCTTCAGCCGCTA CCCCGACCACATGAAGCAGCACGACTTCTTCAAGTCCGCCATGCCCGAAGGC TACGTCCAGGAGCGCACCATCTTCTTCAAGGACGACGGCAACTACAAGACCC GCGCCGAGGTGAAGTTCGAGGGCGACACCCTGGTGAACCGCATCGAGCTGA AGGGCATCGACTTCAAGGAGGACGGCAACATCCTGGGGCACAAGCTGGAGT ACAACTACAACAGCCACAACGTCTATATCATGGCCGACAAGCAGAAGAACG GCATCAAGGTGAACTTCAAGATCCGCCACAACATCGAGGACGGCAGCGTGC AGCTCGCCGACCACTACCAGCAGAACACCCCCATCGGCGACGGCCCCGTGCT GCTGCCCGACAACCACTACCTGAGCACCCAGTCCGCCCTGAGCAAAGACCCC AACGAGAAGCGCGATCACATGGTCCTGCTGGAGTTCGTGACCGCCGCCGGG ATCACTCTCGGCATGGACGAGCTGTACAAGTAACTGCAGTCGAGTCTAGAGG GCCCGTTTAAACCCGCTGATCAGCCTCGACTGTGCCTTCTAGTTGCCAGCCAT CTGTTGTTTGCCCCTCCCCCGTGCCTTCCTTGACCCTGGAAGGTGCCACTCCC ACTGTCCTTTCCTAATAAAATGAGGAAATTGCATCGCATTGTCTGAGTAGGT GTCATTCTATTCTGGGGGGTGGGGTGGGGCAGGACAGCAAGGGGGAGGATT GGGAAGACAATAGCAGGCATGCTGGGGATGCGGTGGGCTCTATGGTAATAC GACTCACTATAGGGAGACCGCGGCCCGGGATCCGCCCCTCTCCCTCCCCCC CCCCTAACGTTACTGGCCGAAGCCGCTTGGAATAAGGCCGGTGTGCGTTTGT CTATATGTTATTTTCCACCATATTGCCGTCTTTTGGCAATGTGAGGGCCCGGA AACCTGGCCCTGTCTTCTTGACGAGCATTCCTAGGGGTCTTTCCCCTCTCGCC AAAGGAATGCAAGGTCTGTTGAATGTCGTGAAGGAAGCAGTTCCTCTGGAA GCTTCTTGAAGACAAACAACGTCTGTAGCGACCCTTTGCAGGCAGCGGAACC CCCCACCTGGCGACAGGTGCCTCTGCGGCCAAAAGCCACGTGTATAAGATAC ACCTGCAAAGGCGGCACAACCCCAGTGCCACGTTGTGAGTTGGATAGTTGTG GAAAGAGTCAAATGGCTCTCCTCAAGCGTATTCAACAAGGGGCTGAAGGAT GCCCAGAAGGTACCCCATTGTATGGGATCTGATCTGGGGCCTCGGTGCACAT GCTTTACATGTGTTTAGTCGAGGTTAAAAAAACGTCTAGGCCCCCCGAACCA CGGGGACGTGGTTTTCCTTTGAAAAACACGATGATAATATGGCCACAACTAG ACCACCATGAACACGATTAACATCGCTAAGAACGACTTCTCTGACATCGAAC TGGCTGCTATCCCGTTCAACACTCTGGCTGACCATTACGGTGAGCGTTTAGCT CGCGAACAGTTGGCCCTTGAGCATGAGTCTTACGAGATGGGTGAAGCACGCT TCCGCAAGATGTTTGAGCGTCAACTTAAAGCTGGTGAGGTTGCGGATAACGC TGCCGCCAAGCCTCTCATCACTACCCTACTCCCTAAGATGATTGCACGCATC AACGACTGGTTTGAGGAAGTGAAAGCTAAGCGCGGCAAGCGCCCGACAGCC TTCCAGTTCCTGCAAGAAATCAAGCCGGAAGCCGTAGCGTACATCACCATTA AGACCACTCTGGCTTGCCTAACCAGTGCTGACAATACAACCGTTCAGGCTGT AGCAAGCGCAATCGGTCGGGCCATTGAGGACGAGGCTCGCTTCGGTCGTATC CGTGACCTTGAAGCTAAGCACTTCAAGAAAAACGTTGAGGAACAACTCAAC AAGCGCGTAGGGCACGTCTACAAGAAAGCATTTATGCAAGTTGTCGAGGCT GACATGCTCTCTAAGGGTCTACTCGGTGGCGAGGCGTGGTCTTCGTGGCATA AGGAAGACTCTATTCATGTAGGAGTACGCTGCATCGAGATGCTCATTGAGTC AACCGGAATGGTTAGCTTACACCGCCAAAATGCTGGCGTAGTAGGTCAAGA CTCTGAGACTATCGAACTCGCACCTGAATACGCTGAGGCTATCGCAACCCGT GCAGGTGCGCTGGCTGGCATCTCTCCGATGTTCCAACCTTGCGTAGTTCCTCC TAAGCCGTGGACTGGCATTACTGGTGGTGGCTATTGGGCTAACGGTCGTCGT CCTCTGGCGCTGGTGCGTACTCACAGTAAGAAAGCACTGATGCGCTACGAAG ACGTTTACATGCCTGAGGTGTACAAAGCGATTAACATTGCGCAAAACACCGC ATGGAAAATCAACAAGAAAGTCCTAGCGGTCGCCAACGTAATCACCAAGTG GAAGCATTGTCCGGTCGAGGACATCCCTGCGATTGAGCGTGAAGAACTCCCG ATGAAACCGGAAGACATCGACATGAATCCTGAGGCTCTCACCGCGTGGAAA CGTGCTGCCGCTGCTGTGTACCGCAAGGACAAGGCTCGCAAGTCTCGCCGTA TCAGCCTTGAGTTCATGCTTGAGCAAGCCAATAAGTTTGCTAACCATAAGGC CATCTGGTTCCCTTACAACATGGACTGGCGCGGTCGTGTTTACGCTGTGTCA ATGTTCAACCCGCAAGGTAACGATATGACCAAAGGACTGCTTACGCTGGCG AAAGGTAAACCAATCGGTAAGGAAGGTTACTACTGGCTGAAAATCCACGGT GCAAACTGTGCGGGTGTCGATAAGGTTCCGTTCCCTGAGCGCATCAAGTTCA TTGAGGAAAACCACGAGAACATCATGGCTTGCGCTAAGTCTCCACTGGAGA ACACTTGGTGGGCTGAGCAAGATTCTCCGTTCTGCTTCCTTGCGTTCTGCTTT GAGTACGCTGGGGTACAGCACCACGGCCTGAGCTATAACTGCTCCCTTCCGC TGGCGTTTGACGGGTCTTGCTCTGGCATCCAGCACTTCTCCGCGATGCTCCGA GATGAGGTAGGTGGTCGCGCGGTTAACTTGCTTCCTAGTGAAACCGTTCAGG ACATCTACGGGATTGTTGCTAAGAAAGTCAACGAGATTCTACAAGCAGACG CAATCAATGGGACCGATAACGAAGTAGTTACCGTGACCGATGAGAACACTG GTGAAATCTCTGAGAAAGTCAAGCTGGGCACTAAGGCACTGGCTGGTCAAT GGCTGGCTTACGGTGTTACTCGCAGTGTGACTAAGCGTTCAGTCATGACGCT GGCTTACGGGTCCAAAGAGTTCGGCTTCCGTCAACAAGTGCTGGAAGATACC ATTCAGCCAGCTATTGATTCCGGCAAGGGTCTGATGTTCACTCAGCCGAATC AGGCTGCTGGATACATGGCTAAGCTGATTTGGGAATCTGTGAGCGTGACGGT GGTAGCTGCGGTTGAAGCAATGAACTGGCTTAAGTCTGCTGCTAAGCTGCTG GCTGCTGAGGTCAAAGATAAGAAGACTGGAGAGATTCTTCGCAAGCGTTGC GCTGTGCATTGGGTAACTCCTGATGGTTTCCCTGTGTGGCAGGAATACAAGA AGCCTATTCAGACGCGCTTGAACCTGATGTTCCTCGGTCAGTTCCGCTTACA GCCTACCATTAACACCAACAAAGATAGCGAGATTGATGCACACAAACAGGA GTCTGGTATCGCTCCTAACTTTGTACACAGCCAAGACGGTAGCCACCTTCGT AAGACTGTAGTGTGGGCACACGAGAAGTACGGAATCGAATCTTTTGCACTG ATTCACGACTCCTTCGGTACCATTCCGGCTGACGCTGCGAACCTGTTCAAAG CAGTGCGCGAAACTATGGTTGACACATATGAGTCTTGTGATGTACTGGCTGA TTTCTACGACCAGTTCGCTGACCAGTTGCACGAGTCTCAATTGGACAAAATG CCAGCACTTCCGGCTAAAGGTAACTTGAACCTCCGTGACATCTTAGAGTCGG ACTTCGCGTTCGCGGATCCAAAAAAGAAGAGAAAGGTAACTAGTGCGGCCG CTTCCCTTTAGTGAGGGTTAATGCTTCGAGCAGACATGATAAGATACATTGA TGAGTTTGGACAAACCACAACTAGAATGCAGTGAAAAAAATGCTTTATTTGT GAAATTTGTGATGCTATTGCTTTATTTGTAACCATTATAAGCTGCAATAAACA AGTTAACAACAACAATTGCATTCATTTTATGTTTCAGGTTCAGGGGGAGATG TGGGAGGTTTTTTAAAGCAAGTAAAACCTCTACAAATGTGGTAAAATCCGAT AAGGATCGATCCGGGCTGGCGTAATAGCGAAGAGGCCCGCACCGATCGCCC TTCCCAACAGTTGCGCAGCCTGAATGGCGAATGGACGCGCCCTGTAGCGGCG CATTAAGCGCGGCGGGTGTGGTGGTTACGCGCAGCGTGACCGCTACACTTGC CAGCGCCCTAGCGCCCGCTCCTTTCGCTTTCTTCCCTTCCTTTCTCGCCACGTT CGCCGGCTTTCCCCGTCAAGCTCTAAATCGGGGGCTCCCTTTAGGGTTCCGA TTTAGAGCTTTACGGCACCTCGACCGCAAAAAACTTGATTTGGGTGATGGTT CACGTAGTGGGCCATCGCCCTGATAGACGGTTTTTCGCCCTTTGACGTTGGA GTCCACGTTCTTTAATAGTGGACTCTTGTTCCAAACTGGAACAACACTCAAC CCTATCTCGGTCTATTCTTTTGATTTATAAGGGATTTTGCCGATTTCGGCCTA TTGGTTAAAAAATGAGCTGATTTAACAAATATTTAACGCGAATTTTAACAAA ATATTAACGTTTACAATTTCGCCTGATGCGGTATTTTCTCCTTACGCATCTGT GCGGTATTTCACACCGCATACGCGGATCTGCGCAGCACCATGGCCTGAAATA ACCTCTGAAAGAGGAACTTGGTTAGGTACCTTCTGAGGCGGAAAGAACCAG CTGTGGAATGTGTGTCAGTTAGGGTGTGGAAAGTCCCCAGGCTCCCCAGCAG GCAGAAGTATGCAAAGCATGCATCTCAATTAGTCAGCAACCAGGTGTGGAA AGTCCCCAGGCTCCCCAGCAGGCAGAAGTATGCAAAGCATGCATCTCAATTA GTCAGCAACCATAGTCCCGCCCCTAACTCCGCCCATCCCGCCCCTAACTCCG CCCAGTTCCGCCCATTCTCCGCCCCATGGCTGACTAATTTTTTTTATTTATGC AGAGGCCGAGGCCGCCTCGGCCTCTGAGCTATTCCAGAAGTAGTGAGGAGG CTTTTTTGGAGGCCTTTTACTTATTTACAGAACTTCGGCATTATCTTGCCGGT TCAAATTACGGTAGTGATACCCCAGAGGATTAGATGGCCAAAGAAGACAAT ATTGAAATGCAAGGTACCGTTCTTGAAACGTTGCCTAATACCATGTTCCGCG TAGAGTTAGAAAACGGTCACGTGGTTACTGCACACATCTCCGGTAAAATGCG CAAAAACTACATCCGCATCCTGACGGGCGACAAAGTGACTGTTGAACTGAC CCCGTACGACCTGAGCAAAGGCCGCATTGTCTTCCGTAGTCGCTGATTGTTT ACCGCCTGATGGGCGAAGAGAAAGAACGAGTAAAAGGTCGGTTTAACCGGC CTTTTGCGCGCATGCCCGACGGCGAGGATCTCGTCGTGACCCATGGCGATGC CTGCTTGCCGAATATCATGGTGGAAAATGGCCGCTTTTCTGGATTCATCGAC TGTGGCCGGCTGGGTGTGGCGGACCGCTATCAGGACATAGCGTTGGCTACCC GTGATATTGCTGAAGAGCTTGGCGGCGAATGGGCTGACCGCTTCCTCGTGCT TTACGGTATCGCCGCTCCCGATTCGCAGCGCATCGCCTTCTATCGCCTTCTTG ACGAGTTCTTCTGAGCGGGACTCTGGGGTTCGAAATGACCGACCAAGCGAC GCCCAACCTGCCATCACGATGGCCGCAATAAAATATCTTTATTTTCATTACAT CTGTGTGTTGGTTTTTTGTGTGAATCGATAGCGATAAGGATCCGCGTATGGT GCACTCTCAGTACAATCTGCTCTGATGCCGCATAGTTAAGCCAGCCCCGACA CCCGCCAACACCCGCTGACGCGCCCTGACGGGCTTGTCTGCTCCCGGCATCC GCTTACAGACAAGCTGTGACCGTCTCCGGGAGCTGCATGTGTCAGAGGTTTT CACCGTCATCACCGAAACGCGCGAGACGAAAGGGCCTCGTGATACGCCTAT TTTTATAGGTTAATGTCATGATAATAATGGTTTCTTAGACGTCAGGTGGCACT TTTCGGGGAAATGTGCGCGGAACCCCTATTTGTTTATTTTTCTAAATACATTC AAATATGTATCCGCTCATGAGACAATAACCCTGATAAATGCTTCAATAATAT TGAAAAAGGAAGAGTATGAGTATTCAACATTTCCGTGTCGCCCTTATTCCCT TTTTTGCGGCATTTTGCCTTCCTGTTTTTGCTCACCCAGAAACGCTGGTGAAA GTAAAAGATGCTGAAGATCAGTTGGGTGCACGAGTGGGTTACATCGAACTG GATCTCAACAGCGGTAAGATCCTTGAGAGTTTTCGCCCCGAAGAACGTTTTC CAATGATGAGCACTTTTAAAGTTCTGCTATGTGGCGCGGTATTATCCCGTATT GACGCCGGGCAAGAGCAACTCGGTCGCCGCATACACTATTCTCAGAATGACT TGGTTGAGTACTCACCAGTCACAGAAAAGCATCTTACGGATGGCATGACAGT AAGAGAATTATGCAGTGCTGCCATAACCATGAGTGATAACACTGCGGCCAA CTTACTTCTGACAACGATCGGAGGACCGAAGGAGCTAACCGCTTTTTTGCAC AACATGGGGGATCATGTAACTCGCCTTGATCGTTGGGAACCGGAGCTGAATG AAGCCATACCAAACGACGAGCGTGACACCACGATGCCTGTAGCAATGGCAA CAACGTTGCGCAAACTATTAACTGGCGAACTACTTACTCTAGCTTCCCGGCA ACAATTAATAGACTGGATGGAGGCGGATAAAGTTGCAGGACCACTTCTGCG CTCGGCCCTTCCGGCTGGCTGGTTTATTGCTGATAAATCTGGAGCCGGTGAG CGTGGGTCTCGCGGTATCATTGCAGCACTGGGGCCAGATGGTAAGCCCTCCC GTATCGTAGTTATCTACACGACGGGGAGTCAGGCAACTATGGATGAACGAA ATAGACAGATCGCTGAGATAGGTGCCTCACTGATTAAGCATTGGTAACTGTC AGACCAAGTTTACTCATATATACTTTAGATTGATTTAAAACTTCATTTTTAAT TTAAAAGGATCTAGGTGAAGATCCTTTTTGATAATCTCATGACCAAAATCCC TTAACGTGAGTTTTCGTTCCACTGAGCGTCAGACCCCGTAGAAAAGATCAAA GGATCTTCTTGAGATCCTTTTTTTCTGCGCGTAATCTGCTGCTTGCAAACAAA AAAACCACCGCTACCAGCGGTGGTTTGTTTGCCGGATCAAGAGCTACCAACT CTTTTTCCGAAGGTAACTGGCTTCAGCAGAGCGCAGATACCAAATACTGTCC TTCTAGTGTAGCCGTAGTTAGGCCACCACTTCAAGAACTCTGTAGCACCGCC TACATACCTCGCTCTGCTAATCCTGTTACCAGTGGCTGCTGCCAGTGGCGAT AAGTCGTGTCTTACCGGGTTGGACTCAAGACGATAGTTACCGGATAAGGCGC AGCGGTCGGGCTGAACGGGGGGTTCGTGCACACAGCCCAGCTTGGAGCGAA CGACCTACACCGAACTGAGATACCTACAGCGTGAGCTATGAGAAAGCGCCA CGCTTCCCGAAGGGAGAAAGGCGGACAGGTATCCGGTAAGCGGCAGGGTCG GAACAGGAGAGCGCACGAGGGAGCTTCCAGGGGGAAACGCCTGGTATCTTT ATAGTCCTGTCGGGTTTCGCCACCTCTGACTTGAGCGTCGATTTTTGTGATGC TCGTCAGGGGGGCGGAGCCTATGGAAAAACGCCAGCAACGCGGCCTTTTTA CGGTTCCTGGCCTTTTGCTGGCCTTTTGCTCACATGGCTCGACAGATCT |
| SEQ ID No.23 | GSDMD-N gene sequence | ATGGGCTCCGCCTTCGAGAGAGTGGTGAGGCGAGTGGTGCAGGAACTGGAC CACGGGGGAGAGTTTATTCCAGTGACCTCACTCCAGTCCTCCACAGGCTTCC AGCCCTACTGCCTGGTGGTGAGAAAGCCAAGCTCTTCCTGGTTTTGGAAACC CCGGTATAAGTGCGTGAATCTGTCAATTAAGGATATCCTGGAGCCCGACGCT GCCGAGCCCGATGTGCAGCGCGGAAGGTCCTTTCACTTTTACGATGCAATGG ATGGCCAGATCCAGGGCTCAGTGGAGCTGGCCGCCCCTGGGCAAGCTAAAA TTGCAGGCGGAGCCGCAGTGAGCGATAGCAGCTCAACGAGTATGAACGTGT ACTCCCTGTCAGTGGACCCTAATACATGGCAGACCCTGCTGCACGAACGCCA TCTGAGACAGCCAGAGCACAAGGTGCTCCAGCAACTTAGGAGCCGGGGCGA TAACGTGTACGTGGTGACAGAAGTGCTCCAGACCCAGAAGGAGGTGGAGGT GACCCGGACCCACAAGAGGGAAGGGTCCGGGCGGTTTTCTCTGCCCGGCGC CACATGTCTCCAGGGCGAGGGCCAGGGGCACCTGAGCCAGAAAAAGACTGT GACAATCCCAAGCGGAAGCACCTTGGCTTTCCGGGTTGCCCAGTTGGTGATC GACAGCGATCTGGACGTGCTGCTGTTCCCAGATAAGAAACAGAGGACATTTC AGCCCCCCGCTACCGGCCATAAGCGCTCCACAAGTGAGGGCGCCTGGCCTCA GCTGCCCAGCGGCCTGTCCATGATGCGGTGTCTGCACAATTTTCTGACCGAC TAA |
| SEQ ID No.24 | GSDMD-N protein sequence | MGSAFERVVRRVVQELDHGGEFIPVTSLQSSTGFQPYCLVVRKPSSSWFWKPRY KCVNLSIKDILEPDAAEPDVQRGRSFHFYDAMDGQIQGSVELAAPGQAKIAGGA AVSDSSSTSMNVYSLSVDPNTWQTLLHERHLRQPEHKVLQQLRSRGDNVYVVT EVLQTQKEVEVTRTHKREGSGRFSLPGATCLQGEGQGHLSQKKTVTIPSGSTLA FRVAQLVIDSDLDVLLFPDKKQRTFQPPATGHKRSTSEGAWPQLPSGLSMMRCL HNFLTD |
| SEQ ID No.25 | GSDME-N gene sequence | ATGGGATTTGCCAAAGCAACCAGGAATTTCTTAGAGAAGTTGATGCTGATG GTGACCTGATTGCAGTATCAAATCTGAATGACTCTGATAAGTTACAGCTTCT AAGTCTGGTGACAAAAAAGAAGAGATTCTGGTGCTGGCAGAGACCCAAGTA CCAGTTTTTATCCCTCACCCTTGGCGATGTACTCATAGAAGACCAATTTCCGA GTCCAGTGGTCGTGGAGTCGGACTTTGTGAAATACGAGGGCAAGTTTGCAAA CCACGTGAGTGGAACCCTGGAGACTGCACTGGGGAAGGTCAAGCTGAACCT GGGGGGCAGCAGCCGCGTAGAGAGCCAGTCTTCATTTGGAACCCTGAGGAA GCAGGAGGTGGATTTGCAGCAGCTCATCAGAGACTCTGCCGAGAGAACAAT AAATCTGAGAAACCCTGTGCTCCAGCAGGTGCTGGAAGGAAGGAATGAGGT CCTGTGCGTTTTGACACAGAAGATCACGACGATGCAGAAGTGTGTGATCTCT GAGCACATGCAGGTCGAGGAGAAGTGTGGTGGCATCGTGGGCATCCAGACC AAGACGGTGCAGGTGTCAGCGACGGAGGATGGGAATGTCACCAAGGACTCC AACGTGGTGCTGGAGATCCCAGCTGCCACCACCATTGCCTACGGTGTCATTG AGTTATACGTGAAACTGGACGGCCAGTTCGAGTTCTGCCTTCTCCGAGGGAA GCAAGGTGGCTTCGAGAACAAGAAGAGAATTGACTCTGTCTACCTGGACCC CCTGGTCTTTCGAGAGTTTGCATTCATAGACATGCCAGATTGA |
| SEQ ID No.26 | GSDME-N protein sequence | MGFAKATRNFLREVDADGDLIAVSNLNDSDKLQLLSLVTKKKRFWCWQRPKY QFLSLTLGDVLIEDQFPSPVVVESDFVKYEGKFANHVSGTLETALGKVKLNLGG SSRVESQSSFGTLRKQEVDLQQLIRDSAERTINLRNPVLQQVLEGRNEVLCVLTQ KITTMQKCVISEHMQVEEKCGGIVGIQTKTVQVSATEDGNVTKDSNVVLEIPAA TTIAYGVIELYVKLDGQFEFCLLRGKQGGFENKKRIDSVYLDPLVFREFAFIDMP D |
| SEQ ID No.27 | *Salmonella* alr gene | 5'- ATGCAAGCGGCAACAGTCGTCATTAACCGCCGCGCTCTGCGACACAACCTGC AACGTCTGCGTGAACTGGCGCCTGCCAGTAAGCTGGTTGCGGTGGTGAAAGC GAACGCTTATGGACACGGTCTTCTGGAGACCGCGCGAACGCTCCCTGATGCT GACGCTTTTGGCGTGGCGCGTCTTGAAGAGGCTCTACGTCTGCGAGCGGGCG GGATCACGCAGCCAATCCTGCTGCTGGAGGGTTTTTTCGACGCCGCCGATTT GCCGACCATTTCCGCGCAATGTCTGCATACCGCCGTACATAATCAAGAGCAG CTTGCCGCCCTGGAGGCGGTGGAGCTGGCGGAGCCGGTAACGGTCTGGATG AAGCTGGATACCGGTATGCATCGTCTCGGCGTGCGTCCCGAAGAGGCGGAG GCGTTCTACCAGCGTCTGACGCACTGTAAAAATGTACGCCAGCCGGTGAATA TCGTCAGCCATTTTGCCCGTGCGGATGAGCCGGAATGCGGCGCTACCGAACA TCAGCTCGACATTTTTAATGCCTTCTGTCAGGGTAAACCCGGTCAGCGCTCTA TTGCCGCGTCTGGCGGTATCCTGCTGTGGCCGCAGTCTCACTTTGACTGGGC GCGTCCGGGCATCATTTTGTATGGCGTATCGCCGCTGGAGCACAAACCCTGG GGGCCGGATTTTGGTTTTCAGCCGGTGATGTCCTTAACCTCCAGTTTGATCGC GGTGCGTGACCACAAAGCGGGCGAACCGGTGGGCTACGGCGGGACATGGGT GAGTGAGCGCGACACGCGCCTGGGCGTGGTGGCGATGGGTTATGGCGATGG CTACCCACGAGCGGCGCCTTCCGGTACGCCAGTACTGGTCAATGGTCGTGAA GTTCCGATTGTCGGGCGGGTGGCGATGGATATGATTTGCGTAGATTTGGGGC CAAACGCGCAGGATAACGCGGGCGATCCGGTGGTCTTATGGGGTGAAGGTC TGCCGGTTGAACGTATCGCTGAAATGACAAAAGTAAGTGCTTACGAACTTAT CACGCGCCTGACCTCAAGGGTGGCGATGAAGTATATTGATTAA-3' |
| SEQ ID No.28 | *Salmonella* Alr protein | MQAATVVINRRALRHNLQRLRELAPASKLVAVVKANAYGHGLLETARTLPDA DAFGVARLEEALRLRAGGITQPILLLEGFFDAADLPTISAQCLHTAVHNQEQLAA LEAVELAEPVTVWMKLDTGMHRLGVRPEEAEAFYQRLTHCKNVRQPVNIVSHF ARADEPECGATEHQLDIFNAFCQGKPGQRSIAASGGILLWPQSHFDWARPGIILY GVSPLEHKPWGPDFGFQPVMSLTSSLIAVRDHKAGEPVGYGGTWVSERDTRLG VVAMGYGDGYPRAAPSGTPVLVNGREVPIVGRVAMDMICVDLGPNAQDNAGD PVVLWGEGLPVERIAEMTKVSAYELITRLTSRVAMKYID |
| SEQ ID No.29 | *Salmonella* dadX gene | 5’- ATGACCCGCCCTATACAGGCCAGCCTTGATTTACAGGTAATGAAACAAAATT TGGCTATTGTGCGCCGGGCGGCCCCAGAGGCGCGCGTCTGGTCGGTAGTGAA AGCCAACGCCTACGGCCACGGTATTGAACGCGTCTGGAGCGCGCTGGGCGC CACAGATGGTTTCGCTATGCTCAACCTTGAAGAGGCGATTACCCTGCGTGAG CGCGGGTGGAAAGGGCCGATATTGATGCTGGAGGGGTTTTTCCATGCGCAA GACCTGGAGGCGTATGACACTTATCGGCTGACCACCTGCATCCACAGTAACT GGCAGTTGAAAGCGCTGCAAAATGCGCGGCTTAACGCGCCGCTGGATATCT ATGTCAAAGTCAACAGCGGCATGAACCGACTCGGTTTTCAGCCTGAGCGGGC GCAGACCGTCTGGCAGCAATTGCGGGCAATGCGCAATGTCGGTGAAATGAC CCTGATGTCACATTTTGCTCAGGCCGATCATCCGGAAGGTATCGGGGAGGCG ATGAGGCGTATTGCGCTGGCGACGGAAGGTCTTCAGTGTGCATACTCGTTAT CAAATTCGGCAGCGACGCTGTGGCATCCCCAGGCGCATTATGACTGGGTCAG GCCGGGCATTATTTTGTACGGCGCGTCGCCGTCAGGACAGTGGCGGGATATT GCCGATACCGGACTAAAACCTGTGATGACGCTGAGTAGCGAAATTATTGGC GTGCAGACGCTGAGCGCGGGTGAAAGGGTAGGCTATGGCGGAGGGTATTCT GTGACTCAGGAACAACGCATTGGCATCGTCGCGGCGGGTTATGCCGATGGCT ATCCACGCCATGCGCCGACCGGGACGCCTGTGCTGGTGGACGGTATCCGTAC CAGAACAGTAGGCACCGTTTCAATGGATATGCTGGCGGTGGATTTGACGCCG TGTCCGCAGGCGGAATCGGCACGCCGGTTGAATTATGGGGCAAAGAAATT AAGGTCGATGATGTCGCTTCTGCGGCAGGCACGCTGGGCTATGAGCTACTGT GCGCCGTAGCGCCGCGTGTGCCGTTTGTGACAACGTAA-3' |
| SEQ ID No.30 | *Salmonella* DadX protein | MTRPIQASLDLQVMKQNLAIVRRAAPEARVWSVVKANAYGHGIERVWSALGA TDGFAMLNLEEAITLRERGWKGPILMLEGFFHAQDLEAYDTYRLTTCIHSNWQL KALQNARLNAPLDIYVKVNSGMNRLGFQPERAQTVWQQLRAMRNVGEMTLM SHFAQADHPEGIGEAMRRIALATEGLQCAYSLSNSAATLWHPQAHYDWVRPGII LYGASPSGQWRDIADTGLKPVMTLSSEIIGVQTLSAGERVGYGGGYSVTQEQRI GIVAAGYADGYPRHAPTGTPVLVDGIRTRTVGTVSMDMLAVDLTPCPQAGIGTP VELWGKEIKVDDVASAAGTLGYELLCAVAPRVPFVTT |
| SEQ ID No.31 | *Salmonella* yhbU promoter | 5’- GCGTGAACGCAGTTTATCTAACACGAGCTTTACTCCCTGTTTCAATAGTCCTG TTATTTTGCCATATCCAATAAATCACATGGCGGCGTAAATCAACAATTGGGT CTGATTTTCTACCCCATTATTGGTGTTACCAATACGCCTTTAACTGCCTTAAA TCAAAAATTGTCGCAGCAAGGTTAACTAAAATCCCTGTTCGTTAACAATTTT GCGTCTCAGACGTAACCCTCAGGATAA-3’ |
| SEQ ID No.32 | *Escherichia coli* ynfK promoter | 5’- CGCTGCACTGGTAAAAGACGCGATGTATAACGGTTCTTTGTTGATTCGTCTG TTGCAGGGTTAACATTTTTTAACTGTTCTACCAAAATTTGCGCTATCTCAATT TGGGCCAGGAAAGCATAACTTAGACTTTCAAGGTTAATTATTTTCCTGGTTT ATATTTGTGAAGCATAACGGTGGAGTTA-3’ |
| SEQ ID No.33 | *Salmonella* cyoA promoter | 5'- AACATTATTTGCCGTTGTAATAACGACCCGGAATTATAACGGCTAAATAACC ATCAATTGATAAAAAATATATTTGTCTTTTTGTGCGCAACAGAATTTTGGAA ATTATTGGTAATTTCTACGCTACCTATTTTCTTTACACGATTCAGCCGATTAA ATTTAAATTTCTGCCGTTGATTGTCCTTTTATTGATAATAAATTGTTAAGTAA TTGTTTTATTTTTACATTGGTTATACCAATTATCCCTCAACATCCCACTTACA GACCTCATCCGTTATAACGCAAAGTTTCAACAGTGTCTTAAAGTTATTTCGA CATACTTGGCAACATATGTGACATGCGCGGCAGAACTTGGTAACACAGAAC CAAT-3' |
| SEQ ID No.34 | *Salmonella* ydcI promoter | 5’- AATGTCATGCCTCCAGTGAATGTTACCTGGAGTGTAGAAAACCTCATCCATT CCGGGGAGAAGAATTTATAAAGTGTGATCCGACCTGAATTCATTATGCAGAA CGGATTGACATGAGCTGGCATATCCCCGACCAGATCGCGGCTTAACACGCTT AACGGCAGTTGAATCATCAGTAAAATAAAACAGTTGTAACTTGAAATGGTA AAATGTTAATAATATTTTGCTATCAGGTTATCAAAAACAAACAACTTTACTT GTCACCCGCACGGCTCTGTTTTTAACATCGCTT-3’ |
| SEQ ID No.35 | ribosome binding site (RBS) | TAGTAAGAAGGAGATATACAT |
| SEQ ID No.36 | pUC origin of replication | CTCGAGCATGTGAGCAAAAGGCCAGCAAAAGGCCAGGAACCGTAAAAAGG CCGCGTTGCTGGCGTTTTTCCATAGGCTCCGCCCCCCTGACGAGCATCACAA AAATCGACGCTCAAGTCAGAGGTGGCGAAACCCGACAGGACTATAAAGATA CCAGGCGTTTCCCCCTGGAAGCTCCCTCGTGCGCTCTCCTGTTCCGACCCTGC CGCTTACCGGATACCTGTCCGCCTTTCTCCCTTCGGGAAGCGTGGCGCTTTCT CATAGCTCACGCTGTAGGTATCTCAGTTCGGTGTAGGTCGTTCGCTCCAAGC TGGGCTGTGTGCACGAACCCCCCGTTCAGCCCGACCGCTGCGCCTTATCCGG TAACTATCGTCTTGAGTCCAACCCGGTAAGACACGACTTATCGCCACTGGCA GCAGCCACTGGTAACAGGATTAGCAGAGCGAGGTATGTAGGCGGTGCTACA GAGTTCTTGAAGTGGTGGCCTAACTACGGCTACACTAGAAGGACAGTATTTG GTATCTGCGCTCTGCTGAAGCCAGTTACCTTCGGAAAAAGAGTTGGTAGCTC TTGATCCGGCAAACAAACCACCGCTGGTAGCGGTGGTTTTTTTGTTTGCAAG CAGCAGATTACGCGCAGAAAAAAAGGATCTCAAGAAGATCCTTTGATCTTTT CTACGGGGTCTGACGCTCAGTGGAACGAAAACTCACGTTAAGGGATTTTGGT CATGAGATTATCAAAAAGGATCTTCACCTAGATCCTTTTAAATTAAAAATGA AGTTTTAAATCAATCTAAAGTATATATGAGTAAGCGGCCGC |
| SEQ ID No.37 | sequence in pSWT007 containing two direct repeat loxP sequences flanking a chloramphenico 1 resistance gene, with *Spe*I and *NotI* sites at both ends | ACTAGTGAACCTCTTCGAGGGACCTAATAACTTCGTATAGCATACATTATAC GAAGTTATATTAAGGGTTCCGGATCCTAATTGATATCTAAGGGCACCAATAA CTGCCTTAAAAAAATTATGCACCACCTTGCCATTCATCACAATACTGTTGCA GTTCATTCAGCATACGACCAACATGAAAACCATCACAAACTGCATGATGAAC CTGAATTGCCAGCGGCATCAGAACTTTATCACCCTGGGTGTAATATTTGCCC ATCGTAAAAACCGGTGCGAAAAAGTTATCCATATTGGCCACATTCAGATCAA ATGAGGTAAAGCTAACCCACGGATTTGCGCTAACAAAGAACATGTTTTCGAT AAAGCCTTTCGGAAAATATGCCAGGTTTTCACCATAACATGCAACATCCTGG CTATAGATGTGCAGGAACTGACGAAAATCATCATGATATTCTGACCACAGGC TGCTAAAGGTTTCGGTCTGTTCATGAAAAACGGTATAACACGGATGAACGCT ATCCCAAATAACCAGTTCACCATCTTTCATTGCCATACGAAATTCCGGATGG GCATTCATCAGACGTGCCAGAATATGAATAAAGGCAGGGTAGAACTTGTGTT TGTTCTTTTTCACGGTTTTCAGAAATGCGGTTATATCCAGCTGAACGGTCTGA TTATAGGTACACTGTGCAACGCTCTGAAATGCTTCAAAGTGTTCTTTACGAT GCCACTGGCTAATATCAACGGTGGTATAACCGGTGATTTCTTTTCCATTTTA GCTTCCTTAGCTCCTGAAAATCTCGATAACTCAAAAAATACGCCCGGTAGTG ATCTTATTTCATTATGGTGAAAGTTGGAACCTCTTACGTGCCGATCAACGTCT CATTTTCGCCAAAAGTTGGCCCAGGGCTTCCCGGTATCAACAGGGACACCAG GATTTATTTATTCTGCGAAGTGATCTTCCGTCACAGGTATTTATTCGGCGCAA AGTGCGTCGGGTGATGCTGCCGATATCGGACCCGGGATCCTAATTGGCTGGA CGTAAACTCCTCTTCAGACCTAATAACTTCGTATAGCATACATTATACGAAG TTATATTAAGGGTTATTGAATATGATCGGGCGGCCGC |
| SEQ ID No.38 | sequence in pSWT001 containing recombinase and Cre recombinase | GAATTCTCAGCCAAACGTCTCTTCAGGCCACTGACTAGCGATAACTTTCCCC ACAACGGAACAACTCTCATTGCATGGGATCATTGGGTACTGTGGGTTTAGTG GTTGTAAAAACACCTGACCGCTATCCCTGATCAGTTTCTTGAAGGTAAACTC ATCACCCCCAAGTCTGGCTATGCAGAAATCACCTGGCTCAACAGCCTGCTCA GGGTCAACGAGAATTAACATTCCGTCAGGAAAGCTTGGCTTGGAGCCTGTTG GTGCGGTCATGGAATTACCTTCAACCTCAAGCCAGAATGCAGAATCACTGGC TTTTTTGGTTGTGCTTACCCATCTCTCCGCATCACCTTTGGTAAAGGTTCTAA GCTTAGGTGAGAACATCCCTGCCTGAACATGAGAAAAAACAGGGTACTCAT ACTCACTTCTAAGTGACGGCTGCATACTAACCGCTTCATACATCTCGTAGATT TCTCTGGCGATTGAAGGGCTAAATTCTTCAACGCTAACTTTGAGAATTTTTGT AAGCAATGCGGCGTTATAAGCATTTAATGCATTGATGCCATTAAATAAAGCA CCAACGCCTGACTGCCCCATCCCCATCTTGTCTGCGACAGATTCCTGGGATA AGCCAAGTTCATTTTTCTTTTTTTCATAAATTGCTTTAAGGCGACGTGCGTCC TCAAGCTGCTCTTGTGTTAATGGTTTCTTTTTTGTGCTCATACGTTAAATCTAT CACCGCAAGGGATAAATATCTAACACCGTGCGTGTTGACTATTTTACCTCTG GCGGTGATAATGGTTGCATGTACTAAGGAGGTTGCGCATGCAGGTACCAATG TCCAATTTACTGACCGTACACCAAAATTTGCCTGCATTACCGGTCGATGCAA CGAGTGATGAGGTTCGCAAGAACCTGATGGACATGTTCAGGGATCGCCAGG CGTTTTCTGAGCATACCTGGAAAATGCTTCTGTCCGTTTGCCGGTCGTGGGCG GCATGGTGCAAGTTGAATAACCGGAAATGGTTTCCCGCAGAACCTGAAGAT GTTCGCGATTATCTTCTATATCTTCAGGCGCGCGGTCTGGCAGTAAAAACTA TCCAGCAACATTTGGGCCAGCTAAACATGCTTCATCGTCGGTCCGGGCTGCC ACGACCAAGTGACAGCAATGCTGTTTCACTGGTTATGCGGCGGATCCGAAAA GAAAACGTTGATGCCGGTGAACGTGCAAAACAGGCTCTAGCGTTCGAACGC ACTGATTTCGACCAGGTTCGTTCACTCATGGAAAATAGCGATCGCTGCCAGG ATATACGTAATCTGGCATTTCTGGGGATTGCTTATAACACCCTGTTACGTATA GCCGAAATTGCCAGGATCAGGGTTAAAGATATCTCACGTACTGACGGTGGG AGAATGTTAATCCATATTGGCAGAACGAAAACGCTGGTTAGCACCGCAGGT GTAGAGAAGGCACTTAGCCTGGGGGTAACTAAACTGGTCGAGCGATGGATT TCCGTCTCTGGTGTAGCTGATGATCCGAATAACTACCTGTTTTGCCGGGTCAG AAAAAATGGTGTTGCCGCGCCATCTGCCACCAGCCAGCTATCAACTCGCGCC CTGGAAGGGATTTTTGAAGCAACTCATCGATTGATTTACGGCGCTAAGGATG ACTCTGGTCAGAGATACCTGGCCTGGTCTGGACACAGTGCCCGTGTCGGAGC CGCGCGAGATATGGCCCGCGCTGGAGTTTCAATACCGGAGATCATGCAAGCT GGTGGCTGGACCAATGTAAATATTGTCATGAACTATATCCGTAACCTGGATA GTGAAACAGGGGCAATGGTGCGCCTGCTGGAAGATGGCGATTAGGGGTACC GAGCTCATGAACGCTTATTACATTCAGGATCGTCTTGAGGCTCAGAGCTGGG CGCGTCACTACCAGCAGCTCGCCCGTGAAGAGAAAGAGGCAGAACTGGCAG ACGACATGGAAAAAGGCCTGCCCCAGCACCTGTTTGAATCGCTATGCATCGA TCATTTGCAACGCCACGGGGCCAGCAAAAAATCCATTACCCGTGCGTTTGAT GACGATGTTGAGTTTCAGGAGCGCATGGCAGAACACATCCGGTACATGGTTG AAACCATTGCTCACCACCAGGTTGATATTGATTCAGAGGTATAAAACGAATG AGTACTGCACTCGCAACGCTGGCTGGGAAGCTGGCTGAACGTGTCGGCATG GATTCTGTCGACCCACAGGAACTGATCACCACTCTTCGCCAGACGGCATTTA AAGGTGATGCCAGCGATGCGCAGTTCATCGCATTACTGATCGTTGCCAACCA GTACGGCCTTAATCCGTGGACGAAAGAAATTTACGCCTTTCCTGATAAGCAG AATGGCATCGTTCCGGTGGTGGGCGTTGATGGCTGGTCCCGCATCATCAATG AAAACCAGCAGTTTGATGGCATGGACTTTGAGCAGGACAATGAATCCTGTAC ATGCCGGATTTACCGCAAGGACCGTAATCATCCGATCTGCGTTACCGAATGG ATGGATGAATGCCGCCGCGAACCATTCAAAACTCGCGAAGGCAGAGAAATC ACGGGGCCGTGGCAGTCGCATCCCAAACGGATGTTACGTCATAAAGCCATG ATTCAGTGTGCCCGTCTGGCCTTCGGATTTGCTGGTATCTATGACAAGGATG AAGCCGAGCGCATTGTCGAAAATACTGCATACACTGCAGAACGTCAGCCGG AACGCGACATCACTCCGGTTAACGATGAAACCATGCAGGAGATTAACACTCT GCTGATCGCCCTGGATAAAACATGGGATGACGACTTATTGCCGCTCTGTTCC CAGATATTTCGCCGCGACATTCGTGCATCGTCAGAACTGACACAGGCCGAAG CAGTAAAAGCTCTTGGATTCCTGAAACAGAAAGCCGCAGAGCAGAAGGTGG CAGCATGACACCGGACATTATCCTGCAGCGTACCGGGATCGATGTGAGAGCT GTCGAACAGGGGGATGATGCGTGGCACAAATTACGGCTCGGCGTCATCACC GCTTCAGAAGTTCACAACGTGATAGCAAAACCCCGCTCCGGAAAGAAGTGG CCTGACATGAAAATGTCCTACTTCCACACCCTGCTTGCTGAGGTTTGCACCG GTGTGGCTCCGGAAGTTAACGCTAAAGCACTGGCCTGGGGAAAACAGTACG AGAACGACGCCAGAACCCTGTTTGAGTTCACTTCCGGCGTGAATGTTACTGA ATCCCCGATCATCTATCGCGACGAAAGTATGCGTACCGCCTGCTCTCCCGAT GGTTTATGCAGTGACGGCAACGGCCTTGAACTGAAATGCCCGTTTACCTCCC GGGATTTCATGAAGTTCCGGCTCGGTGGTTTCGAGGCCATAAAGTCAGCTTA CATGGCCCAGGTGCAGTACAGCATGTGGGTGACGCGAAAAAATGCCTGGTA CTTTGCCAACTATGACCCGCGTATGAAGCGTGAAGGCCTGCATTATGTCGTG ATTGAGCGGGATGAAAAGTACATGGCGAGTTTTGACGAGATCGTGCCGGAG TTCATCGAAAAAATGGACGAGGCACTGGCTGAAATTGGTTTTGTATTTGGGG AGCAATGGCGATGACTCGAG |
| SEQ ID No.39 | sequence in pSWT002 containing two direct repeat loxP sequences flanking a chloramphenico l resistance gene, with *Not*I sites at both ends | GCGGCCGCGACTAGTGAACCTCTTCGAGGGACCTAATAACTTCGTATAGCAT ACATTATACGAAGTTATATTAAGGGTTCCGGATCCTAAGCTTGATATCTAAG GGCACCAATAACTGCCTTAAAAAAATTACGCCCCGCCCTGCCACTCATCGCA GTACTGTTGTAATTCATTAAGCATTCTGCCGACATGGAAGCCATCACAGACG GCATGATGAACCTGAATCGCCAGCGGCATCAGCACCTTGTCGCCTTGCGTAT AATATTTGCCCATGGTGAAAACGGGGGCGAAGAAGTTGTCCATATTGGCCAC GTTTAAATCAAAACTGGTGAAACTCACCCAGGGATTGGCTGAGACGAAAAA CATATTCTCAATAAACCCTTTAGGGAAATAGGCCAGGTTTTCACCGTAACAC GCCACATCTTGCGAATATATGTGTAGAAACTGCCGGAAATCGTCGTGGTATT CACTCCAGAGCGATGAAAACGTTTCAGTTTGCTCATGGAAAACGGTGTAACA AGGGTGAACACTATCCCATATCACCAGCTCACCGTCTTTCATTGCCATACGG AATTCCGGATGAGCATTCATCAGGCGGGCAAGAATGTGAATAAAGGCCGGA TAAAACTTGTGCTTATTTTTCTTTACGGTCTTTAAAAAGGCCGTAATATCCAG CTGAACGGTCTGGTTATAGGTACATTGAGCAACTGACTGAAATGCCTCAAAA TGTTCTTTACGATGCCATTGGGATATATCAACGGTGGTATATCCAGTGATTTT TTTCTCCATTTTAGCTTCCTTAGCTCCTGAAAATCTCGATAACTCAAAAAATA CGCCCGGTAGTGATCTTATTTCATTATGGTGAAAGTTGGAACCTCTTACGTGC CGATCAACGTCTCATTTTCGCCAAAAGTTGGCCCAGGGCTTCCCGGTATCAA CAGGGACACCAGGATTTATTTATTCTGCGAAGTGATCTTCCGTCACAGGTAT TTATTCGGCGCAAAGTGCGTCGGGTGATGCTGCCGATATCGGTACCCGGGAT CCTAAGCTTGGCTGGACGTAAACTCCTCTTCAGACCTAATAACTTCGTATAG CATACATTATACGAAGTTATATTAAGGGTTATTGAATATGATCGGGCGGCCG C |
| SEQ ID No.40 | sseA promoter + RBS site | GCGGCCGCAAGAGAACAACGGCAAGTTACAGGATCCGCAGCAATATCAGCA AAACACCTTATTGCTTGAAGCGATCGAGCAGGCCGAAAATATCATCAACATT ATTTATTATCGTTACCATAACAGCGCACTTGTAGTGAGTGAGCAAGAGTAAA GTAAAAATATCTTAGAGCCTATCCCACCAGGCGTTAATTGGCGCAGCCAGTT TGGACACGGATAGCGCGCAAAAACCGCAGCGTACACGTAGTACGTGAGGTT TGACTCGCTACGCTCGCCCTTCGGGCCGCCGCTAGCGGCGTTCAAAACGCTA ACGCGTTTTGGCGAGCACTGCCCAGGTTCAAAATGGCAAGTAAAATAGCCCT AATGGGATAGGCTCTTAGTTAGCACGTTAATTATCTATCGAAGCTTAAGAAG GAGATATACATATG |
| SEQ ID No.41 | pepT | GTAAACGCAACGGATGGCTGACCGCTGCGGGGTTTGTGGTTAACCACCTTAA TCACTCTTAATGAGGGCGGTCATTCTACGGCAAACCACCGTGATCGCCAATC CTTGTTGCGAATTACTGACTTAGCTTTATAGTCAGAAAGCGTGTCAAAGTGA AATATTCTTGTTTGCAGGGATAAAAGTGACCTGACGCAATATTTGTCTTTTCT TGCTTATTAATAATGTTGTCACGAAAAG |
| SEQ ID No.42 | coding region of asd gene | ATGAAAAATGTTGGTTTTATCGGCTGGCGCGGAATGGTCGGCTCTGTTCTCA TGCAACGCATGGTAGAGGAGCGCGATTTCGACGCTATTCGCCCTGTTTTCTTT TCTACCTCCCAGTTTGGACAGGCGGCGCCCACCTTCGGCGACACCTCCACCG GCACGCTACAGGACGCTTTTGATCTGGATGCGCTAAAAGCGCTCGATATCAT CGTGACCTGCCAGGGCGGCGATTATACCAACGAAATTTATCCAAAGCTGCGC GAAAGCGGATGGCAGGGTTACTGGATTGATGCGGCTTCTACGCTGCGCATGA AAGATGATGCCATTATTATTCTCGACCCGGTCAACCAGGACGTGATTACCGA CGGCCTGAACAATGGCGTGAAGACCTTTGTGGGCGGTAACTGTACCGTTAGC CTGATGTTGATGTCGCTGGGCGGTCTCTTTGCCCATAATCTCGTTGACTGGGT ATCCGTCGCGACCTATCAGGCCGCCTCCGGCGGCGGCGCGCGCCATATGCGC GAGCTGTTAACCCAGATGGGTCAGTTGTATGGCCATGTCGCCGATGAACTGG CGACGCCGTCTTCCGCAATTCTTGATATTGAACGCAAAGTTACGGCATTGAC CCGCAGCGGCGAGCTGCCGGTTGATAACTTTGGCGTACCGCTGGCGGGAAG CCTGATCCCCTGGATCGACAAACAGCTCGATAACGGCCAGAGCCGCGAAGA GTGGAAAGGCCAGGCGGAAACCAACAAGATTCTCAATACTGCCTCTGTGATT CCGGTTGATGGTTTGTGTGTGCGCGTCGGCGCGCTGCGCTGTCACAGCCAGG CGTTCACCATCAAGCTGAAAAAAGAGGTATCCATTCCGACGGTGGAAGAAC TGCTGGCGGCACATAATCCGTGGGCGAAAGTGGTGCCGAACGATCGTGATA TCACTATGCGCGAATTAACCCCGGCGGCGGTGACCGGCACGTTGACTACGCC GGTTGGTCGTCTGCGTAAGCTGAACATGGGGCCAGAGTTCTTGTCGGCGTTT ACCGTAGGCGACCAGTTGTTATGGGGCGCCGCCGAGCCGCTGCGTCGAATGC TGCGCCAGTTGGCGTAG |
| SEQ ID No.43 | sodA | ACGAAAAGTACGGCATTGATAATCATTTTCAATATCATTTAATTAACTATAA TGAACCAA |

The specific sequences of all primers used in the present invention are shown in Table 6.

**Table 6 Specific sequences of all primers used in the present invention**

| **SEQ ID No.** | **No.** | **Sequence** | **Usage** |
|---|---|---|---|
| SEQ ID No.44 | SWTO1 | ATGGAATCCCTGACGTTACAACCCATCGCGCGGTCG ATGGCGCCATTAACCGATCATATTCAATAACCCT | Deletion of *aroA* gene |
| SEQ ID No.45 | SWTO2 | GAAGACTTAGGCAGGCGTACTCATTCGCGCCAGTTGT TCGAAATAATCAGGACTAGTGAACCTCTTCGAGGG | |
| SEQ ID No.46 | SWTO3 | CGGACAATACGCTGGACAAG | Forward primer for identification of *aroA* |
| SEQ ID No.47 | SWTO4 | GCGGCTTTGTAGGTTGACAT | Reverse primer for identification of *aroA* |
| SEQ ID No.48 | SWTO5 | TCCTTAGCTCCTGAAAATCTCG | Reverse primer for detection of chloramphenicol resistance gene |
| SEQ ID No.49 | SWTO6 | TGTGGCGTGTTACGGTGAAA | Forward primer for detection of chloramphenicol resistance gene |
| SEQ ID No.50 | SWTO11 | ATTTGCGGCCGCGTAAACGCAACGGATGGCTGACCGC | notI-pepT-Forward |
| SEQ ID No.51 | SWTO12 | CCCAAGCTTCTTTTCGTGACAACATTATTAATAAG | hindIII-pepT-Reverse |
| SEQ ID No.52 | SWTO13 | CCCAAGCTTTGGAGCGAAACCGATGAAAAATGTTGGT TTTATCGGCTGGC | hindIII-asd-Forward |
| SEQ ID No.53 | SWTO14 | CCGCTCGAGCTACGCCAACTGGCGCAGCATTCGA | xhoI-asd-Reverse |
| SEQ ID No.54 | SWTO15 | GACGAAAAGTACGGCATTGATAATCATTTTCAATATC ATTTAATTAACTATAATGAACCAAC | xhoI-sodA-Forward |
| SEQ ID No.55 | SWTO16 | TCGAGTTGGTTCATTATAGTTAATTAAATGATATTGAA AATGATTATCAATGCCGTACTTTTCGTCTGCA | pstI-sodA-Reverse |
| SEQ ID No.56 | SWTO17 | CGCATACACAGCACATCTCTTTGCAGGAAAAAAACGC TGAACCTCTTCGAGGGACCTAAT | Amplification of cm-*pepT-asd-sodA* and recombination into the chromosome |
| SEQ ID No.57 | SWTO18 | GAACCACACGCAGGCCCGATAAGCGCTGCAATAGCCA ACGAAAAGTACGGCATTGATAATC | |
| SEQ ID No.58 | SWTO19 | ATTTGCGGCCGCGCGTGAACGCAGTTTATCTAACACG | NotI-yhbU-S promoter Forward |
| SEQ ID No.59 | SWTO20 | CCCAAGCTTTTATCCTGAGGGTTACGTCTGAGACG | HindIII-*yhbU*-S promoter Reverse |
| SEQ ID No.60 | SWTO21 | ATTTGCGGCCGCCGCTGCACTGGTAAAAGACG | NotI-*ynfK*-E promoter Forward |
| SEQ ID No.61 | SWTO22 | CCCAAGCTTAACTCCACCGTTATGCTTCAC | HindIII-*-ynfK-*E promoter Reverse |
| SEQ ID No.62 | SWTO35 | CCCAAGCTTTGGAGCGAAACCGATGCAAGCGGCAACA GTCGTC | HindIII-*alr*-S gene Forward |
| SEQ ID No.63 | SWTO36 | CCGCTCGAGTTAATCAATATACTTCATCGCCACCCTTG | XhoI*-alr-*S gene Reverse |
| SEQ ID No.64 | SWTO39 | CCCAAGCTTTGGAGCGAAACCGATGACCCGCCCTATA CAGGC | HindIII-*dadX-*S gene Forward |
| SEQ ID No.65 | SWTO40 | CCCAAGCTTTTACGTTGTCACAAACGGCAC | XhoI-*dadX*-S gene Reverse |
| SEQ ID No.66 | SWTO43 | CCGCTCGAGATTGGTTCTGTGTTACCAAGTTCTGC | XhoI-c*yoA-*S promoter Forward |
| SEQ ID No.67 | SWTO44 | AAAACTGCAGAACATTATTTGCCGTTGTAATAACG | PstI-*cyoA*-S promoter Reverse |
| SEQ ID No.68 | SWTO51 | CCGCTCGAGAAGCGATGTTAAAAACAGAGCCG | XhoI-*ydcI*-S promoter Forward |
| SEQ ID No.69 | SWTO52 | AAAACTGCAGAATGTCATGCCTCCAGTGAATGTTAC | PstI-*ydcI*-S promoter Reverse |
| SEQ ID No.70 | SWTO59 | GGTACGCAAGATTGGGGTAC | Reverse primer 1 for detection of *dadX* deletion |
| SEQ ID No.71 | SWTO61 | TGTCAGCTCTTTACCCAGCG | Identification primer 1 for *dadX* for strain verification |
| SEQ ID No.72 | SWTO70 | GCAATACGATGATGAGTAACTCTCCGTCATTCTTTTAA CAAGGAATTCAACCGATCATATTCAATAACCCT | Forward primer for *alr* gene deletion |
| SEQ ID No.73 | SWTO71 | TGCGCGATACAAGCCGGATAAGCGCAAGCGCCACCCG GCCCGCCGCGTATGACTAGTGAACCTCTTCGAGGG | Reverse primer for *alr* gene deletion |
| SEQ ID No.74 | SWTO72 | CAGGTCTTACGCCGACAGAA | Forward primer for verification of *alr-*deleted strain |
| SEQ ID No.75 | SWTO73 | GTTGCTGAAGAACCGGGTGA | Reverse primer for verification of *alr-*deleted strain |
| SEQ ID No.76 | SWTO75 | CGACGGCGATGCTTAAAGGC | Identification primer 2 for *dadX* for strain verification |
| SEQ ID No.77 | SWTO78 | CACCCCCCCGCAACGTTTGCATAGCGCGCATAACTGA TAAAGGAAGTGAACAGGAAACAGCTATGAC | Universal forward primer for *dadX* gene deletion |
| SEQ ID No.78 | SWTO79 | ACGTCGACCCGCCCCCAAGTGGACCGGTCGACGCCTT AGCCTGAATTAGGGTAAAACGACGGCCAGT | Universal reverse primer for *dadX* gene deletion |
| SEQ ID No.79 | SWTO85 | GCAATACGATGATGAGTAACTCTCCGTCATTCTTTTAA CAAGGAATTCAAGAACAGGAAACAGCTATGAC | Universal forward primer for *alr* deletion for hypoxic expression cassette library amplification (*dadX* as essential gene) |
| SEQ ID No.80 | SWTO86 | TGCGCGATACAAGCCGGATAAGCGCAAGCGCCACCCG GCCCGCCGCGTATGTAAAACGACGGCCAGT | Universal reverse primer for *alr* deletion for hypoxic expression cassette library amplification (*dadX* as essential gene) |
| SEQ ID No.81 | SWTO87 | GCACCTTAGGCTGGACGATG | Identification primer 3 for *dadX* for strain verification |
| SEQ ID No.82 | SWTO93 | TCGCCACTGGACTTTCTGCT | Forward primer for verification of *asd-*deleted strain |
| SEQ ID No.83 | SWTO94 | CTGCGTCGTCCTACCTTCAG | Reverse primer for verification of *asd-*deleted strain |
| SEQ ID No.84 | SWTO95 | AAGAGGTATCCATTCCGACG | Internal forward primer for cm*-pepT-asd-sodA* |
| SEQ ID No.85 | SWTO98 | TAACCGCTCCCTGGAACAAC | Identification primer 1 for *alr* for strain verification |
| SEQ ID No.86 | SWTO99 | GGTAAGCAACGTAACGGTCC | Identification primer 2 for *alr* for strain verification |
| SEQ ID No.87 | SWTO100 | GGCCGCTCACTCATTAGGCACCCCAGGCTTTACACTTT ATGCTTCCGGCTCGTATAATGTGTGGAAA | Notl-lacUV5-HindIII Forward |
| SEQ ID No.88 | SWTO101 | AGCTTTTCCACACATTATACGAGCCGGAAGCATAAAG TGTAAAGCCTGGGGTGCCTAATGAGTGAGC | Notl-lacUV5-HindIII Reverse |
| SEQ ID No.89 | SWTO102 | CGCATACACAGCACATCTCTTTGCAGGAAAAAAACGC TGAACAGGAAACAGCTATGAC | amplification of T7 polymerase , universal forward primer for *asd* deletion |
| SEQ ID No.90 | SWTO103 | GAACCACACGCAGGCCCGATAAGCGCTGCAATAGCCA GTAAAACGACGGCCAGT | amplification of T7 polymerase , universal reverse primer for *asd* deletion |
| SEQ ID No.91 | SWTO104 | GACTGCCGATTGCCATTACC | Forward primer for identification of *Salmonella asd* mutation |
| SEQ ID No.92 | SWTO105 | TGAGGTTCTGCTGTCCGTGC | Reverse primer for identification of *Salmonella asd* mutation |
| SEQ ID No.93 | SWTO106 | CATATCTGCTTCAACAACCTGC | Reverse primer for identification of T7 RNA polymerase |
| SEQ ID No.94 | SWTO107 | CGTGAAGAACTGCCGATGAA | Forward primer for identification of T7 RNA polymerase |
| SEQ ID No.95 | SWTO116 | GAATGGTGGCGTACCGTTAT | pPRO010 detection primer 5 Forward |
| SEQ ID No.96 | SWTO129 | TCAACAAGGGGCTGAAGGAT | pEU011 identification primer 1 Forward |
| SEQ ID No.97 | SWTO130 | TGATGCCGTTCTTCTGCTTG | pEU011 identification primer 1 Reverse |
| SEQ ID No.98 | SWTO131 | AGCAAAGACCCCAACGAGAA | pEU011 identification primer 2 Forward |
| SEQ ID No.99 | SWTO132 | CGGCAGGCTTACCAGTATGAG | pEU011 identification primer 2 Reverse |
| SEQ ID No.100 | SWTO133 | GCTCTAGAACGCGTCGAGCATGCATCTA | Forward primer for amplification of the second IRES (containing XbaI) |
| SEQ ID No.101 | SWTO134 | CCGGAATTCGGCIIIIICAAAGGAAAACCACG | Reverse primer for amplification of the second IRES (containing EcoRI) |
| SEQ ID No.102 | SWTO135 | CCGGAATTCGCCACCATGAACACCATCAACATTGCC | Forward primer for amplification of T7 RNA polymerase (containing Kozak sequence and EcoRI) |
| SEQ ID No.103 | SWTO136 | TCCGAGCTCTTAGGCAAAGGCAAAATCGC | Reverse primer for amplification of T7 RNA polymerase (containing SacI) |
| SEQ ID No.104 | SWTO137 | AGCAAAGACCCCAACGAGAA | Forward identification primer 1 for pEU010 |
| SEQ ID No.105 | SWTO138 | CATATCTGCTTCAACAACCTGC | Reverse identification primer 1 for pEU010 |
| SEQ ID No.106 | SWTO139 | AAGTCGCTCTCATTACTGGCGTAACCGGACAGGATGG GTCTTACCTGGCAGAACAGGAAACAGCTATGAC | Universal forward primer for *gmd* deletion and amplification of T7 polymerase |
| SEQ ID No.107 | SWTO140 | TCTCAAGGAACCACTGGTAAGTACCGGCAAGCCCTGC CTCCAGTGAAATTGTAAAACGACGGCCAGT | Universal reverse primer for *gmd* deletion and amplification of T7 polymerase |
| SEQ ID No.108 | SWTO141 | ATTGGCGCACATGCGGTTAT | Forward primer for identification of *Salmonella gmd* mutation |
| SEQ ID No.109 | SWTO142 | AAGTGCTGCCAGACGCCATAAA | Reverse primer for identification of *Salmonella gmd* mutation |
| SEQ ID No.110 | SWTO143 | TATCTTGCCGGTTCAAATTATGGTAGTGATACCCCAGA GGATTAGGAACAGGAAACAGCTATGAC | Forward primer for *infA* gene deletion |
| SEQ ID No.111 | SWTO144 | AAACCGACCTTTTATCCAGCCGCCCTTCAGGGCGTGA GGAGACAAGTAAAACGACGGCCAGT | Reverse primer for *infA* gene deletion |
| SEQ ID No.112 | SWTO145 | CGCCCATCGTTTATTTCACC | Identification primer 1 for *infA* for strain verification |
| SEQ ID No.113 | SWTO146 | GCGAGGATTATTACCTTCCGTAC | Identification primer 2 for *infA* for strain verification |
| SEQ ID No.114 | SWTO147 | GTCCGCCCTGAGCAAAGA | Forward primer for qPCR detection of EGFP mRNA |
| SEQ ID No.115 | SWTO148 | TCCAGCAGGACCATGTGATC | Reverse primer for qPCR detection of EGFP mRNA |
| SEQ ID No.116 | SWTO149 | TGTCTCAGATGGCGTTCGTG | Reverse identification primer for GSDMD-N |
| SEQ ID No.117 | SWTO150 | AAGACCCCTAGGAATGCTCG | Reverse identification primer for vectors pEU013 and pEU014 |
| SEQ ID No.118 | SWTO151 | GCGGAAGGTCCTTTCACTTT | Forward identification primer for GSDMD-N |
| SEQ ID No.119 | SWTO152 | TGACATTCCCATCCTCCGTC | Reverse identification primer for GSDME-N |
| SEQ ID No.120 | SWTO153 | GGAAGCAGGAGGTGGATTTG | Forward identification primer for GSDME-N |
| SEQ ID No.121 | SWTO154 | TGCCGAGCCCGATGTG | Forward primer for qPCR detection of GSDMD-N mRNA |
| SEQ ID No.122 | SWTO155 | GGCCATCCATTGCATCGTA | Reverse primer for qPCR detection of GSDMD-N mRNA |
| SEQ ID No.123 | SWTO156 | CCAGCTGCCACCACCATT | Forward primer for qPCR detection of GSDME-N mRNA |
| SEQ ID No.124 | SWTO157 | GGCCGTCCAGTTTCACGTAT | Reverse primer for qPCR detection of GSDME-N mRNA |

Although the present application has been described above with reference to preferred embodiments, these embodiments are merely exemplary and illustrative. On this basis, various substitutions and modifications can be made to the present application, all of which fall within the scope of protection of the present application.

## Claims

1. A eukaryotic expression vector, comprising a first expression unit and a second expression unit, wherein the first expression unit comprises a bacteriophage-derived first promoter, a eukaryotic therapeutic protein gene expression module, and a bacteriophage-derived RNA polymerase gene transcription module; the second expression unit comprises a bacteriophage-derived second promoter and a membrane-disrupting protein gene expression module, the first promoter respectively controls the expression of the eukaryotic therapeutic protein gene and the transcription of the bacteriophage-derived RNA polymerase gene, the second promoter controls the expression of the membrane-disrupting protein gene, and the bacteriophage-derived promoters bind to an RNA polymerase encoded by a specific bacteriophage RNA polymerase gene on a host bacterial chromosome.

2. The eukaryotic expression vector according to claim 1, wherein the bacteriophage-derived first promoter and second promoter are selected from the group consisting of T7, T3, and SP6 promoters.

3. The eukaryotic expression vector according to claim 2, wherein the bacteriophage-derived first promoter and second promoter are T7 promoters.

4. The eukaryotic expression vector according to any one of claims 1 to 3, wherein the eukaryotic therapeutic protein gene expression module comprises a sequentially expressed bacteriophage-derived promoter, an IRES sequence, a Kozak sequence, an open reading frame (ORF) region of the eukaryotic therapeutic protein gene, and a 3'-UTR region, and its product mRNA comprises the IRES sequence, the Kozak sequence, the ORF region of the eukaryotic therapeutic protein gene, and the 3'-UTR region.

5. The eukaryotic expression vector according to any one of the preceding claims, wherein the bacteriophage-derived RNA polymerase gene transcription module comprises a sequentially expressed bacteriophage-derived promoter, an IRES sequence, a Kozak sequence, an ORF region of the specific bacteriophage RNA polymerase gene, a polyA tail, and a T7 terminator sequence, and its product mRNA comprises the sequentially expressed IRES sequence, Kozak sequence, ORF region of the specific bacteriophage RNA polymerase gene, and polyA tail.

6. The eukaryotic expression vector according to any one of the preceding claims, further comprising an essential gene *asd* of *Salmonella*, wherein the *asd* gene on the host bacterial chromosome is deleted.

7. The eukaryotic expression vector according to any one of the preceding claims, further comprising a replicon essential for plasmid replication.

8. The eukaryotic expression vector according to claim 7, wherein the replicon is selected from pUC, p15A, ColE1, and R6K.

9. The eukaryotic expression vector according to claim 8, wherein the replicon is pUC.

10. The eukaryotic expression vector according to any one of the preceding claims, wherein the therapeutic protein gene is an N-terminal fragment of a pyroptosis-inducing therapeutic gene derived from eukaryotic cells.

11. The eukaryotic expression vector according to claim 10, wherein the therapeutic protein gene is selected from the group consisting of the N-terminus of GSDM-A, the N-terminus of GSDM-B, the N-terminus of GSDM-C, the N-terminus of GSDM-D, and the N-terminus of GSDM-E.

12. The eukaryotic expression vector according to claim 11, wherein the therapeutic protein gene is the N-terminus of GSDM-D.

13. The eukaryotic expression vector according to claim 11, wherein the therapeutic protein gene is the N-terminus of GSDM-E.

14. The eukaryotic expression vector according to any one of the preceding claims, wherein the membrane-disrupting protein gene is selected from the listeriolysin *hlyA* gene.

15. The eukaryotic expression vector according to any one of the preceding claims, wherein the delivery manner of the eukaryotic expression vector is a cytoplasmic expression mode of an mRNA/DNA mixture, that is, the eukaryotic expression vector DNA and the mRNA produced by the transcription of the first expression unit are mixed and delivered into a eukaryotic cell via the host bacterium, thereby achieving cytoplasmic expression.

16. The eukaryotic expression vector according to claim 15, which adopts a "self-amplifying" mode, wherein the mRNA produced by the transcription of the first expression unit is translated into T7 RNA polymerase in the eukaryotic cytoplasm, which then acts on the eukaryotic expression vector DNA to achieve transcription and translation of the target gene in the cytoplasm, and further re-amplifies the expression of T7 RNA polymerase to form a cycle.

17. The eukaryotic expression vector according to any one of the preceding claims, wherein the host bacterium is a Gram-negative bacterium.

18. A method for constructing the eukaryotic expression vector according to any one of the preceding claims, comprising the following steps:
(1) sequentially ligating the bacteriophage-derived first promoter, the eukaryotic therapeutic protein gene expression module, and the bacteriophage-derived RNA polymerase gene transcription module according to any one of the preceding claims, to construct a first expression unit;
(2) sequentially ligating the bacteriophage-derived second promoter and the membrane-disrupting protein gene expression module according to any one of the preceding claims, to construct a second expression unit;
(3) ligating the plasmid replicon, the *asd* gene, and the above two expression units together to form a complete eukaryotic expression vector.

19. A modified Gram-negative bacterium, comprising the eukaryotic expression vector according to any one of the preceding claims.

20. The Gram-negative bacterium according to claim 19, wherein the specific bacteriophage RNA polymerase gene is a gene corresponding to the bacteriophage-derived first promoter and second promoter.

21. The Gram-negative bacterium according to claim 20, wherein the specific bacteriophage RNA polymerase gene is selected from a T7 RNA polymerase gene, a T3 RNA polymerase gene, and an SP6 RNA polymerase gene.

22. The Gram-negative bacterium according to claim 21, wherein the specific bacteriophage RNA polymerase gene is a T7 RNA polymerase gene.

23. The Gram-negative bacterium according to any one of claims 19 to 22, wherein the expression of the specific bacteriophage RNA polymerase gene is regulated by a constitutive expression promoter.

24. The Gram-negative bacterium according to claim 23, wherein the constitutive expression promoter is a lacUV5 promoter.

25. The Gram-negative bacterium according to any one of claims 19 to 24, further comprising a hypoxia-specific gene expression cassette, wherein the hypoxia-specific gene expression cassette comprises:
a) a forward hypoxia promoter comprising FNR binding sites, wherein said forward hypoxia promoter is capable of being induced for expression under hypoxia;
b) a survival-essential gene; and
c) a reverse hyperoxia promoter comprising FNR and ArcA binding sites, wherein said reverse hyperoxia promoter is capable of functioning under oxygen content conditions of normal organs;
wherein the survival-essential gene is a gene encoding an alanine racemase.

26. The Gram-negative bacterium according to claim 25, wherein:
the FNR binding site of the forward hypoxia promoter or the reverse hyperoxia promoter conforms to the motif TTGATNNNNATCAA, wherein N is any base selected from A, T, C, and G; any base in the TTGAT and ATCAA sequences of the conserved binding site may be substituted, provided that the total number of substituted bases does not exceed 3, and no 3 consecutive adjacent bases are substituted;
the ArcA binding site of the reverse hyperoxia promoter conforms to the motif GTTAATTA, wherein any base may be substituted, provided that the total number of substituted bases does not exceed 2.

27. The Gram-negative bacterium according to claim 25 or 26, wherein the forward hypoxia promoter and/or the reverse hyperoxia promoter of the hypoxia-specific gene expression cassette are promoters derived from Gram-negative bacteria.

28. The Gram-negative bacterium according to any one of claims 25 to 27, wherein
the forward hypoxia promoter is selected from the promoter region sequences of *yhbU* or *ynfK*;
the survival-essential gene is selected from *alr* or *dadX*;
the reverse hyperoxia promoter is selected from the promoter region sequences of *cyoA* or *ydcI*.

29. The Gram-negative bacterium according to claim 28, wherein the forward hypoxia promoter is *yhbU*, which is selected from the group consisting of *Salmonella, Escherichia coli, Shigella, Yersinia, Enterobacter cloacae*, *Cronobacter*, *Klebsiella*, *Pantoea*, *Serratia*, and *Shimwellia*.

30. The Gram-negative bacterium according to claim 28, wherein the forward hypoxia promoter is *ynfK*, which is selected from the group consisting of *Salmonella*, *Escherichia coli*, *Serratia*, *Shigella*, and *Lactobacillus reuteri*.

31. The Gram-negative bacterium according to claim 28, wherein the alanine racemase *alr* gene and/or the *dadX* gene are selected from the group consisting of *Salmonella*, *Escherichia coli*, *Shigella*, *Klebsiella*, *Yersinia*, *Haemophilus*, and *Pseudomonas*.

32. The Gram-negative bacterium according to claim 28, wherein the hypoxia-specific gene expression cassette consists of the forward hypoxia promoter *yhbU*, the survival-essential gene *alr*, and the reverse hyperoxia promoter *cyoA*.

33. The Gram-negative bacterium according to claim 28, wherein the hypoxia-specific gene expression cassette consists of the forward hypoxia promoter *yhbU*, the survival-essential gene *alr*, and the reverse hyperoxia promoter *ydcI*.

34. The Gram-negative bacterium according to claim 28, wherein the hypoxia-specific gene expression cassette consists of the forward hypoxia promoter *ynfK*, the survival-essential gene *dadX*, and the reverse hyperoxia promoter *cyoA*.

35. The Gram-negative bacterium according to claim 28, wherein the hypoxia-specific gene expression cassette consists of the forward hypoxia promoter *ynfK*, the survival-essential gene *dadX*, and the reverse hyperoxia promoter *ydcI*.

36. The Gram-negative bacterium according to any one of claims 25-35, wherein the hypoxia-specific gene expression cassette is regulated by oxygen concentration.

37. The Gram-negative bacterium according to claim 36, wherein the forward hypoxia promoter functions when the oxygen concentration is lower than 1%, and does not function when the concentration is higher than 1%; and/or the reverse hyperoxia promoter functions when the oxygen concentration is higher than 1%, and does not function when the oxygen concentration is lower than 1%.

38. The Gram-negative bacterium according to claim 37, wherein the forward hypoxia promoter functions when the oxygen concentration is lower than 0.8%, and does not function when the concentration is higher than 0.8%; and/or the reverse hyperoxia promoter functions when the oxygen concentration is higher than 0.8%, and does not function when the oxygen concentration is lower than 0.8%.

39. A method for controlling therapeutic protein expression in a prokaryotic cell using the eukaryotic expression vector according to any one of claims 1-17, comprising the following steps:
(1) preparing the eukaryotic expression vector according to any one of claims;
(2) integrating a constitutively expressed bacteriophage RNA polymerase gene expression cassette into a host bacterium chromosome;
(3) deleting the *asd* gene from the host bacterium chromosome;
(4) transforming the eukaryotic expression vector according to any one of the preceding claims into the host bacterium.

40. The method according to claim 39, further comprising integrating the hypoxia-specific gene expression cassette according to any one of claims 25-38 into the host bacterium chromosome.

41. The method according to claim 39 or 40, wherein the host bacterium is a Gram-negative bacterium.

42. The Gram-negative bacterium according to any one of the preceding claims, selected from the group consisting of *Salmonella*, *Escherichia coli*, *Shigella*, *Yersinia*, *Enterobacter cloacae*, *Cronobacter*, *Klebsiella*, *Pantoea*, *Serratia*, *Shimwellia*, *Enterobacter ludwigii*, *Haemophilus*, *Vibrio*, *Pseudomonas*, *Pasteurella*, *Bordetella*, *Bordetella pertussis*, *Acinetobacter baumannii*, *Burkholderia*, *Vibrio vulnificus*, *Bacteroides fragilis*, *Pseudomonas syringae*, *Pseudomonas putida*, *Legionella*, *Klebsiella pneumoniae*, *Vibrio parahaemolyticus*, *Vibrio cholerae*, *Yersinia pestis*, *Catarrhalis coccus*, *Moraxella catarrhalis*, *Campylobacter jejuni*, *Shigella dysenteriae*, *Neisseria gonorrhoeae*, *Haemophilus influenzae*, *Moraxella*, *Neisseria meningitidis*, *Proteus vulgaris*, *Proteus mirabilis*, *Pasteurella haemolytica*, *Legionella pneumophila*, *Yersinia pestis*, *Shigella sonnei*, *Pseudomonas aeruginosa*, *Yersinia enterocolitica*, *Cryptococcus neoformans*, *Burkholderia cenocepacia*, and *Helicobacter pylori*.

43. The Gram-negative bacterium according to claim 42, wherein the bacterium is *Salmonella*.

44. The Gram-negative bacterium according to claim 42 or 43, wherein the bacterium is an attenuated Gram-negative bacterium.

45. Th*e* Gram-negative bacterium according to claim 44, wherein the attenuated Gram-negative bacterium is *Salmonella*.

46. The Gram-negative bacterium according to any one of the preceding claims, wherein the delivery manner of the eukaryotic expression vector is a cytoplasmic expression mode of an mRNA/DNA mixture, that is, the eukaryotic expression vector DNA and the mRNA produced by the transcription of the first expression unit are mixed and delivered into a eukaryotic cell via the host bacterium, thereby achieving cytoplasmic expression.

47. A pharmaceutical composition comprising the eukaryotic expression vector according to any one of claims, or the Gram-negative bacterium according to any one of claims.

48. The pharmaceutical composition according to claim 47, further comprising a pharmaceutically acceptable carrier.

49. The pharmaceutical composition according to claim 48, wherein the pharmaceutically acceptable carrier is selected from the group consisting of disintegrants, binders, fillers, buffers, tonicity agents, stabilizers, antioxidants, surfactants, and lubricants.

50. The pharmaceutical composition according to any one of claims 47-49, for use in treating solid tumors.

51. Use of the eukaryotic expression vector, the Gram-negative bacterium, or the pharmaceutical composition according to any one of claims in the preparation of a medicament for treating tumor.

52. The use according to claim 51, wherein the tumor is a solid tumor.

53. The pharmaceutical composition according to claim 50 or the use according to claim 52, wherein the solid tumor is selected from the group consisting of tumors/cancers of the breast, bone, liver, lung, skin, kidney, stomach, pancreas, prostate, lymph (non-Hodgkin's lymphoma, Hodgkin's lymphoma), intestines (colon cancer, rectal cancer), pelvis (cervical cancer, ovarian malignancy, endometrial cancer, ovarian cancer), nervous system, head and neck, and bladder, etc.

54. The pharmaceutical composition according or the use according to claim 53, wherein the solid tumor is selected from the group consisting of breast cancer, osteosarcoma, liver cancer, lung cancer, melanoma, kidney cancer, stomach cancer, pancreatic cancer, prostate cancer, colon cancer, ovarian cancer, neuroblastoma, squamous cell carcinoma, and bladder cancer.

55. A method for treating a tumor, comprising administering to a subject the eukaryotic expression vector according to any one of the preceding claims, the Gram-negative bacterium according to any one of the preceding claims, or the pharmaceutical composition according to any one of the preceding claims.

56. The method according to claim 55, wherein the tumor is a solid tumor.

57. The method according to claim 56, wherein the solid tumor is selected from the group consisting of tumors/cancers of the breast, bone, liver, lung, skin, kidney, stomach, pancreas, prostate, lymph (non-Hodgkin's lymphoma, Hodgkin's lymphoma), intestines (colon cancer, rectal cancer), pelvis (cervical cancer, ovarian malignancy, endometrial cancer, ovarian cancer), nervous system, head and neck, and bladder, etc.

58. The method according to claim 57, wherein the solid tumor is selected from the group consisting of breast cancer, osteosarcoma, liver cancer, lung cancer, melanoma, kidney cancer, stomach cancer, pancreatic cancer, prostate cancer, colon cancer, ovarian cancer, neuroblastoma, squamous cell carcinoma, and bladder cancer.
